(19) Europäisches Patentamt
European Patent Office
Office européen des brevets

(11) Publication number: 0 405 506 A1

# (12) EUROPEAN PATENT APPLICATION

(21) Application number: 90112261.4

(51) Int. Cl.[5]: C07K 5/00, A61K 37/02

(22) Date of filing: 27.06.90

(30) Priority: 30.06.89 US 375107
06.06.90 US 531771

(43) Date of publication of application:
02.01.91 Bulletin 91/01

(84) Designated Contracting States:
AT BE CH DE DK ES FR GB GR IT LI LU NL SE

(71) Applicant: ABBOTT LABORATORIES
CHAD-0377, AP6D/2, One Abbott Park Road
Abbott Park, Illinois 60064-3500(US)

(72) Inventor: Chung, John Y.-L.
236 Bingham Circle
Mundelein, IL 60060(US)
Inventor: Tufano, Michael D.
6539 N. Washtenaw
Chicago, I. 60645(US)
Inventor: May, Paul D.
18416 -122 ND.ST.
Bristol, WI 53104(US)
Inventor: Shiosaki, Kazumi
1021 Mayfair
Libertyville, IL 60048(US)
Inventor: Nadzan, Alex M.
1690 Young Drive
Libertyville, IL 60048(US)
Inventor: Garvey, David S.
1130 Harlon Court
Lake Forest Illinois 60045(US)
Inventor: Shue, Youe-Kong
259 Sheffield Lane
Vernon Hills, Il 60061(US)
Inventor: Brodie, Mark S.
1319 Cariann Lane
Glenview, IL 60025(US)
Inventor: Holladay, Mark W.
369 Pierce Court
Vernon Hills, Il 60061(US)

(74) Representative: Modiano, Guido et al
MODIANO, JOSIF, PISANTY & STAUB
Modiano & Associati Via Meravigli, 16 16 16
I-20123 Milano(IT)

## (54) Tetrapeptide type-B CCK receptor ligands.

(57) A tetrapeptide type-B CCK receptor ligand of the formula
**A-B-C-D**
or a pharmaceutically acceptable salt thereof, which is useful for treating central nervous system disorders, substance abuse, gastrointestinal disorders, endocrine disorders, eating-related disorders and treatment of shock, respiratory and cardiocirculatory insufficiencies.

EP 0 405 506 A1

Xerox Copy Centre

# TETRAPEPTIDE TYPE-B CCK RECEPTOR LIGANDS

This is a continuation-in-part of U.S. patent application Serial No. 375,107, filed June 30, 1989.

## Technical Field

The present invention relates to novel compounds and compositions that have biological activity as cholecystokinin receptor ligands, processes for preparing such compounds, synthetic intermediates employed in these processes and methods for treating central nervous system disorders, substance abuse, gastrointestinal disorders, endocrine disorders, eating-related disorders and treatment of shock, respiratory and cardiocirculatory insufficiencies.

## Background of the Invention

Cholecystokinin (CCK) and related molecules constitute a family of polypeptides that are widely distributed in various parts of the body including the gastrointestinal tract, endocrine glands and in the peripheral and central nervous systems. CCK was first isolated from hog intestines as a 33 amino acid fragment (CCK-33) (Mutt and Jorpes, Biochem. J. , 125 , 628 (1981)). Recently CCK-33 has been identified in the brain along with two smaller fragments: an octapeptide (CCK-8), which is the predominant form in the brain, and a tetrapeptide (CCK-4) (Dockray, Nature , 264 , 402 (1979).

Two sub-types of the CCK receptor have been identified. Type-A receptors, found predominantly in the periphery, bind CCK-8 with high affinity but have low affinity for desulfated CCK-8 and CCK-4. The brain contains predominantly the Type-B receptors that bind CCK-8, desulfated CCK-8 and CCK-4 with high affinity.

CCK plays a variety of regulatory roles in the periphery including gallbladder contraction, pancreatic enzyme secretion, inhibition of gastric emptying and insulin secretion. Further, CCK in the brain has been suggested to have a role in appetite control, schizophrenia, in memory and cognition, and as a potential therapy for drug abuse. The high concentrations of CCK and CCK receptors found in the brain further support a major brain function for this peptide.

CCK-8 and the desulfated form were shown to be equipotent in enhancing learning and memory in mice and monkeys (Pietrusiak, et al. Soc. Neurosci. Abstr. 13 , 1030, 1988). The equipotency by the two compounds suggest that the actions are mediated by Type-B CCK receptors. In addition, actions of centrally administered CCK were not affected by peripherally administered antagonists that are selective for Type-A CCK receptors.

Several references have disclosed CCK agonists or analogs of CCK-8 and CCK-4. For example, Rivier, U.S. Patent No. 4,490,364, issued December 25, 1984, discloses heptapeptide, octapeptide and nonapeptide analogs of CCK-8 as CCK agonists for stimulating gallbladder contractions, arresting the secretion of gastric acid and treating convulsions.

Bertolini, European Patent Application No. EP0239716, published October 7, 1987, discloses fragments of gastrin containing the tetrapeptide L-trytophyl-L-methionyl-L-aspartyl-L-phenylalanylamide for the therapy of shock and of respiratory and cardiocirculatory insufficiencies.

German Patent Application No. DT2245459, published March 22, 1973, discloses the preparation of N-protected forms of L-tryptophyl-L-methionyl-L-aspartyl-L-phenylalanylamide.

Japanese Patent Application No. JA7138992, published November 16, 1971, discloses the preparation of protected forms of L-tryptophyl-L-methionyl-L-aspartyl-L-phenylalanylamide.

Morley, Fed. Proc. , 27 1314 (1968), discloses analogs of L-tryptophyl-L-methionyl-L-aspartyl-L-phenylalanylamide including Cbz-L-tryptophyl-L-methionyl-L-3-(5-tetrazolyl)alanyl-L-phenylalanylamide.

Morley, Nature , 207 1356 (1965), discloses analogs of L-tryptophyl-L-methionyl-L-aspartyl-L-phenylalanylamide including:
Cbz-Phe-Met-Asp-Phe-$NH_2$; Boc-Trp-Met-Asp-Phe-$NH_2$; Boc-(4-Me)Trp-Met-Asp-Phe$NH_2$; Boc-(5-Me)Trp-Met-Asp-Phe$NH_2$; Boc-(6-Me)Trp-Met-Asp-Phe$NH_2$; 2-indolyl-$CH_2$-C(O)-Met-Asp-Phe$NH_2$; Boc-Trp-Nle-Asp-Phe$NH_2$; Cbz-Trp-Eth-Asp-Phe$NH_2$; Cbz-Trp-Nval-Asp-Phe$NH_2$; Cbz-Trp-Ala-Asp-Phe$NH_2$; Cbz-Trp-Met-Asp-Tyr$NH_2$; and Cbz-Trp-Met-Asp-(OMe)Tyr$NH_2$.

Charpentier, Peptides , 9 835 (1988), discloses peptide analogs of CCK having affinity for central CCK receptors.

Charpentier, Proc. Natl. Acad. Sci. USA , 85 1968 (1988), discloses cyclic peptide CCK analogs having affinity for central CCK receptors.

Horwell, J. Med. Chem. , 30 729 (1987), discloses small peptide CCK analogs as probes for central nervous system CCK receptors.

None of the above-mentioned references disclose or suggest the tetrapeptide analogs of CCK of this invention, which possess affinity and selectivity for Type-B CCK receptors.

## Summary of the Invention

In accordance with the present invention there are Type-B selective cholecystokinin agonists of the formula:

**A-B-C-D I**

or a pharmaceutically acceptable salt thereof.

A is functionalized acetyl or $R_9$-C(O)- wherein $R_9$ is heterotricyclic or carbotricyclic.

B is a functionalized 2-aminopropionyl residue.

Alternatively, A-B taken together is functionalized piperazinedionyl or functionalized 5-amino-3-aza 4-keto-hexanoyl.

C is

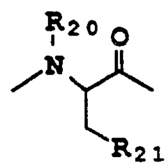

wherein

$R_{20}$ is hydrogen or loweralkyl and

$R_{21}$ is carboxy or tetrazolyl.

Alternatively, B-C taken together is a bridged Ala-Asp residue or a bridged Ala-(tetrazolyl)Ala residue.

D is functionalized ethylamino, functionalized tetrahydroisoquinolyl, functionalized piperazinon-1-yl, dehydro-Phe amide or an analog of dehydro-Phe.

Alternatively, C-D taken together is functionalized succinimidyl.

The term "loweralkyl" as used herein refers to straight or branched chain alkyl radicals having from 1 to 7 carbon atoms including, but not limited to, methyl, ethyl, propyl, isopropyl, butyl, isobutyl, sec-butyl, pentyl, hexyl, heptyl and the like.

The term "alkenyl" as used herein refers to a $C_2$ to $C_{12}$ straight or branched chain of carbon atoms which contains a carbon-carbon double bond including, but not limited to, vinyl, allyl, propenyl, butenyl, isoprenyl and the like.

The term "alkylene" as used herein refers to a straight or branched chain spacer group containing 1 to 6 carbon atoms including, but not limited to, $-CH_2-$, $(-CH(CH_3)-$, $-(CH_2)_4-$ and the like.

The term "alkenylene" as used herein refers to a straight or branched chain spacer group containing 2 to 6 carbon atoms and a carbon-carbon double bond including, but not limited to $-CH=CH-$, $-C(CH_3)=CH-$, $-CH=CH-CH_2-$ and the like.

The term "halo" or "halogen" as used herein refers to chloro, bromo, iodo or fluoro.

The term "haloalkyl" as used herein refers to a loweralkyl radical in which one or more hydrogen atoms are replaced with halogen including, but not limited to chloromethyl, fluoromethyl, dichloroethyl, trifluoromethyl and the like.

The term "alkoxy" as used herein refers to $-OR_{51}$ wherein $R_{51}$ is loweralkyl.

The term "thioalkyoxy" as used herein refers to $-SR_{52}$ wherein $R_{52}$ is loweralkyl.

The term "acyl" as used herein refers to $-C(O)R_{53}$ wherein $R_{53}$ is loweralkyl.

The term "functionalized acetyl" as used herein includes:

I)

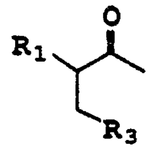

wherein $R_1$ is
(1) hydrogen,
(2) halo,
(3) hydroxy,
(4) alkoxy,
(5) thioalkoxy,
(6) amino,
(7) alkylamino,
(8) (N-protected)amino,
(9) (N-protected)(alkyl)amino,
(10) dialkylamino,
(11) substituted loweralkyl wherein the substituent is independently selected from (N-protected)-amino, alkylamino and dialkylamino,
(12) substituted alkenyl wherein the substituent is independently selected from (N-protected)-amino, alkylamino and dialkylamino,
(13) $R_5$-$R_4$-C(O)-N($R_6$)- wherein
$R_4$ is $C_1$ to $C_6$ alkylene or $C_2$ to $C_6$ alkenylene,
$R_5$ is phenyl, substituted phenyl, (N-protected)-amino, amino, alkylamino, dialkylamino, alkoxy or thioalkoxy and.
$R_6$ is hydrogen or loweralkyl; and
$R_3$ is
(1) naphthyl,
(2) substituted naphthyl,
(3) phenyl,
(4) substituted phenyl or
(9) benzoheterocyclic; (II) $R_7$-$R_8$-Y- wherein
Y is absent or -C(O)-,
$R_7$ is
(1) naphthyl,
(2) substituted naphthyl or
(3) benzoheterocyclic and
$R_8$ is
(1) $C_1$ to $C_6$ alkylene or
(2) $C_2$ to $C_6$ alkenylene;

III)

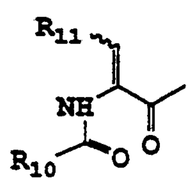

wherein
$R_{10}$ is
(1) naphthyl,
(2) substituted naphthyl or
(3) benzoheterocyclic and
$R_{11}$ is
(1) phenyl,
(2) substituted phenyl or

(3) monocyclic heteroaromatic;

IV)

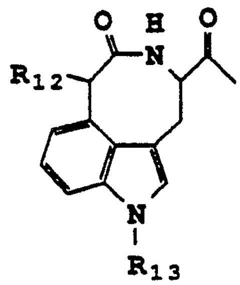

wherein
$R_{12}$ is
(1) hydrogen,
(2) loweralkyl,
(3) halo,
(4) amino,
(5) alkylamino,
(6) dialkylamino,
(7) hydroxy,
(8) alkoxy or
(9) thioalkoxy and
$R_{13}$ is
(1) hydrogen,
(2) loweralkyl or
(3) acyl; and

V)

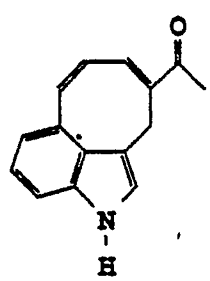

The term "functionalized 2-aminopropionyl" as used herein includes:

I)

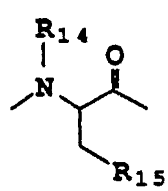

wherein
$R_{14}$ is
(1) hydrogen or
(2) loweralkyl and
$R_{15}$ is
(1) loweralkyl,
(2) cycloalkyl or

(3) $C_1$ to $C_4$ alkylene substituted with thioalkoxy or alkoxy; and

II)

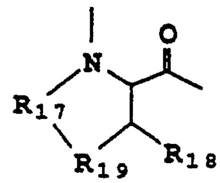

wherein
$R_{17}$ is $C_2$ to $C_4$ alkylene,
$R_{18}$ is
(1) hydrogen,
(2) loweralkyl,
(3) alkoxy or
(4) thioalkoxy and
$R_{19}$ is
(1) S,
(2) O or
(3) absent.

The term "functionalized piperazinedionyl" as used herein includes

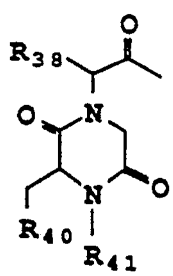

wherein $R_{38}$ is loweralkyl or $R_{38}$ is loweralkyl substituted with alkoxy or thioalkoxy,
$R_{40}$ is
(1) naphthyl,
(2) substituted naphthyl or
(3) benzoheterocyclic and $R_{41}$ is
(1) hydrogen or
(2) loweralkyl.

The term "functionalized succinimidyl" as used herein includes

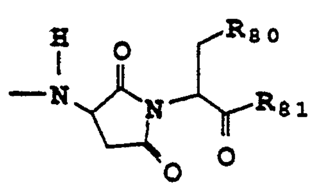

wherein
$R_{80}$ is
(1) phenyl,
(2) substituted phenyl,
(3) naphthyl,
(4) substituted naphthyl,
(5) loweralkyl,
(6) cycloalkyl,

(7) heterocyclic or
(8) bicyclic heterocyclic and
$R_{81}$ is
(1) $-NHR_{25}$ wherein $R_{25}$ is hydrogen, loweralkyl, hydroxy or alkoxy or
(2) $-NHNHR_{26}$ wherein $R_{26}$ is hydrogen or loweralkyl.

The term "functionalized 5-amino-3-aza 4-keto-hexanoyl" as used herein includes

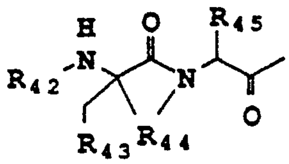

wherein
$R_{42}$ is
(1) hydrogen or
(2) an N-protecting group, $R_{43}$ is
(1) naphthyl,
(2) substituted naphthyl or
(3) benzoheterocyclic, $R_{44}$ is $C_2$ to $C_4$ alkylene and
$R_{45}$ is
(1) loweralkyl or
(2) loweralkyl substituted by -SH, thioalkoxy, -OH or alkoxy.

The term "alkoxycarbonyl" as used herein refers to $-C(O)OR_{54}$ wherein $R_{54}$ is loweralkyl.

The term "alkylamino" as used herein refers to $-NHR_{55}$ wherein $R_{55}$ is loweralkyl.

The term "dialkylamino" as used herein refers to $-NR_{56}R_{57}$ wherein $R_{56}$ and $R_{57}$ are independently selected from loweralkyl.

The term "(N-protected)amino" as used herein refers to $-NHR_{75}$ wherein $R_{75}$ is an N-protecting group.

The term "functionalized ethylamino" as used herein includes:

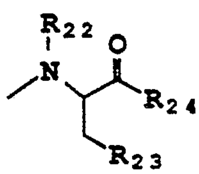

wherein
$R_{22}$ is
(1) hydrogen or
(2) loweralkyl, $R_{23}$ is
(1) phenyl,
(2) substituted phenyl,
(3) naphthyl,
(4) substituted naphthyl,
(5) loweralkyl,
(6) cycloalkyl,
(7) heterocyclic or
(8) bicyclic heterocyclic and
$R_{24}$ is
(1) $NHR_{25}$ wherein $R_{25}$ is hydrogen, loweralkyl, hydroxy or alkoxy or
(2) $-NHNHR_{26}$ wherein $R_{26}$ is hydrogen or loweralkyl.

The terms "dehydro-Phe amide" and "dehydro-Phe amide analog" as used herein include

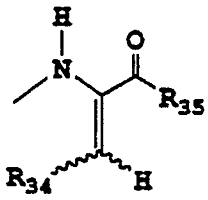

wherein
$R_{34}$ is
(1) phenyl
(2) substituted phenyl,
(3) naphthyl,
(4) substituted naphthyl,
(5) heterocyclic or
(6) bicyclic heterocyclic and
$R_{35}$ is
(1) $NHR_{36}$ wherein $R_{36}$ is hydrogen, hydroxy, alkoxy or loweralkyl or
(2) $-NHNHR_{37}$ wherein $R_{37}$ is hydrogen or loweralkyl.

The terms "bridged Ala-Asp residue" and "bridged Ala-(tetrazolyl)Ala residue" as used herein includes

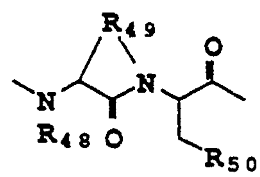

wherein
$R_{48}$ is
(1) hydrogen or
(2) loweralkyl, $R_{49}$ is $C_2$ to $C_4$ alkylene and
$R_{50}$ is
(1) carboxy or
(2) tetrazolyl.

The term "loweralkylsulfonyl" as used herein refers to $-SO_2R_{58}$ wherein $R_{58}$ is loweralkyl.

The term "cycloalkyl" as used herein refers to cyclic alkyl radical having from 3 to 8 carbon atoms, including, but not limited to, cyclopropyl, cyclobutyl, cyclopentyl, cyclohexyl and the like.

The term "substituted phenyl" as used herein refers to a phenyl group which is substituted with one, two or three substituents independently selected from loweralkyl, halogen, haloalkyl, alkoxy, benzyloxy, thioalkyoxy, hydroxy, alkoxycarbonyl, carboxy, amino, alkylamino, dialkylamino, nitro and $-OSO_3H$.

The term "substituted naphthyl" as used herein refers to a naphthyl group which is substituted with one, two or three substituents independently selected from loweralkyl, halogen, haloalkyl, alkoxy, benzyloxy, thioalkyoxy, hydroxy, alkoxycarbonyl, carboxy, amino, alkylamino, dialkylamino, nitro and $-OSO_3H$.

The term "heterocyclic" as used herein refers to a 5-or 6-membered ring containing one, two or three nitrogen atoms; one nitrogen and one sulfur atom; or one nitrogen and one oxygen atom; wherein the 5-membered ring has 0-2 double bonds and the 6-membered ring has 0-3 double bonds; wherein the nitrogen and sulfur heteroatoms may optionally be oxidized; and wherein the nitrogen heteroatom may optionally be quaternized. Heterocyclics include, but are not limited to, pyridyl, furyl, thienyl, tetrahydrofuryl, imidazolyl, piperazinyl, piperidinyl, pyrrolidinyl, thiazolyl and the like. Heterocyclics can be unsubstituted or substituted with one or two substituents independently selected from loweralkyl, halogen, haloalkyl, alkoxy, benzyloxy, thioalkyoxy, hydroxy, alkoxycarbonyl, acyl, formyl, carboxy, amino, alkylamino, dialkylamino, nitro, loweralkylsulfonyl and $-OSO_3H$.

The term "monocyclic heteroaromatic" refers to a 5-or 6-membered aromatic ring containing one, two or three nitrogen atoms; one nitrogen and one sulfur atom; or one nitrogen and one oxygen atom including, but not limited to, pyridyl, pyrimidyl, furyl, thienyl and the like. Monocyclic heteroaromatics can be unsubstituted or substituted with one or two substituents independently selected from loweralkyl, halogen, haloalkyl, alkoxy, benzyloxy, thioalkyoxy, hydroxy, acyl, formyl, alkoxycarbonyl, carboxy, amino, alkylamino,

dialkylamino, nitro, loweralkylsulfonyl and $-OSO_3H$.

The term "bicyclic heterocyclic" as used herein refers to a system of two fused rings, one of which is a heterocyclic ring and the other ring being selected from benzene and heterocyclic. Bicyclic heterocyclics can be unsubstituted or substituted with one or two substituents independently selected from loweralkyl, halogen, haloalkyl, alkoxy, benzyloxy, thioalkyoxy, hydroxy, acyl, formyl, alkoxycarbonyl, carboxy, amino, alkylamino, dialkylamino, nitro, loweralkylsulfonyl and $OSO_3H$.

The term "benzoheterocylic" as used herein refers to a heterocyclic ring to which is fused a benzene ring. Benzoheterocyclics include, but are not limited to, indolyl, indolinyl, benzofuryl, benzothienyl, benzimidazolyl, quinolyl, isoquinolyl and the like. In addition, benzoheterocyclics can be unsubstituted or substituted with one or two substituents independently selected from loweralkyl, halogen, haloalkyl, alkoxy, benzyloxy, thioalkyoxy, hydroxy, acyl, formyl, alkoxycarbonyl, carboxy, amino, alkylamino, dialkylamino, nitro, loweralkylsulfonyl and $-OSO_3H$.

The term "heterotricyclic" as used herein refers to a system of three fused rings, one ring being a heterocyclic ring defined as above and the other two rings being independently selected from heterocyclic as defined above and benzene. Heterotricyclic groups can be unsubstituted or substituted with one or two substituents independently selected from loweralkyl, halogen, haloalkyl, alkoxy, benzyloxy, thioalkyoxy, hydroxy, acyl, formyl, alkoxycarbonyl, carboxy, amino, alkylamino, dialkylamino, nitro, loweralkylsulfonyl and $-OSO_3H$. Heterotricyclics include, but are not limited to carbazolyl, $\beta$-carbolinyl and the like.

The term "carbotricyclic" as used herein refers to a system of three fused carbocyclic rings, each ring containing 5 or 6 carbon atoms and each ring being unsaturated or saturated. Carbotricyclic groups can be unsubstituted or substituted with one or two substituents independently selected from loweralkyl, halogen, haloalkyl, alkoxy, benzyloxy, thioalkyoxy, hydroxy, acyl, formyl, alkoxycarbonyl, carboxy, amino, alkylamino, dialkylamino, nitro, loweralkylsulfonyl and $-OSO_3H$.

The term "functionalized tetrahydroisoquinolyl" as used herein includes

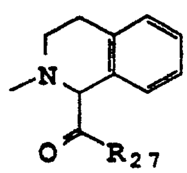

wherein
$R_{27}$ is
(1) $NHR_{28}$ wherein $R_{28}$ is hydrogen, hydroxy, alkoxy or loweralkyl,
(2) alkoxy or
(3) $-NHNHR_{29}$ wherein $R_{29}$ is hydrogen or loweralkyl.

The term "functionalized piperazinon-1-yl" as used herein includes

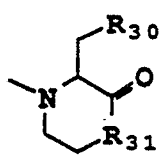

wherein
$R_{30}$ is
(1) phenyl,
(2) substituted phenyl,
(3) naphthyl,
(4) substituted naphthyl,
(5) loweralkyl,
(6) heterocyclic or
(7) bicyclic heterocyclic and
$R_{31}$ is
(1) $-N(R_{32})-$ wherein $R_{32}$ is hydrogen or loweralkyl or
(2) $-N(NH(R_{33}))-$ wherein $R_{33}$ is hydrogen or loweralkyl.

The term "N-protected' or "N-protecting group" as used herein refers to those groups intended to protect the N-terminus of an amino acid or peptide or to protect an amino group against undesirable reactions during a synthetic procedure or to prevent the attack of exopeptidases on the compounds or to increase the solubility of the compounds and includes, but is not limited to, sulfonyl, acetyl, pivaloyl, t-butyloxycarbonyl (BOC), benzyloxycarbonyl (Cbz), benzoyl or an L- or D-aminoacyl residue, which may itself be N-protected.

All amino acid residues identified herein are in the natural L-configuration unless otherwise designated with "D-", (e.g., D-Trp). In keeping with standard peptide nomenclature, J. Biol. Chem. , 243:3557-59, (1969), abbreviations for amino acid residues are used herein. The compounds of formula I contain two or more asymmetric carbon atoms and thus can exist as pure diastereomers, mixtures of distereomers, diastereomeric racemates or mixtures of diastereomeric racemates. The present invention includes within its scope all of the isomeric forms.

Other abbreviations used herein are as shown in the following Table of Correspondence:

TABLE OF CORRESPONDENCE

| SYMBOL | REPRESENTS |
|---|---|
| Tyr | L-tyrosine |
| Gly | glycine |
| Phe | L-phenylalanine |
| Met | L-methionine |
| Ala | L-alanine |
| Ser | L-serine |
| Ile | L-isoleucine |
| Leu | L-leucine |
| Thr | L-threonine |
| Val | L-valine |
| Pro | L-proline |
| Lys | L-lysine |
| His | L-histidine |
| Gln | L-glutamine |
| Glu | L-glutamic acid |
| Trp | L-tryptophan |
| Arg | L-arginine |
| Asp | L-aspartic acid |
| Asn | L-asparagine |
| Cys | L-cysteine |
| α-Nal | 3-(1-naphthyl)alanine |
| β-Nal | 3-(2-naphthyl)alanine |
| Cha | 3-(cyclohexyl)alanine |
| Pip | pipecolinic acid |

Ctp

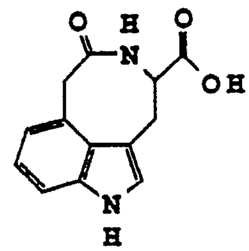

Tpp

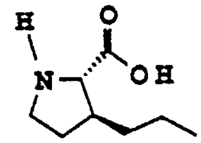

Cpp

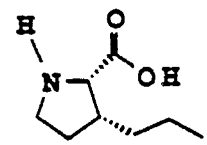

Tiq

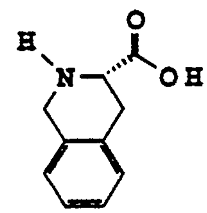

Nct

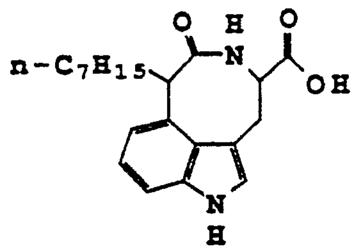

(Dehydro)Phe

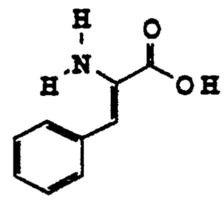

Asu

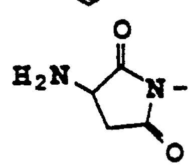

The abbreviation ψ(CH=CH) indicates that the amide (-C(O)NH-) bond of a peptide has been replaced by a double bond. For example, Trp-ψ(CH=CH)Leu represents a tryptophan residue bonded to a leucine residue wherein the amide bond is replaced as shown below.

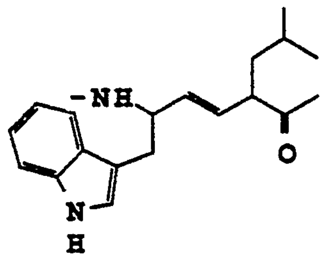

It is noted that all amino acid residue sequences are represented herein by formulae whose left to right orientation is in the conventional direction of amino-terminus to carboxy-terminus.

Exemplary compounds of the present invention include:

Ctp-Leu-Asp-Phe-$NH_2$;
BOC-Trp-Leu-Asp-Tiq-$NH_2$;
β-Carboline-3-carbonyl-Leu-Asp-Phe-$NH_2$;
Nct-Leu-Asp-Phe-$NH_2$;
BOC-Trp-Leu-Asp-(N-Me)Phe-$NH_2$;
Ctp-Leu-Asp-(N-Me)Phe-$NH_2$;
2(S)-[3-((tert-Butyloxycarbonylamino)-3-(indol-3-yl-methyl)-2-oxo-1-pyrrolidinyl)-4-methyl-pentanoyl-Asp-Phe-$NH_2$;
4-Carboxamide-[3,4,5,6-c,d]-indolyl) Leu-Asp-Phe-$NH_2$;
BOC-Trp-Leu-[β-1(2)H-tetrazol-5-yl]-L-alanyl-Phe-$NH_2$;
D-Ctp-Leu-Asp-Phe-$NH_2$;
3-(3-Quinolyl)-propionyl-Leu-Asp-Phe-$NH_2$;
3-(2-Naphthyl)-propionyl-Leu-Asp-Phe-$NH_2$;
3-(9-Phenanthryl)-propionyl-Leu-Asp-Phe-$NH_2$;
Cbz-Trp-(NMe)Leu-Asp-Phe-$NH_2$;
3-Carboxy-2(S)-[3(R)-(Boc-Tryptophan carboxamide)-2-oxo]-1-pyrrolidinyl-2(S)-propionic acid phenylalanine amide;
N-(4-Hydroxycinnamoyl)-Trp-Met-Asp-Phe-$NH_2$;
N-(β-Naphthoyl)-(dehydro)Phe-Leu-Asp-Phe-$NH_2$;
N-(α-Naphthoyl)-(dehydro)Phe-Leu-Asp-Phe-$NH_2$;
BOC-Trp-Leu-Asp-(dehydro)Phe-$NH_2$;
BOC-Trp-Leu-Asp-(m-nitro)Phe-$NH_2$;
BOC-Trp-Leu-Asp-(m-[N-ethyl]amino)Phe-$NH_2$;
Ctp-Leu-Asp-(dehydro)Phe-$NH_2$;
BOC-Trp-Met-Asu-Phe-$NH_2$;
BOC-Trp-Tpp-Asp-(N-Me)Phe-$NH_2$;
BOC-Trp-Nle-Asp-(N-Me)Phe-$NH_2$;
BOC-Trp-Nle-Asp-(dehydro)Phe-$NH_2$;
BOC-Trp-Leu-Asu-Phe-$NH_2$;
$N^{\alpha}$-BOC-$N^{in}$-formyl-Trp-Leu-Asp-Phe-$NH_2$;
$N^{\alpha}$-BOC-$N^{in}$-(2,3-dihydroindole-Trp)-Leu-Asp-Phe-$NH_2$;
(2,3-Dihydroindole-Trp)-Leu-Asp-Phe-$NH_2$;
BOC-5-amino-2-(3′-indolylmethyl)pentanoyl-Leu-Asp-Phe-$NH_2$;
BOC-5-amino pentanoyl-Leu-Asp-Phe-$NH_2$;
BOC-5-amino-2-(3′-indolylmethyl)-2-pentenoyl Leu Asp-Phe-$NH_2$;
$N^{\alpha}$-BOC-$N^{in}$-propionyl-Trp-Leu-Asp-Phe-$NH_2$;
3-(3-Indolyl)propionyl-Nle-Asp-Phe-$NH_2$;
8-(N-(t-Butoxycarbonyl)amino)-2-(3-indolemethyl)-2-octanoyl-Leu-Asp-Phe-$NH_2$;
N-(Indole-2-propyl)Leu-Asp-Phe-$NH_2$;
BOC-Gly-ψ(CH=CH)-Trp-Leu-Asp-Phe-$NH_2$;
BOC-Trp-Leu-Asp-Phe-$OCH_3$;
Ctp-Leu-Asp-Phe-$NHNH_2$; BOC-Trp-Leu-Asp-Trp-$NH_2$;
BOC-Trp-Leu-Asp-α-Nal-$NH_2$;
BOC-Trp-Leu-Asp-β-Nal-$NH_2$; BOC-Trp-Leu-Asp-Cha-$NH_2$;
BOC-Trp-Pro-Asp-Phe-$NH_2$;

BOC-Trp-Tpp-Asp-Phe-$NH_2$;
Ctp-Tpp-Asp-Phe-$NH_2$;
BOC-$\beta$-Nal-Leu-Asp-Phe-$NH_2$;
BOC-Trp-Leu-Asp-3(S)-benzyl-2-oxo-4-piperazine;
Ctp-Tpp-Asp-(NMe)Phe-$NH_2$;
Ctp-Cpp-Asp-Phe-$NH_2$;
BOC-Trp-Tpp-Asp-Trp-$NH_2$;
BOC-$\beta$-Nal-Tpp-Asp-$\alpha$-Nal-$NH_2$;
BOC-$\beta$-Nal-Tpp-Asp-$\beta$-Nal-$NH_2$;
Ctp-Tpp-Asp-$\alpha$-Nal-$NH_2$;
Ctp-Tpp-Asp-$\beta$-Nal-$NH_2$;
Ctp-Tpp-Asp-Cha-$NH_2$;
Ctp-1,4-thiazane-3-carbonyl-Asp-Phe-$NH_2$;
Ctp-Pip-Asp-Phe-$NH_2$;
2-(3-[Indole-3-methyl]diketopiperazinyl)valeryl-Asp-Phe-$NH_2$;
2-(3-[Indole-3-methyl]diketopiperazinyl)isocaproyl-Asp-Phe-$NH_2$;
2-(3-[Indole-3-methyl]diketopiperazinyl)caproyl-Asp-Phe-$NH_2$;
BOC-Trp-(N-1,4-thiazepine-2-carbonyl)-Asp-Phe-$NH_2$-BOC-Trp-Cha-Asp-Phe-$NH_2$; and
2-(3-[Naphthyl-2-methyl]diketopiperazinyl)isocaproyl-Asp-Phe-$NH_2$.

Preferred compounds of the present invention include:
Ctp-Leu-Asp-Phe-$NH_2$;
BOC-Trp-Leu-Asp-Tiq-$NH_2$;
Ctp-Leu-Asp-N-Me-Phe-$NH_2$;
Ctp-Leu-Asp-(dehydro)Phe-$NH_2$;
BOC-Trp-Tpp-Asp-Phe-$NH_2$;
Ctp-Tpp-Asp-Phe-$NH_2$;
Ctp-Tpp-Asp-(NMe)Phe-$NH_2$;
Ctp-Tpp-Asp-$\alpha$-Nal-$NH_2$;
Ctp-Tpp-Asp-$\beta$-Nal-$NH_2$; and
Ctp-1,4-thiazane 3-carbonyl Asp-Phe-$NH_2$.

## General Synthetic Procedures

The compounds of the present invention, represented by formula I, can be prepared via a number of processes that are considered standard in peptide synthesis. A detailed description of these methods is contained in "The Peptides, Vol. 1", Gross and Meienhofer, Eds., Academic Press, New York, 1979. Coupling methods that are employed include the carbodiimide method (1,3-dicyclohexylcarbodiimide [DCC], 1-(3-dimethylaminopropyl-3-ethylcarbodiimide hydrochloride [EDCI]) with the option of racemization preventing additives (1-hydroxybenzotriazole [HOBT]), the mixed anhydride method (typically using isobutylchloroformate), the azide method, the acid chloride method, the symmetrical anhydride method, the use of bis(2-oxo-3-oxazolidinyl)phosphinic chloride (BOP-Cl), and the active ester method (N-hydroxysuccinimide esters, 4 nitrophenol esters, 2,4,5-trichlorophenol esters, and the like).

The compounds of the invention are prepared by stepwise coupling of the amino acids or by coupling together fragments of dipeptide length or greater. Thus, the free carboxylic acid moiety from one amino acid or peptide fragment is activated and allowed to condense with the free nitrogen group of the second amino acid or peptide fragment. The coupling reactions are conducted in solvents such as methylene chloride ($CH_2Cl_2$), tetrahydrofuran (THF), dimethylformamide (DMF) or other such solvents under an inert atmosphere such as nitrogen ($N_2$) or argon (Ar).

During the coupling process, the non-participating carboxylic acids or amines on the reacting set of amino acids or peptide fragments are protected by protecting groups which can be selectively removed at a later time if desired. A detailed description of these groups and their selection and chemistry is contained in "The Peptide, Vol. 3", Gross and Meienhofer, Eds., Academic Press, New York 1981. Thus, useful protective groups for the amino groups are benzyloxycarbonyl (Cbz), t-butyloxycarbonyl (BOC), 2,2,2-trichloroethoxycarbonyl (Troc), t-amyloxycarbonyl, 4-methoxybenzyloxycarbonyl, 2-(trimethylsilyl)-ethyoxycarbonyl, 9-fluorenylmethoxycarbonyl (FMOC), phthaloyl, acetyl, formyl, trifluoroacetyl, and the like.

Examples of useful protective groups for the carboxylic acid includes esters such as methyl, ethyl, benzyl, t-butyl, 2,2,2-trichloroethyl, allyl, 4-nitrobenzyl, and the like. Removal of these protecting groups can

be accomplished selectively by employing various acid or base catalyzed hydrolytic, hydrogenolytic, thermal or dissolving metal conditions.

For the production of a compound of the invention where any one or several of the constituent amino acids bear an N-alpha-alkyl group, specifically methyl, the corresponding N-alpha-alkyl amino acid can be prepared via the method described by Benoiton (Can. J. Chem., 55, 906 (1977)) or Shuman ("Peptides: Proceedings of the Seventh American Peptide Symposium", D. Rick, E. Gross, Eds., Pierce Chemical Co., Rockford, IL 1982, p 617) wherein the BOC or Cbz protected amino acid is treated with a base in the presence of a chelating agent such as a crown ether and then quenched with methyl iodide. An alternative method described by Freidinger (J. Org. Chem., 48, 77 (1983)) in which triethylsilane reduction of the oxazolidinone of an amino acid directly produces the N-alpha-methyl derivative can also be utilized.

The following examples will serve to further illustrate preparation of the novel compounds of the invention.

## Example 1

### CTP-Leu-Asp-Phe-$NH_2$

## Example 1a

### Bromoacetyl-L-Trp-OBn

To a solution of L-Trp-OBn hydrochloride (15.0 g, 45.35 mmol) and triethylamine (9.18 g, 90.68 mmol) in absolute ethanol (200 ml) cooled to 0 °C was added bromoacetyl chloride (7.13 g, 45.34 mmol) dropwise over 10 min. The reaction mixture was stirred overnight with warming to ambient temperature. The solvent was removed in vacuo and the residue partitioned between aqueous 0.1 N HCl (250 ml) and ethyl acetate (250 ml). After drying with $Na_2SO_4$, the organic layer was concentrated in vacuo and the residue chromatographed (ethyl acetate/hexanes) to yield 12.60 g of a clear colorless oil.

## Example 1b

### 4-Benzyloxycarbonyl-6-oxo-3,4,5,6-tetrahydro-1H,5H-azocin[4,5,6-c,d]indole (Ctp-OBn)

The title compound was prepared by an adaptation of an original procedure reported by O. Yonemitsu et al. [J.Am.Chem.Soc. , 88 ,3941-3945 (1966)]. A solution of Example 1a (6.7 g, 16.14 mmol) in 40% ethanol/water (1 l) at 80 °C was irradiated (Hanovia 450W. Hg lamp/vycor filter) for 3 h. The resulting mixture was concentrated in vacuo to 600 ml and the aqueous layer extracted with ethyl acetate (3x). After drying with $Na_2SO_4$, the solvent was removed in vacuo and the residue chromatographed ethyl acetate) to yield a mixture of 2- and 4-substituted cyclized products (1.20 g) along with 4.90 g unreacted starting material. The mixture was recrystallized (ethyl acetate/diethyl ether/ hexanes) to yield 0.45 g white crystalline solid. $^1H$ NMR (DMSO-$d_6$, 300 MHz) $\delta$ 3.42 (m, 3H), 4.10 (br d, 1H), 4.30 (br s, 1H), 5.25 (s, 2H), 6.75 (d, 1H), 6.94 (t, 1H), 7.12 (m, 3H), 7.30 (m, 5H). $[\alpha]_D$ = -75.1° (c=1, MeOH). mp 149-151 °C.

## Example 1c

Ctp-Leu-Asp(OBn)-Phe-$NH_2$

A mixture of Example 1b (0.20 g, 0.60 mmol) and 5% Pd/$BaSO_4$ (0.04 g) in methanol (10 ml) was hydrogenated under one atmosphere of hydrogen at ambient temperature for 0.5 h. The catalyst was filtered and the solvent removed in vacuo to yield 0.13 g of free acid (CTP-OH) as a pink solid sufficient for use without further purification. To the acid (0.13 g, 0.55 mmol) in DMF (8 ml) cooled to 0 °C were added Leu-Asp(OBn)-Phe-$NH_2$ hydrochloride [J. Martinez, et al, J. Med. Chem. , 28 , 1874 (1985)](0.28 g, 0.55 mmol), diphenyl phosphoryl azide (0.18 g, 0.65 mmol) (DPPA), and N-methyl morpholine (0.12 g, 1.20 mmol). The mixture was stirred overnight with warming to ambient temperature. The solvent was concentrated to 2 ml and the residue dissolved in ethyl acetate (50 ml). The organic layer was washed with aqueous solutions of saturated $NaHCO_3$ (3x) and 0.1N HCl (3x). After drying with $Na_2SO_4$, the solvent was removed in vacuo. The residue was triturated from ethyl acetate/diethyl ether and the resulting solid collected and recrystallized from ethanol/water to yield 0.34 g white solid in two crops. MS(FAB+) m/e 709 $(M+H)^+$. $^1H$ NMR (DMSO-$d_6$, 300 MHz) $\delta$ 0.85 (m, 6H), 1.40 (m, 1H), 1.55 (m, 1H), 2.59 (m, 1H), 2.79 (m, 2H), 2.95 (m, 1H), 3.25-3.56 (m, 4H), 4.15 (m, 1H), 4.32 (m, 2H), 4.55 (m, 1H), 5.10 (s, 2H), 6.74 (d, 1H), 6.95 (t, 1H), 6.99 (d, 1H), 7.10 (m, 9H), 7.30 (m, 5H), 7.83 (d, 1H), 8.20 (d, 1H), 8.37 (d, 1H), 10.93 (br s, 1H).

Example 1d

Ctp-Leu-Asp-Phe-$NH_2$

A mixture of example 1c (0.21 g, 0.29 mol) and 5% Pd/$BaSO_4$ (0.05 g) in DMF (10 ml) was hydrogenated under one atmosphere hydrogen at ambient temperature overnight. The solvent was filtered twice through celite and concentrated to 1 ml. The solvent was triturated with ethyl acetate to yield 0.17 g white solid. MS (FAB+) m/e 619 $(M+H)^+$. $^1H$ NMR (DMSO-$d_6$, 300 MHz) $\delta$ 0.85 (m, 6H), 1.35 (m, 2H), 1.55 (m, 1H), 2.45 (m, 1H), 2.65 (m, 2H), 2.77 (m, 1H), 2.97 (m, 1H), 4.12 (m, 2H), 4.45 (q, 1H), 6.73 (d, 1H), 6.93 (t, 1H), 6.97 (d, 1H), 7.05 (m, 8H), 7.30 (br s, 1H), 7.83 (d, 1H), 8.18 (d, 1H), 8.27 (d, 1H), 10.93 (br s, 1H), 12.35 (br s, 1H). Anal calcd for $C_{32}H_{38}N_6O_7 \cdot H_2O$: C 60.35, H 6.13, N 13.20: Found: C 60.71 H 6.12 N 13.15. mp 236-237.5 °C (dec).

Example 2

Boc-Trp-Leu-Asp-Tiq-$NH_2$

Example 2a

3(S)-1,2,3,4 Tetrahydroisocuinoline-3-carboxamide

Benzyl 3(S)-1,2,3,4-Tetrahydroisoquinoline-3-carboxylate p-Toluenesulfonate (11.68 g, 26.62 mmol) prepared according to the method of K.Hayashi et al., Chem. Pharm. Bull. 31 (1), 312-314,(1983) was partitioned between ethyl acetate (300 ml) and $NaHCO_3$ (300 ml). After drying with $Na_2SO_4$, the organic phase was filtered and the solvent removed in vacuo to yield 7.11 g slightly yellow oil. This oil (7.11 g) was dissolved in methanol and cooled to -20 °C. The solution was saturated with dry gaseous ammonia, capped with a septa and allowed to stand at 0 °C overnight. The solvent was removed in vacuo and the residue

triturated with diethyl ether to yield 4.08 g white solid. mp 157-158 °C.$[\alpha]_D$ -57 °C (c=1, MeOH).

Example 2b

Boc-Asp-(OBn)-Tiq-$NH_2$

To an ice cold suspension of example 2a (7.0 g, 39.27 mmol) and Boc-Asp(OBn)OH(12.86 g, 39.27 mmol) in ethyl acetate (250 ml) were added 4-dimethylamino pyridine(4-DMAP) (0.97g, 0.79 mmol), and N,N′-dicyclohexylcarbodiimide(DCCI) (9.84g, 47.72 mmol) in ethyl acetate (20 ml). The reaction was stirred at 0 °C for 4 h and then the solution was filtered. The organics were washed with aqueous solutions of 0.1N HCl (3x) and saturated $NaHCO_3$ (3x). After drying with $Na_2SO_4$ the solvent was removed in vacuo and the residue chromatographed (ethyl acetate/hexanes) to yield 10.10 g white amorphous solid. MS (EI) m/e 481 ($M^+$). $^1H$ NMR ($CDCl_3$, 300 MHz) $\delta$ 1.39 (s, 9H), 2.78 (dd, 1H), 2.93 (dd, 1H), 3.15 (dd, 1H), 3.45, (dd, 1H), 7.48 (m, 1H), 4.95 (m, 6H), 5.30 (m, 1H), 6.63 (br s, 1H), 7.13 (m, 4H), 7.30 (m, 5H).

Example 2c

Boc-Asp-Tiq-NH

A mixture of example 2b (0.66 g, 1.38 mmol) and 10% Pd/C (0.050 g) in methanol (25 ml) was hydrogenated under one atmosphere of hydrogen at ambient temperature for 0.5 h. The catalyst was filtered and the sovent removed in vacuo to yield 0.54 g white amorphous solid. $^1H$ NMR ($CDCl_3$, 300 MHz) $\delta$ 1.39 (br s, 9H), 2.70 (m, 1H), 2.98 (m, 2H), 3.25 (m, 1H), 4.77,(m, 1H), 5.0 (m, 1H), 6.92 (br s, 1H), 7.12 (m, 4H), 7.30 (br s, 1H).

Example 2d

Leu-Asp-Tiq-$NH_2$ Trifluoroacetate

A solution of example 2c (0.54 g, 1.38 mmol) in acetic acid (5 ml) was treated with 1.4N HCl/acetic acid (10 ml) at ambient temperature for 0.5 h. The solution was triturated with diethyl ether to yield 0.37 g of a hygroscopic white solid which was used without further purification. To a solution of the solid (0.37 g, 1.13 mmol) and Boc-Leu-N-hydroxysuccinimide ester (0.37 g, 1.13 mmol) in DMF (10 ml) cooled to 0 °C was added N,N-diisopropylethylamine (0.15 g, 1.13 mmol). The mixture was stirred overnight with warming to ambient temperature. The solvent was concentrated in vacuo to 5 ml and triturated with ethyl acetate to yield 0.42 g white solid containing diketopiperazine by-product. The crude solid was treated with 80% trifluoroacetic acid/water for 0.3 h and triturated with dietyl ether to yield 0.29 g white solid devoid of the diketopiperazine by-product. $^1H$ NMR (DMSO-$d_6$, 300 MHz),$\delta$ 0.82 (m, 6H), 1.44 (m, 2H), 1.55 (m, 1H), 2.47 (m, 2H), 2.67 (m, 6H), 3.00 (dd, 1H), 3,2 (m, 1H),3.67 (m, 1H), 4.33 (m, 1H), 4.55 (m, 1H), 7.15 (m, 5H),7.30 (br s, 1H), 8.50 (m, 2H), 8.67 (m, 1H),

Example 2e

Boc-Trp-Leu-Asp-Tiq-$NH_2$

To a solution of example 2d (0.073 g, 0.14 mmol) in DMF (3 ml) cooled to 0 °C were added Boc-Trp-2,4,5- trichlorophenyl ester (0.068 g, 1.14 mmol) and N,N-diisopropylethyl amine (0.18 g,0.14 mmol). The mixture was stirred overnight with warming to ambient temperature. Cyclohexane (15 ml) and water (15 ml) were added and the mixture gently warmed on a steam bath forming a turbid suspension. The solid was filtered and washed with cyclohexane (2x) to yield 0.11 g white solid. MS(EI) m/e 691 ($M^+$). $^1H$ NMR (DMSO-$d_6$,300 MHz), δ 0.80 (m, 6H), 1.30 (s, 9H), 1.35 (m, 2H), 1.55 (m, 1H), 2,63 (m, 1H), 2.73 (m, 2H), 2.93 (m, 2H), 4.15 (m, 1H), 4.33 (m, 2H), 4.45 (q, 1H), 6.82 (d, 1H), 6.93 (t, 1H), 7.02 (t, 1H), 7.12 (br s, 1H), 7.15 (m, 6H), 7.55 (d, 1H), 8.82 (m, 2H), 8.25 (d, 1H), 10.78 (br s, 1H), 12.38 (br s, 1H). Anal calcd for $C_{35}H_{45}N_6O_8.H_2O$: C 60.43, H 6.76, N 12.09; Found: C 60.50 H 6.61, N 11.95. mp 200-201.5 °C.

Example 3

β-Carboline-3-carbonyl-Leu-Asp-Phe-$NH_2$

Example 3a

β-Carboline-3-carbonyl-Leu-Asp(OBn)-Phe-$NH_2$

To a solution of β-carboline-3-carboxylic acid (0.15 g, 0.70 mmol) prepared according to the method of M.Cain et. al. J.Med.Chem. 25 , 1081-1091, (1982). in DMF (10 ml) cooled to 0 °C were added Leu-Asp-(OBn)-Phe-NH 2 hydrochloride (0.37 g, 0.70 mmol), diphenylphosphoryl azide (0.21 g, 0.78 mmol), and triethyl amine (0.14 g, 1.41 mmol). The mixture was stirred overnight with warming to ambient temperature. Ethyl acetate (30 ml) was added and the organic phase washed with aqueous solutions of 0.10 N HCl (3x) and 0.50 N NaOH (3x). After drying with $Na_2SO_4$ the solvent was removed in vacuo and the residue triturated with cold ethyl acetate to yield 0.26 g yellow solid. MS(FAB+) m/e 677 $(M+H)^+$. $^1H$ NMR(DMSO-$d_6$, 300 MHz). δ 0.85 (m, 6H), 1.45 (m, 3H), 2.57 (dd, 1H), 2.80 (m, 2H), 2.95 (dd, 1H), 4.33 (m, 1H), 4.60 (m, 2H), 5.0 (m, 2H), 7.07 (m, 13H), 7.57 (m, 2H), 7.93 (d, 1H), 8.35 (d, 1H), 8.55 (dd, 2H), 8.87 (s, 1H), 8.93 (d, 1H), 12.00 (br s, 1H). Anal calcd for $C_{38}H_{40}N_6O_6 \cdot 0.5H_2O$: C 66.29, H 5.83, N 12.26; Found: C 66.29, H 5.68, N 12.03. mp 187-189.5 °C.

Example 3b

β-Carboline-3-carbonyl-Leu-Asp Phe-$NH_2$

A mixture of example 3a (0.15 g, 0.22 mmol) and 5% Pd/ $BaSO_4$ (0.035 g) in methanol (10 ml) was hydrogenated under one atmosphere of hydrogen at ambient temperature for 1.2 h. The catalyst was filtered and the solvent removed in vacuo. The residue was dissolved in methanol (1 ml) and triturated with diethyl ether to yield 0.96 g tan solid. MS(FAB+) m/e 587 $(M+H)^+$. $^1H$ NMR (DMSO-$d_6$, 300 MHz), 0.85 (m, 6H), 1.45 (m, 3H), 2.43 (m, 1H), 2.66 (dd, 1H), 2.80 (m, 2H), 3.00 (dd, 1H), 4.30 (m, 1H), 4.32 (m, 1H), 4.62 (m, 1H), 7.06 (m, 1H), 7.15 (m, 4H), 7.26 (m, 2H), 7.56 (m, 2H), 7.94 (d, 1H), 8.35 (d, 1H), 8.51 (dd, 2H), 8.85 (br s, 1H), 8.93 (d, 1H), 12.01 (br s, 1H), 12.35 (br s, 1H). Anal calcd for $C_{31}H_{34}N_6O_6 \cdot 2H_2O$: C 59.80, H 6.15, N 13.50; Found: C 59.80, H 5.94, N 13.36.

## Example 4

### Nct-Leu-Asp-Phe-$NH_2$

## Example 4a

### 2-Bromo-nonanoic acid

To a mixture of nonanoic acid (20 g, 126 mmol) and bromine (24.3 g 152 mmol) was added phosphorous trichloride (5.2 g, 38 mmol) slowly over 0.5 h. The mixture was refluxed for 3 d. and then diluted with ethyl acetate (200 ml). The organic phase was washed with aqueous saturated $NaHSO_3$ (3x), separated, dried ($Na_2SO_4$), and the solvent removed in vacuo to yield 27.0 g brown oil. $^1H$ NMR ($CDCl_3$, 60 MHz), $\delta$ 0.70 (m, 3H), 1.20 (m, 9H), 1.90 (m, 3H), 4.13 (m, 1H), 10.80 (br s, 1H).

## Example 4b

### 2-Bromo-nonanoyl-Trp-OBn

To a solution of example 4a (1.5 g, 6.30 mmol) in ethyl acetate (150 ml) cooled to 0 °C were added L-Trp-OBn hydrochloride (2.10 g, 6.30 mmol), triethylamine (0.64 g, 6.30 mmol) and N,N′ dicyclohexylcarbodiimide (1.57 g, 7.60 mmol). The resulting suspension was stirred overnight with warming to ambient temperature. The solution was filtered and the supernatant was washed with aqueous solutions of 0.1N HCl (3x) and 0.5N NaOH (3x). After drying with $Na_2SO_4$ the solvent was removed in vacuo to yield 3.10 g brown oil. $^1H$ NMR ($CDCl_3$, 60 MHz), $\delta$ 0.80 (m, 3H), 1.10 (br s, 9H), 1.67 (m, 3H), 3.20 (d, 2H), 3.95 (t, 1H), 4.55 (m, 1H), 4.95 (br s, 2H), 6.60 (m, 2H), 6.93 (m, 8H), 7.72 (m, 2H).

## Example 4c

### 4-Benzyoxycarbonyl-6-oxo-7-heptyl-3,4,6,7,tetrahydro-1H-5H-azocin[4,5,6-c,d] indole,(Nct-OBn)

A solution of example 4b (3.10 g, 6.0 mmol) in 40% ethanol/water (1 l) was reacted under similar conditions to those descibed in example lb. The residue was chromatographed (ethyl acetate/hexanes) to yield 0.19 g waxy oil. MS (EI) m/e 432 ($M^+$). $^1H$ NMR (CDCl3, 300 MHz), $\delta$ 0.85 (m, 3H), 1.15 (m, 10H), 1.93 (m, 1H), 2.35 (m, 1H), 3.47 (dd, 1H), 3.93 (q, 1H), 4.43 (q, 1H), 4.80 (m, 1H), 5.19 (m, 2H), 6.20 (d, 1H), 6.75 (br s, 1H), 7.05 (d, 1H), 7.13 (t, 1H), 7.20 (d, 1H), 7.93 (m, 5H), 8.00 (br s, 1H).

## Example 4d

### Nct-OH

A mixture of example 4c (0.16 g, 0.37 mmol), and 5% Pd/$BaSO_4$ in methanol (5 ml) was hydrogenated under one atmosphere hydrogen at ambient temperature for 1.0 h. The catalyst was filtered and the solvent removed in vacuo to yield 0.13 g light orange powder. $^1H$ NMR(DMSO-$d_6$,300 MHz), δ 0.84 (m, 3H), 1.15 (m, 9H), 2.12 (m, 1H), 3.40 (m, 2H), 3.86 (m, 1H), 4.34 (m, 1H), 6.73 (d, 1H), 6.96 (t, 1H), 7.13 (d, 1H), 7.16 (br s, 1H), 7.18 (m, 1H), 8.23 (d, 1H), 10.85 (br s, 1H).

## Example 4e

## Nct-Leu-Asp(OBn)-Phe-$NH_2$

A solution of example 4d (0.13 g, 0.37 mmol), Leu-Asp(OBn)-Phe-$NH_2$ hydrochloride (0.21 g, 0.37 mmol), N-methylmorpholine (0.09 g, 0.91 mmol), and DPPA (0.11 g, 0.41 mmol) in DMF (3 ml) was stirred at 0 °C for 3 days. The solvent was concentrated in vacuo to 1 ml and diluted with ethyl acetate (30 ml). The organics were washed with aqueous solutions of 0.1N HCl (3x) and 0.5N NaOH (3x). After drying with $Na_2SO_4$ the solvent was removed in vacuo and the residue triturated with cold ethyl acetate to yield 0.60 g tan solid. MS (FAB+) m/e 806 $(M+H)^+$. $^1H$ NMR(DMSO-$d_6$,300 MHz),.δ 0.75 (m, 9H), 1.15 (m, 12H), 1.45 (m, 3H), 1.80 (m, 1H), 2.05 (m, 1H), 2.43 (dd, 1H), 2.93 (q, 1H), 3.05 (dd, 1H), 3.30 (m, 2H), 3.55 (m, 1H), 3.93 (m, 1H), 4.25 (m, 2H), 4.50 (m, 1H), 4.65 (m, 1H), 6.75 (d, 1H), 7.02 (m, 13H).

## Example 4f

## Nct-Leu-Asp-Phe-$NH_2$

A mixture of example 4e (0.045 g, 0.056 mmol) and 5% Pd/$BaSO_4$ (10 mg) was hydrogenated under one atmosphere of hydrogen at ambient temperature for 1.0 h. The catalyst was filtered, the solvent removed in vacuo, and the residue chromatographed on silica gel using ethyl acetate/pyridine/acetic acid/water (31.4/4.0/1.2/2.2). The solvents were removed in vacuo and the residue suspended in water (2 ml) and lyopholized to yield 0.014 g white solid. MS(FAB+) m/e 717 $(M+H)^+$. $^1H$ NMR(DMSO-$d_6$, 300 MHz), δ 0.70 (m, 9H), 1.10 (m, 12H), 1.55 (m, 1H), 1.70 (m, 1H), 2.05 (m, 1H), 2.30 (m, 2H), 2.65 (m, 1H), 2.98 (m, 1H), 3.05 (m, 1H), 3.40 (m, 1H), 3.55 (m, 1H), 3.70 (m, 1H), 4.25-4.60 (m, 3H), 6.71 (d, 1H), 6.75-7.10 (m, 10H), 7.45 (br s, 1H), 8.01 (d, 1H), 8.05 (br s, 1H), 8.25 (d, 1H), 10.85 (br s, 1H). Anal calcd for $C_{40}H_{52}N_6O_7 \cdot 2.5H_2O$: C 62.08, H 7.42, N 10.86; Found C 62.00, H 7.09, N 10.65.

## Example 5

## Boc-Trp-Leu-Asp-(NMe)Phe-$NH_2$

## Example 5a

## N-CBZ-(NMe)Phe-OH

To a solution of L-N-Me-Phe-OH (2.73 g, 15.23 mmol) and N,N-diisopropylethyl amine (4.13 g, 18.27

mmol) in dioxane/water (1:1 v/v, 100 ml), cooled to 0 °C was added benzyl chloroformate (3.10 g, 18.27 mmol) dropwise over 0.3 h. The reaction was stirred overnight with warming to ambient temperature. Water (75 ml) was added and the solution acidified to pH 3-4 with solid citric acid and the aqueous phase extracted with ethyl acetate (3x) After drying with $Na_2SO_4$, the solvent was removed in vacuo to yield 4.54 g clear, colorless oil. MS(EI) m/e 313 ($M^+$). $^1H$ NMR ($CDCl_3$, 300 MHz) δ 2.65 (m, 3H), 2.96 (m ,1H), 3.28 (m, 1H), 4.84 (m, 1H), 5.02 (m, 2H), 7.10 (m, 10H).

Example 5b

N-CBZ-(NMe)-Phe-$NH_2$

To a solution of example 5a (4.54 g, 14.5 mmol) and N-methylmorpholine (1.47 g, 14.5 mmol) in THF (100 ml) cooled to -20 °C was added ethyl chloroformate (1.56 g, 14.5 mmol). After stirring for 0.25 h the solution was saturated with dry gaseous ammonia. The solution was warmed to 0 °C and stirred for 2.5 h. The solvent was removed in vacuo and the residue partitioned between ethyl acetate (100 ml) and water (100 ml). The organic layer was separated, dried with $Na_2SO_4$, and the solvent removed in vacuo. The residue was recrystallized (ethyl acetate/hexanes) to yield 2.34 g white solid in two crops. MS(EI) m/e 312 ($M^+$). $^1H$ NMR(DMSO-$d_6$, 300 MHz) δ 2.70 (m, 3H), 2.85 (m, 1H), 3.14 (m, 1H), 4.75 (m, 3H), 7.10 (m, 11H), 7.45 (m, 1H) .

Example 5c

(NMe)-Phe-$NH_2$

A mixture of example 5b (2.0 g, 6.40 mmol) and 5% Pd/$BaSO_4$ in methanol (100 ml) was hydrogenated under one atmosphere of hydrogen at ambient temperature overnight. The catalyst was filtered and the solvent was removed in vacuo to yield 1.14 g white solid. MS(EI) m/e 179 $(M+H)^+$.

Example 5d

Boc-Asp(OBn)-(NMe)Phe-$NH_2$

To a solution of example 5c (1.0 g, 5.61 mmol) in ethyl acetate (100 ml) cooled to 0 °C were added Boc-Asp(OBn)OH (1.81 g, 5.61 mmol) 4-DMAP(0.07 g, 0.56 mmol) and DCCI (1.39 g, 6.73 mmol). The suspension was stirred vigorously at 0 °C for 4 h. and filtered. The filtrate was washed with aqueous solutions of 0.1N HCl (3x) and saturated $NaHCO_3$ (3x). After drying with $Na_2SO_4$ the solvent was removed in vacuo. The residue was chromatographed (ethyl acetate/hexanes) to yield 1.62 g amorphous solid. MS-(EI) m/e 483 ($M^+$).

Example 5e

Asp(OBn)-(NMe)Phe-$NH_2$ hydrochloride

Example 5d (1.62 g, 3.35 mmol) in acetic acid (10 ml) was treated with 1.5N HCl/acetic acid (8 ml) for 0.3 h. The solution was triturated with diethyl ether to yield 1.40 g white solid. MS(FAB+) m/e 384 $(M+H)^+$.

Example 5f

Boc-Leu-Asp(OBn)-(NMe)Phe-$NH_2$

To a solution of Boc-Leu-OH (1.10 g, 4.77 mmol) in methylene chloride (50 ml) cooled to 0 °C was added 1-(3-dimethylaminopropyl)-3-carbodiimide(EDCI)(0.45 g, 2.38 mmol). After stirring at 0 °C for 1.5 h. example 5e (1.00 g, 2.38 mmol) was added. N-methylmorpholine (0.48 g, 4.77 mmol) was added slowly over 1 h at 0 °C then the solution was allowed to warm to ambient temperature. The solvent was removed in vacuo and the residue partitioned between ethyl acetate (100 ml) and saturated aqueous $NaHCO_3$ (100 ml). The organics were separated, dried with $Na_2SO_4$, and the solvent removed in vacuo. The residue was chromatographed (ethyl acetate/ hexanes) to yield 1.0 g white amorphous solid. MS(FAB+) m/e 597 $(M+H)^+$. $^1H$ NMR(DMSO-$d_6$, 300 MHz) $\delta$ 0.78 (m, 6H), 1.13 (m, 1H), 1.25-1.60 (m, 11H), 2.14-2.95 (m, 5H), 3.05 (m, 2H), 3.85 (m, 1H), 4.6-5.10 (m, 4H), 6.75 (t, 1H), 7.02 (m, 5H), 7.30 (m, 5H), 8.11 (m, 1H). Anal calcd for $C_{32}H_{44}N_4O_7.0.5H_2O$: C 63.43, H 7.49, N 9.26; Found: C 63.55, H 7.48, N 9.07.

Example 5g

Leu-Asp(OBn)-(NMe)Phe-$NH_2$ hydrochloride

To a solution of example 5f (0.68 g, 1.12 mmol) in acetic acid (2 ml) at ambient temperature was added 1.5N HCl/acetic acid (5 ml). After 0.5 h the solvent was removed in vacuo and the residue dissolved in methanol (1 ml). The solution was triturated with diethyl ether to yield 0.59 g white solid. MS(FAB+) m/e 497 $(M+H)^+$. $^1H$ NMR(DMSO-$d_6$, 300 MHz), $\delta$ 0.82 (m, 8H), 0.98 (m, 1H), 1.23 (m, 2H), 2.18-2.90 (m, 4H), 3.12 (m, 1H), 3.59 (m, 1H), 4.40 (m, 1H), 4.98 (m, 3H), 7.03 (m, 5H), 7.32 (m, 5H), 7.53 (br s, 1H), 8.18 (br s, 2H), 8.88 (dd, 1H).

Example 5h

Boc-Trp-Leu-Asp(OBn)-(NMe)Phe-$NH_2$

A solution of example 5g (0.1 g, 0.18 mmol), Boc-Trp-N-hydroxysuccinimide ester (0.75 g, 0.18 mmol), and N-methylmorpholine (0.24 g, 0.18 mmol) in DMF (2 ml) was stirred at 0 °C for 18 h. Ethyl acetate (30 ml) was added and the organics washed with aqueous solutions of 0.1N HCl (3x) and saturated $NaHCO_3$ - (3x). After drying with $Na_2SO_4$, the solvent was removed in vacuo and the residue chromatographed (ethyl acetate) to yield 0.08 g white solid. MS(FAB+) m/e 783 $(M+H)^+$.

Example 5i

Boc-Trp-Leu-Asp-(NMe)Phe-$NH_2$

A mixture of example 5h (0.06 g, 0.07 mmol) and 5% Pd/$BaSO_4$ (0.02 g) in methanol (5 ml) was hydrogenated under one atmosphere hydrogen at ambient temperature for 1.5 h. The catalyst was filtered and the solvent removed in vacuo. The residue was chromatographed on silica gel using ethyl acetate/pyridine/acetic acid/water (31.4/4.0/1.2/2.2). The solvents were removed in vacuo and the residue suspended in water (5 ml) and lyopholized to yield 0.04 g tan solid. MS(CI/$NH_3$) m/e 693 $(M+H)^+$. $^1H$ NMR-(DMSO-$d_6$, 300 MHz) δ 0.78 (m, 6H), 1.10 (m, 2H), 1.23 (m, 10H), 2.01-2.40 (m, 1H), 2.70 (m, 8H), 4.12 (m, 2H), 4.60-5.20 (m, 2H), 6.70 (m, 1H), 6.90 (m, 12H), 7.56 (m, 1H), 7.80 (m, 1H), 8.22 (m, 1H), 10.75 (m, 1H).

## Example 6

### Ctp-Leu-Asp-(NMe)Phe-$NH_2$

## Example 6a

### Ctp-Leu-Asp(OBn)-(NMe)Phe-$NH_2$

To a solution of CTP-OH (0.096 g, 0.40 mmol), as prepared in example 1c, and example 5g (0.21 g, 0.40 mmol) in DMF (5 ml) cooled to 0 °C were added DPPA (0.13 g, 0.47 mmol) and N-methylmorpholine (0.087 g, 0.87 mmol). The mixture was stirred overnight with warming to ambient temperature. Ethyl acetate (50 ml) was added and the organics washed with aqueous solutions of 0.1N HCl (3x) and saturated $NaHCO_3$ (3x). After drying with $Na_2SO_4$ the solvent was removed in vacuo and the residue chromatographed(ethyl acetate) to yield 0.10 g white solid. MS(FAB+) m/e 723 $(M+H)^+$. $^1H$ NMR(DMSO-$d_6$,300 MHz) δ 0.83 (m, 7H), 1.23 (m, 1H), 1.30 (m, 1H), 1.52 (m, 1H), 2.12-2.60 (m, 1H), 2.62-2.95 (m, 4H), 3.06-3.50 (m, 6H), 4.13 (m, 3H), 4.53-5.12 (m, 3H), 6.73 (d, 1H), 6.92-7.25 (m, 9H), 7.34 (m, 5H), 7.48 (br s, 1H), 8.12 (dd, 2H), 8.37 (d, 1H), 8.61 (d, 1H), 10.90 (br s, 1H).

## Example 6b

### Ctp-Leu-Asp-(NMe)Phe-$NH_2$

A mixture of example 6a (0.96 g, 0.13 mmol) and 5% Pd/$BaSO_4$ (0.015 g), in methanol was hyrogenated under one atmosphere of hydrogen at ambient temperature for 0.5 h. The catalyst was filtered and the solvent removed in vacuo to yield 0.075 g tan solid. MS(FAB+) m/e 633 $(M+H)^+$. $^1H$ NMR(DMSO-$d_6$,300 MHz) δ 0.82 (m, 7H), 1.23 (m, 1H), 1.31 (m, 1H), 1.51 (m, 5H), 2.12-2.60 (m, 1H), 2.56-3.60 (m, 6H), 4.12 (m, 3H), 4.54 (m, 1H), 4.33 (m, 1H), 5.10 (m, 1H), 6.73 (m, 1H), 6.92 (m, 2H), 7.10 (m, 6H), 7.45 (m, 2H), 8.10 (d, 1H), 8.19 (d, 1H), 8.59 (d, 1H), 10.91 (br s, 1H). Anal calcd for $C_{33}H_{40}N_6O_7.2H_2O$: C 59.25, H 6.58, N 12.57; Found: C 59.31, H 6.44, N 12.00.

## Example 7

### 2(S)-[3(S)-((*Tert*-butyloxycarbonylamino)-3-(indol-3-yl methyl)-2-oxo-1-pyrrolidinyl)-4-methyl-pentanoyl-Asp-Phe-$NH_2$

Example 7a

2(S)(3-Carboxy-3(indol-3-ylmethyl)-2-oxo-1-pyrrolidinyl-4-methyl pentanoic acid methyl ester

2(S)(3-carboxy-2-oxo-1-pyrrolidinyl)-4-methyl pentanoic acid methyl ester was prepared according to the method of R.M. Freidinger, J.Org.Chem. 50 (19),3631-3633, (1985). A solution of isopropylidene cyclopropane-1,1-dicarboxylate (5.0 g, 29.30 mmol), Leucine methyl ester hydrochloride (5.31 g, 29.3 mmol), and triethylamine (2.97 g, 29.3 mmol) in degassed DMF (50 ml) was heated at 65 °C for 1 h. The solution was cooled to ambient temperature and poured into a mixture of 10% aqueous $H_2SO_4$ (100 ml) and ethyl acetate (100 ml) and the organic layer separated. After drying with $Na_2SO_4$, the solvent was removed in vacuo to yield 7.55g as a brown oil. To the crude oil (7.55 g) in degassed DMF (50 ml) were added gramine methiodide (13.2 g, 42.94 mmol) and 1,8-diazabicyclo[5.4.0]undec-7-ene (DBU) (5.16 g, 33.90 mmol). The mixture was heated at 85 °C for 18 h and cooled to ambient temperature. The mixture was poured into ethyl acetate (100 ml) and saturated aqueous $NaHCO_3$ (100 ml). The aqueous phase was separated, acidified to pH 3-4 with 10% aqueous $H_2SO_4$, and extracted with ethyl acetate (3x). After drying with $Na_2SO_4$, the solvent was removed in vacuo and the residue chromatographed (1% acetic acid/ ethyl acetate) to yield 1.56 g white solid. MS(FAB+) m/e 387 $(M+H)^+$. $^1H$ NMR(DMSO-$d_6$,300 MHz), $\delta$ 0.65 (m, 6H), 1.23-1.80 (m, 3H), 1.90 (m, 1H), 2.12 (m, 1H), 2.77-3.48 (m, 6H), 3.51 (m, 3H), 4.98 (m, 1H), 6.92 (m, 3H), 7.28 (d, 1H), 7.97 (d, 1H), 10.90 (br s, 1H), 12.70 (br s, 1H).

Example 7b

2(S)-[3-((*Tert*-butyloxycarbonylamino)-3-(indol-3-ylmethyl)-2-oxo-1-pyrrolidinyl)-4-methyl-pentanoic acid]methyl ester

A solution of example 7a (1.22 g, 3.16 mmol), and DPPA (0.96 g, 3.49 mmol) in *tert*-butyl alcohol (50 ml) was refluxed for 48 h under nitrogen. The solvent was removed in vacuo and the residue chromatographed (ethyl acetate/hexanes) to yield 0.48 g clear oil. MS(EI) m/e 457 $(M^+)$. $^1H$ NMR ($CDCl_3$,300 MHz) $\delta$ 0.68 (m, 6H), 1.35-1.73 (m, 12H), 2.25 (m, 3H), 3.05 (m, 3H), 3.58 (m, 3H), 4.52-4.82 (m, 1H), 5.23-5.38 (m, 1H), 7.05 (m, 3H), 7.32 (m, 1H), 7.61 (t, 1H), 8.06 (m, 1H).

Example 7c

2(S)-[3-((*Tert*-butyloxycarbonylamino)-3-(indol-3-ylmethyl)-2-oxo-1-pyrrolidinyl)-4-methyl-pentanoic acid]

To a solution of example 7b (0.46 g, 0.10 mmol) in methanol (5 ml) cooled to 0 °C was added 0.5N NaOH (2.1 ml). The solution was stirred at 0 °C for 18 h., acidified to pH 3-4 with solid citric acid, and extracted with ethyl acetate (3x). After drying with $Na_2SO_4$ the solvent was removed in vacuo to yield 0.40 g clear oil. MS(EI) m/e 443 (M+).

Example 7d

2(S)-[3-((*Tert*-butyloxycarbonylamino)-3-(indol-3-ylmethyl)-2-oxo-1-pyrrolidinyl)-4-methyl-pentanoyl-Asp(OBn)-Phe-$NH_2$

To a solution of example 7c (0.34, 0.78 mmol) and N-methylmorpholine (NMM) (0.08g, 0.78 mmol) in THF (5 ml) at 0 °C was added isobutyl chloroformate (0.107 g, 0.78 mmol). After 10 min. a cold (0 °C) solution of Asp(OBn)-Phe-$NH_2$ trifluoroacetate (0.38 g, 0.78 mmol) and NMM (0.08 g, 0.78 mmol) in DMF (5 ml) was added. The reaction was warmed to 0 °C and stirred for 1h. Ethyl acetate (30 ml) was added and the organics washed with aqueous solutions of 0.1N HCl (3x) and saturated $NaHCO_3$ (3x). After drying with $Na_2SO_4$, the solvent was removed in vacuo to yield 0.58 g waxy solid. MS(CI/$NH_3$) m/e 795 $(M+H)^+$.

Example 7e

2(S)-[3-((*Tert*-butyloxycarbonylamino)-3-(indol-3-ylmethyl)-2-oxo-1-pyrrolidinyl)-4-methyl-pentanoyl-Asp-Phe-$NH_2$

A mixture of example 7d (0.58 g, 0.73 mmol) and 5% Pd/$BaSO_4$ in methanol (15 ml) was hydrogenated under one atmosphere hydrogen at ambient temperature overnight. The catalyst was filtered and the solvent removed in vacuo. The residue was chromatographed on silica gel using ethyl acetate/pyridine/acetic acid/water (31.4/4.0/1.2/2.2) to yield 0.14 g of a more mobile diastereomer (isomer A) and 0.12 g of a less mobile diastereomer (isomer B) . **Isomer A**: MS(CI/$NH_3$) m/e 705 $(M+H)^+$. $^1H$ NMR(DMSO-$d_6$,300 MHz) $\delta$ 0.38 (dd, 6H), 0.72 (m, 1H), 1.30 (m, 11H), 2.17 (m, 2H), 2.38 (m, 1H), 2.50 (m, 2H), 2.72 (m, 5H), 4.28 (m, 2H), 4.46 (m, 1H), 6.93 (t, 1H), 7.04 (t, 1H), 7.10 (m, 4H), 7.21 (m, 5H), 7.51 (m, 3H), 7.92 (d, 1H), 10.98 (br s, 1H), 12.12 (br s, 1H). Anal calcd for $C_{37}H_{48}N_6O_8 \cdot H_2O$: C 61.46, H 6.97, N 11.63; Found: C 61.70, H 6.81, N 11.52. **Isomer B**: MS(CI/$NH_3$) m/e 705 $(M+H)^+$. $^1H$ NMR(DMSO,300 MHz) $\delta$ 0.75 (m, 7H), 1.30-1.62 (m, 11H), 2.0 (m, 2H), 2.40-3.75 (m, 8H), 4.31 (m, 1H), 4.45 (m, 2H), 6.68 (br s, 1H), 6.94 (t, 1H), 7.00 (t, 1H), 7.10 (m, 8H), 7.31 (m, 2H), 7.48 (d, 1H), 7.79 (m, 1H), 8.22 (m, 1H), 10.85 (br s, 1H), 12.30 (br s, 1H), Anal calcd for $C_{37}H_{48}N_6O_8.H_2O$: C 61.46, H 6.97, N 11.63; Found: C 61.30, H 6.90, N 11.12.

Example 8

4-Carboxamide-[3,4,5,6-c,d]-indolyl)-Leu-Asp-Phe-$NH_2$

Example 8a

4-(1-Acetyl-indol-4yl)-but-2-enoic acid methyl ester

To a solution of 1-acetyl-indole-4-carboxaldehyde (2.40 g, 11.93 mmol) prepared according to the method of H. Plieninger et. al. Chem.Ber. 89 , 270, (1956). in THF (100 ml) was added methyl-(triphenylphosphoranylidene)acetate (4.78 g, 14.3 mmol). The solution was stirred at ambient temperature overnight. The solvent was removed in vacuo and the residue chromatographed (ethyl acetate/ hexanes). Recrystallization from ethyl acetate/hexanes resulted in 2.12 g white solid in two crops. MS(CI/$NH_3$) m/e 258 $(M+H)^+$. $^1H$ NMR ($CDCl_3$, 300 MHz) $\delta$ 2.60 (s, 3H), 3.70 (s, 3H), 3.75 (dd, 2H), 3.75 (m, 1H), 6.63 (dd, 1H), 7.05 (d, 1H), 7.12-7.22 (m, 2H), 7.29 (t, 1H), 7.43 (d, 1H), 8.35 (d, 1H).

Example 8b

4-(Indol-4yl)-but-2-enoic acid methyl ester

To a solution of example 8a (1.0 g, 3.89 mmol) in methanol (60 ml) at ambient temperature was added magnesium ethoxide (1.0 g, 8.73 mmol). The reaction was stirred vigorously for 10 min and poured into 0.1 N HCl (100 ml). The aqueous layer was extracted with ethyl acetate (3x) and the organic layer washed with saturated brine (3x). After drying with $Na_2SO_4$ the solvent was removed in vacuo to yield 0.81 g greenish oil. MS(EI) m/e 215 ($M^+$).

Example 8c

4-(3-Formyl-indol-4yl)-but-2-enoic acid methyl ester

Phosphorous oxychoride (0.65 g, 4.16 mmol) was added dropwise to DMF (3 ml) at 0 °C. after 0.5 h example 8b (0.81 g, 3.70 mmol) in DMF (3 ml) was added at 0 °C, then the mixture warmed to 35 °C for 0.75 h. The solution was cooled to ambient temperature and crushed ice (10 g) was added. Solid $NaHCO_3$ was added and to pH 8, the mixture diluted with water (100 ml), and the aqueous extracted with ethyl acetate (3x). The organic phase was separated, dried with $Na_2SO_4$, and the solvent removed in vacuo. The residue was chromatographed (ethyl acetate/ hexanes) to yield 0.39 g purple solid. MS(CI/$NH_3$) m/e 244 $(M+H)^+$. 1H NMR($CDCl_3$, 300 MHz) δ 3.68 (s, 3H), 4.26 (m, 2H), 5.14 (m, 1H), 7.06 (d, 1H), 7.18 (m, 3H), 7.84 (d, 1H), 9.09 (br s, 1H), 9.88 (s, 1H).

Example 8d

4-Methoxycarbonyl-[3,4,5,6,-c,d]-indole

To a solution of example 8c (0.34 g, 1.38 mmol) in benzene (10 ml) was added DBU (0.25 g, 1.65 g) and the mixture refluxed overnight. After cooling to ambient temperature the mixture was poured into 0.1 N HCl (100 ml) and extracted with ethyl acetate (3x). After drying with $Na_2SO_4$ the solvent was removed in vacuo to yield 0.29 g purple solid. MS (CI/$NH_3$) m/e 225 (M+). $^1H$ NMR ($CDCl_3$, 300 MHz) δ 3.70 (s, 3H), 5.65 (d, 1H), 5.85 (dd, 1H), 6.11 (dd, 1H), 6.64 (m, 3H), 7.08 (br s, 1H), 7.78 (br s, 1H).

Example 8e

4-Carboxylic acid-[3,4,5,6-c,d]-indole

To a solution of example 8d (0.10 g, 0.44 mmol) in methanol (5 ml) at ambient temperature was added 0.05 N KOH (10.67 ml). The solution was refluxed overnight and then poured into ethyl acetate (100 ml) and water (100 ml). The aqueous phase was separated, acidified to pH 3-4 with aqueous 10% $H_2SO_4$, and extracted with ethyl acetate (3x). After drying with $Na_2SO_4$ the solvent was removed in vacuo to yield 0.086 g purple solid. MS(EI) m/e 211 ($M^+$).

Example 8f

4-Carboxamide-[3,4,5,6-c,d]-indolyl)-Leu-Asp-Phe-$NH_2$

To a solution of example 8e (0.05 g, 0.23 mmol) in THF (3 ml) at -20 °C were added N-methylmorpholine (0.024 g, 0.23 mmol) and isobutyl chloroformate (0.033 g, 0.23 mmol). After 10 min. the reaction was warmed to 0 °C and Leu-Asp-Phe-$NH_2$ (0.097 g, 0.23 mmol) in cold (0 °C) DMF/$H_2O$ (80.20 v/v, 3 ml) was added dropwise. The reaction was stirred overnight with warming to ambient temperature. The mixture was poured into water (50 ml) and extracted with ethyl acetate (3x). After drying with $Na_2SO_4$ the solvent was removed in vacuo and the residue chromatographed on silica gel using ethyl acetate/pyridine/acetic acid/water (31.4/4.0./1.2/2.2) to yield 0.018 g off white solid. MS(CI/$NH_3$) m/e 586 $(M+H)^+$. $^1$H NMR(DMSO-$d_6$,300 MHz) δ 0.80 (m, 6H), 1.30 (m, 1H), 1.49 (m, 1H), 2.40 (m, 1H), 2.59 (m, 1H), 2.78 (m, 1H), 2.98 (m, 1H), 4.17 (m, 1H), 4.29 (m, 1H), 4.42 (m, 1H), 5.48 (m, 2H), 5.95 (d, 1H), 6.51 (m, 3H), 6.84 (s, 1H), 7.16 (m, 7H), 7.78 (m, 2H), 8.16 (m, 1H), 10.88 (br s, 1H).

Example 9

Boc-Trp-Leu-[β-1(2)H-tetrazol-5-yl]-L-alanyl-Phe-$NH_2$

Example 9a

N-tert-Butyloxycarbonyl-L-β-cyanoalanyl-Phe-$NH_2$

To a solution of N-Boc-β-cyanoalanine (1.68 g, 7.85 mmol) in THF (100 ml) at -20 °C was added N-methylmorpholine (NMM) (0.79 g, 7.80 mmol) and isobutyl chloroformate (1.07 g,7.80 mmol). After 0.25 h a solution of L-Phe-$NH_2$ (1.56 g, 7.85 mmol) and NMM (0.79 g, 7.80 mmol) in DMF (20 ml) cooled to 0 °C was added dropwise. After stirring at -20 °C for 1 h the reaction was warmed to 0 °C and then overnight with warming to ambient temperature. The solvent was removed in vacuo and the residue recrystallized from isopropanol to yield 1.78 g as a white crystalline solid. $^1$H NMR (DMSO-$d_6$, 300 MHz) δ 1.38 (s, 9H), 2.40 (m, 1H), 2.78 (m, 2H), 2.95 (dd, 1H), 4.23 (m, 1H), 4.32 (m, 1H), 7.15 (m, 5H), 7.29 (d, 1H), 7.47 (br s, 1H), 7.95 (d,1H).

Example 9b

Boc-[β-1(2)H-tetrazol-5-yl]-L-alanyl-Phe-$NH_2$

The procedure of J.S. Morley J. Chem.Soc. (C), 809-813, (1968) was employed. A mixture of example 9a (1.0 g, 2.77 mmol), sodium azide (0.40 g, 6.10 mmol) and ammonium chloride (0.33 g, 6.10 mmol) in DMF (5 ml) was heated at 95 °C for 24 h under $N_2$. The solvent was removed in vacuo and the residue triturated with cold 0.1 N HCl. The precipitate was collected and dissolved in dilute $NH_4OH$ (10 ml) and filtered, The aqueous solution was acidified with 4N HCl and the resulting precipitate filtered to yield 0.24 g yellow solid. MS(EI) m/e 403 $(M^+)$. 1H NMR (DMSO-$d_6$,300 MHz) δ 1.30 (s, 9H), 2.77 (m, 1H), 2.98 (m, 2H), 3.15 (dd, 1H), 4.32 (m, 2H), 7.10 (m, 7H), 7.52 (br s, 1H), 7.91 (d, 1H).

Example 9c

Boc-Leu-[β-1(2)H-tetrazol-5-yl]-L-alanyl-Phe-$NH_2$

To a suspension of example 9b (0.30 g, 0.74 mmol) in methylene chloride (5 ml) cooled to 0 °C was added trifluoroacetic acid (5 ml). The resulting homogeneous solution was stirred for 0.3 h. and the solvent removed under vacuo to yield 0.30 g brown oil that was used without further purification. To the oil (0.3 g, 0.72 mmol) suspended in ethyl acetate/methylene chloride (1:1 v/v, 5 ml) at ambient temperature was added Boc-Leu-N-hydroxysuccinimide ester (0.24 g, 0.72 mmol) and N,N-diisopropylethyl amine (0.09 g, 0.72 mmol). The reaction was stirred overnight at ambient temperature and the solvent removed in vacuo. The residue was dissolved in ethyl acetate (20 ml) and washed with aqueous solutions of 0.1 N HCl (3x) and saturated brine (3x). After drying with $Na_2SO_4$ the solvent was removed in vacuo and the residue triturated with ethyl acetate/ diethyl ether to yield 0.14 g white solid. MS(FAB+) m/e 517 $(M+H)^+$. $^1H$ NMR-(DMSO-$d_6$, 300 MHz) δ 0.79 (m, 6H), 1.23 (m, 2H), 1.33 (s, 9H), 1.46 (m, 1H), 2.72 (m, 1H), 3.02 (m, 1H), 3.18-3.52 (m, 2H), 3.85 (m, 1H), 4.13 (m, 1H), 4.61 (m, 1H), 6.95 (d, 1H), 7.14 (m, 7H), 7.56 (br s, 1H), 8.03 (d, 1H), 8.13 (d, 1H).

Example 9d

Boc-Trp-Leu-[β-1(2)H-tetrazol-5-yl]-L-alanyl-Phe-$NH_2$

To a solution of example 9c (0.12 g, 0.21 mmol) in acetic acid (2 ml) was added 1.45N HCl/ acetic acid (2 ml). The mixture was allowed to stand at ambient temperature for 0.5 h. and the solvent was removed in vacuo to yield 0.093 g white solid that was sufficient for use without further purification. To the solid (0.093 g, 0.20 mmol) in DMF (2 ml) at ambient temperature were added N,N-diisopropylethyl amine (0.027 g, 0.20 mmol), and Boc-Trp-N-hydroxysuccinimide ester (0.083g, 0.21 mmol). After stirring 1 h, the reaction was diluted with ethyl acetate (50 ml) and washed with an aqueous solution of 0.1N HCl (3x). After drying with $Na_2SO_4$ the solvent was removed in vacuo and the residue trituated with ethyl acetate/diethyl ether to yield 0.10 g white solid. MS(FAB+) m/e 703. $^1H$ NMR(DMSO-$d_6$,300 MHz) δ 0.80 (m, 6H), 1.28 (s, 9H), 1.31 (m, 2H), 1.53 (m, 2H), 2.78 (m, 1H), 3.02 (m, 2H), 3.16 (m, 2H), 4.16 (m, 1H), 4.24 (m, 2H), 4.59 (m, 1H), 6.77 (d, 1H), 6.92 (t, 1H), 7.01 (t, 1H), 7.11 (m, 8H), 7.57 (d, 1H), 7.78 (br s, 1H), 7.87 (m, 1H), 8.16 (d, 1H), 8.22 (d, 1H), 10.82 (br s, 1H). Anal calcd for $C_{35}H_{46}N_{10}O_6.1.5H_2O$: C 57.58, H 6.77, N 19.19; Found: C 57.50, H 6.39, H 18.97.

Example 10

D-Ctp-Leu-Asp-Phe-$NH_2$

Example 10a

Bromoactetyl-D-Trp-OBn

To a solution of D-Trp-OBn hydrochloride (9.30 g, 28.11 mmol) and triethylamine (6.11 g, 60.40 mmol) in absolute ethanol (200 ml) cooled to 0 °C was added bromoacetyl chloride (5.31 g, 33.70 mmol) dropwise over 10 min. The reaction mixture was stirred overnight with warming to ambient temperature. The solvent was removed in vacuo and the residue partitioned between aqueous 0.1 N HCl (250 ml) and ethyl acetate (250 ml). After drying with $Na_2SO_4$, the organic layer was concentrated in vacuo and the residue

chromatographed (ethyl actetate/hexanes) to yield 4.60 g of a clear colorless oil.

Example 10b

D-Ctp-OBn

A solution of Example 10a (4.0 g, 9.66 mmol) in 40% ethanol/water (1 l) at 80 °C was irratiated (Hanovia 450W. Hg lamp/ vycor filter) for 3 h. The resulting mixture was concentrated in vacuo to 600 ml and the aqueous layer extracted with ethyl acetate (3x). After drying with $Na_2SO_4$, the solvent was removed in vacuo and the residue chromatographed (ethyl acetate) to yield a mixture of 2-and 4-substituted cyclized products. The mixture was recrystallized (ethyl acetate/diethyl ether/ hexanes) to yield 0.0.12 g white crystalline solid. $^1H$ NMR (DMSO-$d_6$,300 MHz) δ 3.42 (m, 3H), 4.10 (br d, 1H), 4.30 (br s, 1H), 5.25 (s, 2H), 6.75 (d, 1H), 6.94 (t, 1H), 7.12 (m, 3H), 7.30 (m, 5H).

Example 10c

D-Ctp-Leu-Asp(OBn)-Phe-$NH_2$

A mixture of Example 10b (10 g, 0.29 mmol) and 5% Pd/$BaSO_4$ (0.02 g) in methanol (10 ml) was hydrogenated under one atmosphere of hydrogen at ambient temperature for 0.5 h. The catalyst was filtered and the solvent removed in vacuo to yield 0.063 g of free acid (D-CTP-OH) as a pink solid sufficient for use without further purification. To the acid (0.063 g, 0.26 mmol) in DMF (5 ml) cooled to 0 °C were added Leu-Asp(OBn)-Phe-$NH_2$ hydrochloride (0.13 g, 0.26 mmol), diphenyl phosphoryl azide (0.085 g, 0.30 mmol) (DPPA), and N-methyl morpholine (0.057 g, 0.56 mmol). The mixture was stirred overnight with warming to ambient temperature. The solvent was concentrated to 2 ml and the residue dissolved in ethyl acetate (50 ml). The organic layer was washed with aqueous solutions of saturated $NaHCO_3$ (3x) and 0.1N HCl (3x). After drying with $Na_2SO_4$, the solvent was removed in vacuo. The residue was triturated from ethyl acetate/hexanes to yield 0.07 g white solid. MS(FAB+) m/e 709 $(M+H)^+$. $^1H$ NMR (DMSO-$d_6$, 300 MHz) δ 0.90 (m, 6H), 1.35-1.70 (m, 3H), 2.55 (m, 1H), 2.78 (m, 2H), 2.95 (m, 1H), 3.10-3.55 (m, 2H), 4.10-4.65 (m, 6H), 5.09 (s, 2H), 6.75 (d, 1H), 6.73 (m, 2H), 7.14 (m, 14H), 7.83 (d, 1H), 8.31 (d, 1H), 8.43 (d, 1H), 10.93 (br s, 1H).

Example 10d

D-Ctp-Leu-Asp-Phe-$NH_2$

A mixture of example 10c (0.04 g, 0.056 mol) and 5% Pd/$BaSO_4$ (0.01 g) in acetic acid (5 ml) was hydrogenated under one atmosphere hydrogen at ambient temperature overnight. The solvent was filtered twice through celite and removed in vacuo. The residue was suspended in water (1 ml) and lyopholized to yield .028 g MS (FAB+) m/e 619 $(M+H)^+$. $^1H$ NMR (DMSO-$d_6$,300 MHz) δ 0.78 (m, 6H), 1.30 (m, 2H), 1.55 (m, 1H), 2.35-3.60 (m, 7H), 4.15 (m, 2H), 4.33 (m, 2H), 4.49 (m, 1H), 6.75 (d, 1H), 6.92 (m, 2H), 7.11 (m, 12H), 7.84 (d, 1H), 8.28 (d, 1H), 8.36 (d, 1H), 10.90 (br s, 1H), 12.35 (br s, 1H).

Example 11

3-(3-Quinolyl)-propionic-Leu-Asp-Phe-$NH_2$

Example 11a

3-(3-Quinolyl)-propionic acid 2,4,5-trichlorophenyl ester

To a soution of quinoline 3-carboxaldehyde (490 mg, 3.1 mmol) in THF (10 ml) was added (carboethoxymethylene) triphenylphosphorane and then the reaction was left at ambient temperature for two days. The solvent was evaporated in vacuo and the residue chromatographed on silica gel (ethyl acetate/hexanes 1:2) to afford a white solid which was then dissolved in a mixture of ethyl acetate-methanol and 10% Pd/C was added. The reaction mixture was vigorously stirred under 1 atomosphere of hydrogen for 2.5 h after which time the catalyst was removed by filtration through celite and the solvents evaporated to afford an orange oil. This oil was dissolved in a mixture of 1N sodium hydroxide/THF/methanol and left at ambient temperature for 1 h. The reaction mixture was acidified with 1N hydrochloric acid and the product extracted with ethyl acetate. Evaporation of the solvents in vacuo gave 200 mg of a yellow solid which was dissolved in a mixture of ethyl acetate, methylene chloride, and THF. To the solution was added DCC (205 mg, 1 mmol) and 2,4,5-trichlorophenol (200 mg, 1 mmol) and the reaction mixture was stirred at room temperature for 2 h. The solvents were evaporated in vacuo and the product purified by chromatography on silica gel (ethyl acetate/hexanes 2:3) to afford 160 mg of a colorless oil; MS (EI) m/e 379 $(M)^{+}$. $^1H$ NMR ($CDCl_3$, 60 MHz) $\delta$ 2.8-3.5 (m, 4H), 7.1 (s, 1H), 7.4 (s, 1H), 7.5-8.2 (m, 4H), 8.8 (d, 1H, J=2.5 Hz)

Example 11b

3-(3-Quinolyl)propionic-Leu-Asp-Phe-$NH_2$

To a solution of Leu-Asp-Phe$NH_2$ (157 mg, 0.4 mmol) in DMF (2 ml) was added diisopropylethylamine (156 mg, 1.2 mmol) and the active ester of example 11a (155 mg, 0.41 mmol). The reaction mixture was left at ambient temperature overnight and then it was diluted with water, made acidic with 1N hydrochloric acid, and then lyophilized. The white foamy solid residue was recrystalized from aqueous ethanol to afford 125 mg of a white solid mp 235-237 °C (dec.). MS (FAB+) m/e 576 $(M+H)^{+}$. $^1H$ NMR (DMSO-$d_6$, $D_2O$, 300 MHz) $\delta$ 0.54 (d, 3H, J=6.3 Hz), 0.62 (d, 3H, J=6.3 Hz), 1.0-1.4 (m, 3H), 2.4-2.7 (m, 4H), 2.82 (dd, 1H, J=14.5, 9.0 Hz), 2.97-3.10 (m, 3H), 3.7 (HOD), 4.17 (dd, 1H, J=10.5, 4.8 Hz), 4.33 (dd, 1H, J=9, 4.5 Hz), 4.46 (dd, 1H, J=6.3, 6.3 Hz), 7.1-7.3 (m, 5H), 7.58 (m, 1H), 7.72 (m, 1H), 7.89 (d, 1H), 7.98 (d, 1H, J=8.4 Hz), 8.10 (d, 1H), 8.75 (d, 1H, J=3 Hz). Anal calcd for $C_{31}H_{37}N_5O_6 \cdot 1.5H_2O$: C 61.77, H 6.69, N 11.62; Found: C 61.79, H 6.59, H 11.68.

Example 12

3-(2-Naphthyl)propionyl-Leu-Asp-Phe-$NH_2$

Example 12a

3-(2-Naphthyl)-propionic acid 2,4,5-trichlorophenyl ester

2-Naphthaldehyde (388 mg, 2.43 mmol) was reacted in a similar fashion as that described in example 11a to afford 355 mg of a colorless oil. $^1$H NMR ($CDCl_3$, 300 MHz) δ 3.03 (t, 2H, J=7.0 Hz), 3.25 (t, 2H, J=7.0 Hz), 7.15 (s, 1H), 7.35-7.51 (m, 3H), 7.52 (s, 1H), 7.70 (br s, 1H), 7.78-7.84 (m, 3H).

Example 12b

3-(2-Naphthyl)-propionyl-Leu-Asp-Phe-$NH_2$

To a solution of the hydrochloride salt of Leu-Asp-Phe$NH_2$ (73 mg, 0.17 mmol) in DMF (1 ml) at 4 °C was added diisopropylethylamine (54 mg, 0.42 mmol) and the active ester of example 12a (65 mg, 0.17 mmol). The reaction mixture was stored at 0 °C for five days after which time it was diluted with water and the precipitate collected by suction filtration. The solid was purified by column chromatography on silica gel using ethyl acetate/pyridine/acetic acid/water (62/10/3/5.5). The solvents were evaporated in vacuo and the residue lyophilized to yield 45 mg of a white solid; mp 224-226 °C (dec.); $[\alpha]^{24}$D -30.8° (c=0.5, DMF). $^1$H NMR (DMSO-$d_6$, 300 MHz) δ 0.67 (d, 3H, J=7 Hz), 0.71 (d, 3H, J=7 Hz, 1.23-1.40 (m, 3H), 2.35-2.68 (masked m, 4H), 2.83 (dd, 1H, J=14.5, 9.3 Hz), 2.93-3.08 (m, 3H), 3.33 ($H_20$), 4.23 (m, 1H), 4.32 (ddd, 1H, J=9, 9, 4.5 Hz), 4.45 (dd, J=13.5, 6.6 Hz), 7.10-7.27 (m, 7H), 7.31 (br s, 1H, 7.37 (dd, 1H, J=9.6, 1.1 Hz), 7.4-7.5 (m, 2H), 7.66 (br s, 1H), 7.77-7.9 (m, 4H), 8.04 (d, 1H, J=7.5 Hz), 8.27 (d, 1H, J=7.5 Hz). Anal calcd for $C_{32}H_{38}N_4O_6.0.5H_2O$: C 65.85, H 6.73, N 9.59; Found: C 65.60, H 6.34, H 9.37

Example 13

3-(9-Phenanthryl)propionyl-Leu-Asp-Phe-$NH_2$

Example 13a

3-(9-Phenanthryl)propionic acid 2,4,5-trichlorophenyl ester

Phenanthrene-9-carboxaldehyde (760 mg, 3.68 mmol) was reacted in a similar fashion as that described in example 11a to afford 547 mg of a white solid. $^1$H NMR ($CDCl_3$, 60 MHz) δ 2.8-3.3 (m, 2H), 3.3-3.8 (m, 2H), 7.0-7.2 (m, 2H), 7.3-8.2 (m, 7H), 8.4-8.8 (m, 2H).

Example 13b

3-(9-Phenanthryl)propionyl-Leu-Asp-Phe-$NH_2$

To a solution of Leu-Asp-Phe$NH_2$ (137 mg, 0.35 mmol) and diispropylethylamine (90 mg, 0.70 mmol) in DMF was added the active ester of example 13a (143 mg, 0.35 mmol) and the reaction mixture was stirred overnight at ambient temperature. The mixture was diluted with dilute hydrochloroic acid and the solvent

removed in vacuo. The solid residue was triturated with hot aqueous ethanol and the remaining precipitate collected by suction filtration. The precipitate was dissolved in a small volume of DMF, diluted with water, and then lyophilized to afford 140 mg of a white solid; mp 244-246 °C (dec.); MS (FAB+) m/e 625 (M+H)-$^+$. $^1$H NMR (DMSO-$d_6$, 300 MHz) δ 0.56-0.9 (m, 6H), 1.2-1.6 (m, 3H), 2.48 (masked dd, 1H), 2.55-2.75 (m, 3H), 2.85 (dd, 1H, J=14.5, 9.2 Hz), 3.03 (dd, 1H, J=14, 4.5 Hz), 3.72 ($H_2O$), 4.2-4.4 (m, 2H), 4.48 (m, 1H), 7.13-7.3 (m, 8H), 7.52-7.72 (m, 4H), 7.81 (d, 1H, J=8.5 Hz), 7.87 (m, 1H), 8.09-8.18 (m, 2H), 8.27 (d, 1H, J=7.4 Hz), 8.78 (m, 1H), 8.87 (m, 1H). Anal calcd for $C_{36}H_{40}N_4O_6.0.75H_2O$: C 67.74, H 6.55, N 8.78; Found: C 67.74, H 6.52, H 8.67.

## Example 14

### 3-Carboxy-2(S)-[3(R)-(Boc-Tryptophan carboxamide)-2-oxo]pyrrolidinyl-propionic acid phenylalanine amide

## Example 14a

### Boc-D-aspartic acid-β(2,5 dimethylpyrazole amide)-α benzyl ester

To a solution of Boc-D-Asp(OBn) (750 mg, 2.2 mmol) and N-methylmorpholine (682 mg, 2.6 mmol) in THF (15 ml) at 0 °C was added isobutylchloroformate (314 mg, 2.3 mmol) dropwise with stirring. After the addition was complete the reaction mixture was stirred at 0 °C an additional 10 min the 2,5-dimethylpyrazole (240 mg, 2.6 mmol) was added all in one portion. Stirring was continued for 10 min then the reaction was quenched by the addition of water. The organics were extracted with ethyl acetate (3x); the organic phase was washed with 1N hydrochloric acid, saturated aqueous sodium bicarbonate, and finally brine. The organic phase was dried ($Na_2SO_4$) and concentrated in vacuo to afford a colorless oil which was carried on without characterization.

## Example 14b

### 2-(R)-((Tert-butyloxycarbonyl)amino)-4-oxo-butyric acid benzyl ester

The aldehyde was prepared via a modification of the procedure reported by R. Nishizawa et. al. [ J. Med. Chem. , 20 , 510 (1977)]. To a solution of the crude product prepared in example 14a (218 mg, 0.57 mmol) in anhydrous THF (25 ml) at -78 °C under nitrogen was added a 1M THF solution of $LiAlH_4$ (190 µl, 1.9 mmol) and the reaction was maintained at -78 °C for 30 min. The reaction was quenched at -78 °C by the rapid addition of 1N hydrochloric acid (4 ml) and then the mixture was warmed to ambient temperature. The product was extracted with ethyl acetate and the organic phase was dried ($Na_2SO_4$) and then concentrated under reduced pressure to give an oily residue which was purified by flash chromatography (ethyl acetate/hexanes 1:2) to yield 76 mg of a colorless oil. $^1$H NMR ($CDCl_3$, 60 MHz) δ 1.4 (s, 9H), 2.9 (m, 2H), 4.3 (m, 1H), 5.1 (s, 2H), 7.3 (s, 5H), 9.6 (s, 1H).

## Example 14c

### 3-Carboxy-[3(R)-((tert-butyloxycarbonyl)amino)-2-oxo]-1-pyrrolidinyl-2(S)-propoionic acid phenylalanine amide

To a solution of example 14b (45 mg, 0.21 mmol) and Asp-Phe-$NH_2$ (58 mg, 0.21 mmol) in ethanol at ambient temperature was added sodium cyanoborohydride (32 mg, 0.52 mmol) and the reaction mixture was warmed to 40 °C for 48 h. After cooling to room temperature, the solvent was evaporated in vacuo and the residue purified by column chromatography on silica gel using ethyl acetate/pyridine/acetic acid/water (78:10:3:5.5). The solvents were removed in vacuo and the residue lyophilized to yield 35 mg of a white solid; mp 144-146 °C; $[\alpha]^{26}D$ -38.7° (c=0.5, ethanol); MS (FAB+) m/e 463 $(M+H)^+$. $^1H$ NMR (DMSO-$d_6$, 300 MHz) δ 1.38 (s, 9H), 2.26 (dd, 1H, J=16.5, 6 Hz), 2.30 (dd, 1H, J=16.5, 7 Hz), 2.48 (masked m, 1H), 2.6-2.84 (m, 3H), 3.57 (dd, 1H, J=14, 5 Hz), 3.35 ($H_2O$), 4.25 (m, 1H), 4.44 (m, 1H), 4.82 (t, 1H, J=7 Hz), 6.55 (br s, 1H), 7.12 (br s, 1H), 7.13-7.28 (m, 5H), 7.32 (d, 1H. J=7.5 Hz), 8.09 (d, 1H, J=8.4 Hz).

## Example 14d

### 3-Carboxy-2(S)-[3(R)-(Boc-Tryptophan carboxamide)-2-oxo]pyrrolidinyl-propionic acid phenylalanine amide

To a solution of example 14c (26 mg, 0.056 mmol) in methylene chloride (2 ml) at 0 °C was added trifluoroacetic acid (2 ml) and the reaction mixture was maintained at 0 °C for 2.5 h. After diluting with toluene (4 ml), the solvents were evaporated in vacuo and the solid residue kept under high vacuum over night. The solid was dissolved in DMF (2 ml) to which were added diispropylethyl amine (29 mg, 0.22 mmol) and Boc-Trp N-hydroxysuccinimide ester (34 mg, 0.08 mmol) and the reaction mixture was left at ambient temperature over night. The DMF was evaporated in vacuo and the residue chromatographed on silica gel using ethyl acetate/pyridine/acetic acid/water (78:10:3:5.5). The solvents were evaporated and the residue diluted with water and lyophilized. The foamy solid was dissolved in ethyl acetate and washed with 0.25N hydrochloric acid. The solvent was evaporated in vacuo and the residue dissolved in acetone, diluted with water, and lyophilized to yield 10 mg of a white solid; mp 145-148 °C; MS (FAB+) m/e 649 $(M+H)^+$. $^1H$ NMR (DMSO-$d_6$, $D_2O$ 300 MHz) δ 1.25 (s, 9H), 1.89 (dd, 1H, J=16.5, 6 Hz), 2.26 (dd, 1H, J=16.5, 6 Hz), 2.40 (dd, 1H, J=16.5, 8.4 Hz), 2.57-5.83 (m, 3H), 2.86 (dd, 1H, J=14.4, 8.4 Hz), 2.92-3.10 (m, 2H), 3.40 (m, 1H), 3.65 (HOD), 4.10-4.25 (m, 2H), 4.38 (dd, 1H, J=9.6, 4.5 Hz) 4.53 (m, 1H), 6.94 (dd, 1H, 7.8, 7.8 Hz), 7.05 (dd, 1H. 7.8, 7.8 Hz), 7.09 (s, 1H), 7.10-7.25 (m, 5H), 7.32 (d, 1H, J=7.8 Hz), 7.52 (d, 1H, J=7.8 Hz). Anal calcd for $C_{33}H_{40}N_6O_8 \cdot 1.75H_2O$: C 58.26, H 6.44, N 12.35; Found: C 58.26, H 6.44, H 12.35.

## Example 15

### Cbz-Trp-(NMe)Leu-Asp-Phe-$NH_2$

## Example 15a

### Asp(OtBu)-Phe-$NH_2$

To a suspension of Cbz-Asp(OtBu) (1.313 g, 4.0 mmol) in THF (50 ml) and DMF (20 ml) at -10 °C under nitrogen were added N-methylmorpholine (415 mg, 4.1 mmol) and isobutylchloroformate (560 mg, 4.1 mmol). The suspension was stirred at -10 °C for 15 min then phenylalanine amide in a mixture of DMF (5 ml) and water (2 ml) was added all in one portion. Stirring was continued as the reaction was allowed to gradually warm to ambient temperature overnight. The reaction mixture was concentrated in vacuo and the

residue diluted with ethyl acetate and then washed with 1N hydrochloric acid (3x), satured aqueous sodium bicarbonate (3x), and brine (1x). The organic phase was dried ($NaSO_4$) and then the solvent evaporated in vacuo. The residual solid was recrystalized from hot ethyl acetate-hexane to afford 770 mg of a white solid which was dissolved in methanol (20 ml). To this solution was added 10% Pd/C (50 mg) and the mixture was stirred vigorously under one atmosphere of hydrogen for 1 h. The catalyst was removed by filtration and the volatiles evaporated in vacuo to give 500 mg of an oil; MS (FAB+) m/e 336 $(M+H)^+$. $^1H$ NMR ($DMSO-d_6$, 300 MHz) $\delta$ 1.38 (s, 9H), 1.85 (br s, 1H), 2.17 (dd, 1H, J=16, 8 Hz), 2.45 (dd, 1H, J=16, 5 Hz), 2.85 (dd, 1H, J=14, 9 Hz), 3.03 (dd, 1H, J=14, 5 Hz), 3.3 ($H_2O$), 4.42 (m, 1H), 7.1-7.3 (m, 5H), 7.46 (s, 1H), 8.05 (d, 1H, J=9 Hz).

## Example 15b

### Cbz-Trp-(NMe)Leu-Asp(OtBu)-Phe-$NH_2$

To a solution of Cbz-(NMe)Leu-OH (160 mg, 0.57 mmol) and the dipeptide in example 15a (192 mg, 0.57 mmol), in methylene chloride (3 ml) were added diisopropylethyl amine (155 mg, 1.2 mmol) and bis(2-oxo-3-oxazolidinyl)phosphinic chloride (BOP-Cl) (160 mg, 0.63 mmol). The reaction mixture was stirred at ambient temperature for three days. The solution was washed with saturated aqueous sodium bicarbonate and brine, then 1N hydrochloric acid and brine. The organic phase was dried ($Na_2SO_4$) and then the solvent was evaporated in vacuo. The residue was purified by chromatography on silica gel (ethyl acetate/hexanes 3:1) to afford 150 mg of an oil which was dissolved in methanol (5 ml) and 10% Pd/C (20 mg) was added. The reaction mixture was stirred vigorously under one atmosphere of hydrogen for 1 h. The catalyst was removed by passage through filter paper and the supernatent was concentrated in vacuo. The residue was azeotroped with toluene to afford a white solid which was then dissolved in methylene chloride and diisopropylethyl amine (41 mg, 0.32 mmol), Cbz-Trp (54 mg, 0.16 mmol), and BOP-Cl (43 mg, 0.17 mmol) were added. The reaction mixture was stirred at ambient temperature overnight and then it was diluted with methylene chloride, washed with 1N hydrochloric acid, saturated aqueous sodium bicarbonate, and brine. The organic phase was dried ($NaSO_4$) and the solvents evaporated in vacuo to give an oily residue which was purified by column chromatography on silica gel (ethyl acetate) to yield 61 mg of a white foam which was carried on without characterization.

## Example 15c

### Cbz-Trp-(NMe)Leu-Asp-Phe-$NH_2$

To a solution of the tetrapeptide of example 15b (61 mg, 0.08 mmol) in methylene chloride (1 ml) at 0 °C was added anisole (50 mg, 0.46 mmol) and trifluoroacetic acid (1 ml) and the reaction mixture was stored at 4 °C for two days. The reaction mixture was diluted with toluene and then the solvents were evaporated in vacuo. The residue was purified by column chromatography on silica gel (ethyl acetate/pyridine/acetic acid/water, 15:2:0.6:1.1). The product fractions were pooled, diluted with water, and then lyophilized to yield 38 mg of a white solid; mp 107-109 °C; MS (FAB-) m/e 725 $(M-H)^-$. $^1H$ NMR ($DMSO-d_6$, $D_2O$ 300 MHz) $\delta$ 0.42 (d, 0.5H, J=6.6 Hz), 0.48 (d, 0.5H, J=6.6 Hz), 0.78 (d, 2.5H, J=6.6 Hz), 0.85 (d, 2.5H, J=6.6 Hz), 1.25-1.55 (m, 3H), 2.45-2.7 (masked m) 2.75-3.15 (m, 5H), 3.7 (HOD), 4.37 (d, 1H, J=14 Hz), 4.96-5.17 (m, 2H), 6.99 (t, 1H, J=7.5 Hz), 7.08 (t, 1H, J=7.5 Hz), 7.13-7.4 (m, 13H), 7.57 (d, 1H, J=7.5 Hz). Anal calcd for $C_{39}H_{46}N_6O_8 \cdot 1.25H_2O$: C 62.51, H 6.52, N 11.21; Found: C 62.74, H 6.33, N 10.84.

## Example 16

N-(4-Hydroxycinnamoyl)-Trp-Met-Asp-Phe-$NH_2$

Example 16a

4-Hydroxycinnamic Acid N-Hydroxysuccinimide Ester

To a solution of 4-hydroxycinnamic acid (1.0 gm, 6.09 mmol) and N-hydroxysuccinimde (0.72 gm, 6.09 mmol) in 45 ml tetrahydrofuran (THF) chilled in an ice bath was added dicyclohexylcarbodiimide (DCC) (1.22 gm, 5.91 mmol). The flask was capped with a drierite filled drying tube and allowed to stir approximately 60 h while slowly warming to room temperature. The reaction mixture was subsequently cooled in an ice bath and vacuum filtered. The filtrate was concentrated in vacuo to yield the crude product which was purified by flash chromatography on silica gel (ethyl acetate-hexane) and recrystallization of relevant fractions from ethyl acetate-hexane to give 0.85 gm of a white solid. $^1$H NMR (Acetone-$d_6$, 300 MHz) $\delta$ 2.50 (s, 4H), 6.60 (d, J=16.5Hz, 1H), 6.94 (d, J=9Hz, 2H), 7.7 (d, J=9Hz, 2H), 7.87 (d, J=16. 5Hz, 1H). Anal.calc.for $C_{13}H_{11}NO_5$: C 59.76, H 4.25, N 5.36; Found: C 59.58, H 4.26, N 5.08. mp 199-203.5 °C (d).

Example 16b

N-(4-Hydroxycinnamoyl)-Trp-Met-Asp-Phe-$NH_2$

To a mixture of the active ester prepared in Example 16a (0.032gm, 0.12 mmol) and the hydrochloride salt of Trp-Met-Asp-Phe-$NH_2$ prepared according to Morley, et.al. J.Chem.Soc. 555 (1966) (0.080 gm, 0.12 mmol) in dimethylformamide (DMF) in an ice bath under a nitrogen atmosphere was added diisopropylethylamine (DIEA)(0.045 ml, 0.25 mmol). The mixture was allowed to stir overnight while slowly warming to room temperature. An additional 0.015 g, 0.06 mmol of the active ester was added followed by two drops of DIEA. After stirring an additional 4 h at room temperature the reaction was quenched by pouring into an ice cold 10% citric acid solution. The crude product was collected by vacuum filtration and purified by preparative reverse phase $C_{18}$ HPLC (Acetonitrile-50 mmol ammonium acetate, pH 4.5) to give after lyophilization 0.015 g of a white solid. MS( FAB+ ) m/e 743 ( M+H )$^+$. $^1$H NMR (DMSO-$d_6$ ) $\delta$ 1.7-1.96 (cm, 2H), 2.0 (s, 3H), 2.3-3.5 (cm, 8H), 4.33 (cm, 2H), 4.48 (cm, 1H), 4.68 (cm, 1H), 6.5 (d, J=16.5Hz, 1H), 6.77 (d, J=9Hz, 2H), 6.95 (t, 1H), 7.04 (t, 1H), 7.1-7.5 (cm, 14H), 7.62 (d, J=7.5Hz, 1H), 8.0 (cm, 1H), 8.23 (cm, 3H), 9.88 (br s, 1H), 10.84 (br s, 1H). Anal calc for $C_{38}H_{42}N_6O_8S \cdot 2H_2O$ : C 58.59, H 5.96, N 10.79; found: C 58.36, H 5.63, N 10.66. mp 214-216 °C (d)

Example 17

N-($\beta$-Naphthoyl)-(dehydro)Phe-Leu-Asp-Phe--$NH_2$

Example 17a

2-Naphthoic acid N-Hydroxysuccinimide Ester

To a solution of 2-Naphthoic acid (2 g, 11.62 mmol) and N-hydroxysuccinimide (1.34 g, 11.62 mmol) in 75 ml of a 2:1 v/v mixture of ethyl acetate-1,2-dimethoxyethane (glyme) which was chilled in an ice bath was added DCC (2.4 g, 11.62 mmol) in one portion. The flask was capped with a drierite filled drying tube and allowed to stir overnight while slowly warming to room temperature. The reaction mixture was subsequently filtered and the filtrate concentrated in vacuo. The residue was recrystallized from ethyl acetate-hexane to yield 2.73 g (two crops) of white crystals. $^1H$ NMR($CDCl_3$, 300 MHz) $\delta$ 2.95 (s, 4H), 7.5-7.75 (cm, 2H), 7.85-8.2 (cm, 4H), 8.76 (s, 1H). Anal calc for $C_{15}H_{11}NO_4$: C 66.90, H 4.12, N 5.20; found: C 66.87, H 4.13, N 5.17

Example 17b

2-$\beta$-Naphthyl-4-benzylidene-5(4H)-oxazolone

The product from example 17a (1.07 g, 3.98 mmol) was combined with glycine methyl ester hydrochloride (0.55 g, 4.38 mmol) in dimethylformamide (DMF) (10 ml) and the resulting solution cooled in an ice bath. To the cold solution was added DIEA (0.76 ml, 4.4 mmol) followed by removal of the ice bath. The flask was capped with a drierite filled drying tube and allowed to stir overnight. The reaction mixture was subsequently partitioned between ethyl acetate and water. The organic phase was washed with 10% HCl, water, dried with magnesium sulfate, filtered and concentrated in vacuo to give 0.91 g of solid crude product sufficient for use without further purification. The ester (0.91 g, 3.74 mmol) was dissolved in methanol (25 ml) and treated at room temperature with potassium hydroxide (0.7 g, 12 mmol) in water (10 ml). After one hour the reaction mixture was partitioned between ethyl acetate and dilute aqueous HCl. The organic phase was washed once with water, dried with magnesium sulfate, filtered and concentrated in vacuo to give 0.74 g of the crude product as a white solid sufficient for use without further purification. The acid (0.74 g, 3.23 mmol) was suspended in acetic anhydride (30 ml) to which was added sodium acetate trihydrate (1.3 g, 9.81 mmol) and benzaldehyde (0.33 ml, 3.27 mmol). The flask was capped with a water cooled condenser/drierite filled drying tube and the mixture heated on a steam bath for one hour. The reaction mixture was subsequently concentrated in vacuo and the residue partitioned between water and ethyl acetate. The organic phase was washed. with 5% sodium bicarbonate solution, water, dried with magnesium sulfate,filtered and concentrated in vacuo. The crude product was recrystallized from ethyl acetate-hexane to give 0.34 g of a bright yellow solid. An additional 0.09 g of the product was obtained from the mother liquor by flash chromatography on silica gel (ethyl acetate-hexane). $^1H$ NMR (DMSO-$d_6$,300 MHz) $\delta$ 7.6 (s, 1H), 7.5-7.8 (cm, 5H), 8.0-8.4 (cm, 6H), 8.75 (s, 1H). Anal calc for $C_{20}H_{13}NO_2$: C 80.24, H 4.39, N 4.68; found: C 80.43, H 4.51, N 4.35. mp 164-168.5 °C.

Example 17c

N-($\beta$-Naphthoyl)-(dehydro)Phe-Leu-Asp-Phe-$NH_2$

The azalactone from example 17b (0.336 g, 1.12 mmol) was combined with Leu-Asp-Phe-$NH_2$ prepared according to Kenner,G.W., et.al. J.Chem.Soc. (C),761 (1968) (0.44 g, 1.12 mmol) in DMF (15 ml) containing a catalytic amount of p-dimethylaminopyridine (DMAP) followed by the addition of DIEA (0.2 ml, 1.23 mmol) and heating, under nitrogen, to 55-60 °C. After 2.5 h the reaction mixture was partitioned between ethyl acetate and dilute aqueous HCl. The organic phase was washed once with water, dried (magnesium sulfate), filtered and concentrated in vacuo. The crude product was recrystallized from aqueous ethanol to give 0.637 g of a white crystalline solid. MS(FAB+) m/e 692 $(M+H)^+$. $^1H$ NMR (DMSO-$d_6$, 300 MHz) $\delta$ 0.9 (d, J=6Hz, 6H), 1.4-1.8 (cm, 3H), 2.55-3.1 (cm, 4H), 4.2-4.6 (cm, 3H), 7.0-7.24 (cm, 8H), 7.26-7.45 (cm, 3H), 7.6-7.75 (cm, 5H), 8.0-8.16 (cm, 4H), 8.2 (d, J=7.5Hz, 1H), 8.5 (d, J=7.5Hz, 1H), 8.7 (s, 1H), 10.45 (s, 1H),

12.47 (br s, 1H). Anal calc for $C_{39}H_{41}N_5O_7 \cdot 0.5H_2O$. C 66.83, H 6.05, N 9.99; found: C 66.77, H 6.02, N 9.86. mp 212.5-214 °C (d).

Example 18

N-(α-Naphthoyl)-(dehydro)Phe-Leu-Asp-Phe-$NH_2$

Example 18a

1-Naphthoic acid N-hydroxysuccinimide ester

The ester was prepared by coupling 1-naphthoic acid (2 g, 11.62 mmol) with N-hydroxysuccinimide (1.34 g, 11.62 mmol) as outlined in example 17a to yield 2.57 g of a white crystalline product. $^1H$ NMR ($CDCl_3$, 300 MHz) δ 2.95 (br s, 4H), 7.5-7.8 (cm, 3H), 7.93 (d, J=9Hz, 1H), 8.6 (d, J=7.5Hz, 1H), 8.46 (dd, J=7.5Hz, 1.5Hz, 1H), 8.81 (d, J=9Hz ,1H). Anal calc for $C_{15}H_{11}NO_4$: C 66.90, H 4.12, N 5.20; found: C 66.77, H 4.22, N 5.33.

Example 18b

2-α-Naphthyl-4-benzylidene-5(4H)-oxazolone

The product from example 18a (1.07 g, 3.98 mmol) was reacted with glycine methyl ester hydrochloride (0.55 g, 4.38 mmol) as in example 17b to yield a crude product, 1.0 g, which was subsequently treated with potassium hydroxide (0.7 g, 12 mmol) in methanol (25 ml) as in example 17b to give 0.75 g of the corresponding acid as a white crystalline solid. The azalactone was prepared by condensing the above acid (0.75 g, 3,27 mmol) with benzaldehyde (0.33 ml, 3.27 mmol) in the presence of sodium acetate trihydrate (1.38 g, 9.81 mmol) as in example 17b to yield after flash chromatography on silica gel (ethyl acetate-hexane) 0.346 g of a yellow crystalline solid. $^1H$ NMR (DMSO-$d_6$,300 MHz) δ 7.45 (s, 1H), 7.5-7.65 (cm, 3H), 7.65-7.8 (cm, 2H), 7.8-7.9 (t, 1H), 8.14 (d, J=7.5Hz, 1H), 8.25-8.4 (cm, 2H), 9.4 (d, J=9.0Hz, 1H). Anal calc for $C_{20}H_{13}NO_2$: C 80.24, H 4.39, N 4.68; found: C 80.29, H 4.48, N 4.55. mp 134-137 °C.

Example 18c

N-(α-Naphthoyl)-(dehydro)Phe-Leu-Asp-Phe-$NH_2$

The azalactone from example 18b (0.18 g, 0.60 mmol) was condensed with Leu-Asp-Phe-$NH_2$ (0.24 g, 0.60 mmol) as in example 17c to give 0.272 g of a crystalline product. MS (FAB+) m/e 692 (M+H)+, m/e 714 (M+Na)+. $^1H$ NMR (DMSO-$d_6$, 300 MHz) δ 0.92 (cm, 6H), 1.4-1.88 (cm, 3H), 2.45-3.1 (cm, 4H), 4.26-4.6 (cm, 3H), 7.04-7.29 (cm, 8H), 7.3-7.49 (cm, 3H), 7.5-7.7 (cm, 5H), 7.78-7.9 (t, 2H), 8.0 (d, J=7.5Hz, 1H), 8.08 (d, J=9Hz, 1H), 8.24-8.4 (cm, 3H), 10.3 (s, 1H), 12.4 (br s, 1H). Anal calc for $C_{39}H_{41}N_5O_7$: C 67.70, H 5.98, N 10.12; found: C 67.57, H 6.05, N 10.02.

Example 19

Boc-Trp-Leu-Asp-(dehydro)Phe-$NH_2$

Example 19a

Azalactone of Boc-(β-t-Butyl)Asp-β-Phenylserine

To a solution of Boc-(β-t-butyl)Aspartic acid (0.34 g, 1.17 mmol) in THF (20 ml) under a nitrogen atmosphere was added N-methylmorpholine (NMM) (0.13 ml, 1.17 mmol). The mixture was cooled to -10/-15 °C and isobutylchloroformate (IBCF) (0.15 ml, 1.17 mmol) added in one portion via syringe. After 7 min at low temperature the resulting suspension was warmed to 0/+5 °C and a previously prepared suspension of d, 1-β-Phenylserine hydrate (0.21 g, 1.17 mmol) in a total of 6 ml of water containing NMM (0.13 ml, 1.17 mmol) added dropwise over 2 min. The resulting clear solution was allowed to stir while slowly warming to room temperature. The reaction was quenched by pouring into 10% citric acid solution and the crude product recovered by extraction with ethyl acetate, dried (magnesium sulfate), filtered and concentrated in vacuo. Purification by flash chromatography on silica gel (ethyl acetate/hexane/acetic acid) then ethyl acetate/acetone/acetic acid) gave a foam (0.29 g). This material was subsequently dissolved in acetic anhydride and sodium acetate trihydrate (0.26 g, 1.91 mmol) added. The flask was capped with a drierite filled drying tube and the mixture stirred at room temperature for 6 h. The reaction mixture was concentrated in vacuo to dryness then partitioned between chloroform and 10% citric acid solution. The aqueous phase was extracted twice with chloroform. The combined extracts were dried (magnesium sulfate), filtered and concentrated in vacuo. The crude product was purified by flash chromatography on silica gel (ethyl acetate-hexane) to give 0.221 g of the pure azalactone as a foam. $^1H$ NMR (DMSO-$d_6$, 300 MHz) δ 1.4 (s, 18H), 2.65-3.0 (cm, 2H), 4.92 (q, 1H), 7.36 (s, 1H), 7.45-7.55 (cm, 3H), 7.6 (d, J=9Hz, 1H), 8.2 (cm, 2H).

Example 19b

Boc-(β-t-butyl)Asp-(dehydro)Phe-$NH_2$

The product from example 19a (0.029 g, 0.071 mmol) was dissolved in dioxane (2.5 ml) and treated at room temperature with concentrated ammonium hydroxide solution (2.5 ml). After 30 min the reaction was quenched by pouring into 10% citric acid solution. The crude product was recovered by extraction with chloroform, dried (magnesium sulfate), filtered and concentrated in vacuo. Purification by preparative thin-layer chromatography (silica gel-GF/1mm, ethyl acetate) gave the pure product (0.015 g). $^1H$ NMR (DMSO-$d_6$, 300 MHz) δ 1.4 (s, 18H), 2.4-2.8 (cm, 2H), 4.36 (q, 1H), 7.22 (s, 1H), 7.26-7.4 (cm, 5H), 7.57 (cm, 2H), 9.55 (s, 1H).

Example 19c

Boc-Leu-Asp-(dehydro)Phe-$NH_2$

The dipeptide amide as prepared in example 19b (0.127 g, 0.293 mmol) was treated with 5 ml of 1.5N HCl in glacial acetic acid. After one hour the reaction mixture was frozen and lyophilized to give in quantitative yield the fully deprotected dipeptide amide as the hydrochloride salt. This material (0.092 g, 0.293 mmol) was subsequently combined with Boc-Leu-OSu (0.098 g, 0.29 mmol) in DMF (5 ml) solution in an ice bath and treated with DIEA (0.11 ml, 0.645 mmol). The flask was capped with a drierite filled drying tube and allowed to stir overnight while slowly warming to room temperature. The reaction was quenched by pouring into aqueous citric acid and the crude product isolated by extraction with ethyl acetate followed by drying (magnesium sulfate), filtration and concentration in vacuo. Recrystallization from ethyl acetate gave 0.091 g of the pure compound. $^1$H NMR (DMSO-$d_6$, 300 MHz) δ 0.85 (t, 6H), 1.34 (s, 9H), 1.3-1.7 (cm, 3H), 2.55-2.9 (cm, 2H), 3.96 (cm, 1H), 4.57 (cm, 1H), 7.08 (d, J=6Hz, 1H), 7.15 (br s, 1H), 7.25-7.45 (cm, 5H), 7.55 (d, J=7.5Hz, 1H),8.47 (d, J=6Hz, 1H), 9.35 (s, 1H), 12.55 (br s, 1H). Anal calc for $C_{24}H_{34}N_4O_7 \cdot 0.5H_2O$: C 57.69, H 7.07, N 11.22; found: C 57.86, H 6.71, N 11.08. mp 178.5-180.5° C (d)

## Example 19d

### Boc-Trp-Leu-Asp-(dehydro)Phe-$NH_2$

The product from example 19c (0.084 g, 0.171 mmol) was treated with 5 ml 1.5N HCl in glacial acetic acid at room temperature. After one hour the reaction mixture was frozen and lyophilized to give in quantitative yield the deprotected tripeptide amide as the hydrochloride salt. This material (0.073 g, 0.171 mmol) was subsequently combined with Boc-Trp-OSu (0.069 g, 0.171 mmol) in DMF solution (2 ml) in an ice bath and treated with DIEA (0.066 ml, 0.376 mmol). The flask was capped with a drierite filled drying tube and allowed to stir overnight while slowly warming to room temperature. The reaction was quenched by pouring into ice cold aqueous citric acid and the crude product isolated by vacuum filtration. Recrystallization from aqueous ethanol gave 0.065 g of pure tetrapeptide. MS (FAB+) m/e 677 (M+H)+. $^1$H NMR (DMSO-$d_6$, 300 MHz) δ 0.85 (cm, 6H), 1.15 and 1.3 (s, 9H total), 1.4-1.75 (cm, 3H), 2.5-3.2 (cm, 4H), 4.2 (cm, 1H), 4.4 (cm, 1H), 4.6 (cm, 1H), 6.86 (d, J=7.5Hz, 1H), 6.97 (t, 1H), 7.06 (t, 1H), 7.12 (s, 1H), 7.17 (s, 1H), 7.27 (s, 1H),7.27-7.45 (cm, 5H), 7.55-7.65 (cm, 3H), 8.0 (d, J=7.5Hz, 1H), 8.52 (d, J=7.5Hz, 1H), 9.54 (s, 1H), 10.78 (s, 1H), 12.6 (br s, 1H) Anal Calc for $C_{35}H_{44}N_6O_8 \cdot H_2O$: C 60.49, H 6.69, N 12.08; found: C 60.16, H 6.26, N 11.74. mp 192-194° C (d).

## Example 20

### Boc-Trp-Leu-Asp-(m-nitro)Phe-$NH_2$

## Example 20a

### N-Acetylamino-(m-nitro)benzyl-diethylmalonate

To a solution of freshly prepared sodium ethoxide (0.1 g, 4.3 mmol sodium, 25 ml absolute ethanol) under nitrogen at room temperature was added solid N-acetylamino diethylmalonate (1 g, 4.60 mmol). After 25 min solid m-nitro-benzylbromide (1 g, 4.60 mmol) was added and the resulting mixture heated to reflux overnight. The crude product was isolated by partition of the reaction mixture between ethyl acetate and 10% citric acid solution. The organic phase was washed once with water, dried (magnesium sulfate), filtered and concentrated in vacuo. Purification by flash chromatography on silica gel (ethyl acetate-hexane) gave 0.58 g of a solid product. $^1$H NMR (DMSO-$d_6$, 300 MHz) δ 1.18 (t, 6H), 1.95 (s, 3H), 3.58 (s, 2H), 4.17 (cm,

4H), 7.46 (d, J=7.5Hz, 1H), 7.61 (t, 1H), 7.8 (s, 1H), 8.15 (d, 1H), 8.23 (s, 1H). Anal calc for $C_{16}H_{20}N_2O_7$: C 54.53, H 5.73, N 7.95; found: C 54.35, H 5.65, N 7.80. mp 156-157 °C.

## Example 20b

### Boc-m-nitro-Phenylalanine

The product from example 20a (0.061 g, 0.172 mmol) was suspended in 6N HCl (6 ml) and refluxed for 4 h. The reaction mixture was concentrated in vacuo, diluted with 1:1 v/v dioxane-water and made basic with sodium hydroxide. To this was added excess Boc-carbonate and the mixture allowed to stir overnight at room temperature. The reaction mixture was diluted with 10% citric acid solution and the crude product isolated by extraction with ethyl acetate, followed by drying (magnesium sulfate), filtration and concentration in vacuo. Purification by preparative tlc gave 0.038 g product as an amber colored glass. $^1H$ NMR (DMSO-$d_6$, 300 MHz) $\delta$ 1.28 (s, 9H), 2.85-3.3 (cm, 2H), 4.15 (cm, 1H), 7.18 (d, J=9Hz, 1H), 7.6 (t, 1H), 7.75 (d, J=7.5Hz, 1H), 8.1 (d, J=9Hz, 1H), 8.15 (s, 1H). Anal calc for $C_{14}H_{18}N_2O_6$: C 54.18, H 5.86, N 9.03; found: C 54.25, H 5.70, N 8.59. mp 117.7-118 5 °C.

## Example 20c

### Boc-m-nitro-Phenylalanine amide

The product from example 20b (1.7 g, 5.48 mmol) was dissolved in THF (50 ml) and treated with NMM (0.58 ml, 5.30 mmol). The resulting solution was cooled in an ice bath under a nitrogen atmosphere and to this cold solution was added ethyl chloroformate (0.46 ml, 4.82 mmol). After 10 min ammonia gas was slowly bubbled into the suspension for approximately 10 min. The flask was subsequently capped with a drierite filled drying tube and allowed to slowly warm to room temperature The crude product was isolated by concentration of the reaction mixture in vacuo, dilution with water and vacuum filtration. The solid product was dried in vacuo at room temperature. The product was purified by recrystallization from ethyl acetate-hexane. $^1H$ NMR (DMSO-$d_6$, 300 MHz) $\delta$ 1.25 (s, 9H), 2.75-3.2 (cm, 2H), 4.14 (cm, 1H), 6.48 (d, J=9Hz, 1H), 7.1 (s, 1H), 7.48 (s, 1H), 7.59 (t, 1H), 7.75 (d, J=7.5Hz, 1H), 8.08 (d, J=7.5Hz, 1H),8.2 (s, 1H). Anal calc for $C_{14}H_{19}N_3O_5$: C 54.35, H 6.20, N 13.59; found: C 54.35, H 6.24, N 13.51. mp 189-191.5 °C (d)

## Example 20d

### Boc-Asp($\beta$-tert-butyl)-(m-nitro)Phe-$NH_2$

The product from example 20c (0.98 g, 3.18 mmol) was treated with 16 ml of 1.5 N HCl in glacial acetic acid at room temperature. After 45 min anhydrous ether (100 ml) was added dropwise with vigorous stirring. The hydrochloride was collected by vacuum filtration to yield after drying in vacuo at room temperature 0.77 g of a white solid sufficiently pure for use as isolated. To a solution of Boc-Asp(b-t-butyl) ester (0.92 g, 3.18 mmol) in THF (50 ml) under nitrogen at room temperature was added NMM (0.35 ml, 3.18 mmol) and the resulting solution cooled to -10 °C. Isobutyl chloroformate (IBCF) (0.41 ml, 3.18 mmol) was added in two portions and the activation allowed to proceed 6 min before a previously prepared slurry of the above hydrochloride (0.77 g, 3.13 mmol) in a DMF (16 ml)/DMSO (0.25 ml) mixture containing NMM (0.35 ml, 3.18 mmol) was added dropwise over 2 min. The reaction mixture was subsequently warmed to 0/+5 °C and allowed to stir approximately 2.5 h. The reaction was quenched by pouring into a mixture of ethyl acetate,

10% citric acid and water. The organic phase was washed once with 10% citric acid, twice with 5 % sodium bicarbonate solution and once with water, then dried (magnesium sulfate), filtered and concentrated in vacuo to yield the crude product as a pink glass. Purification by recrystallization from ethyl acetate-hexane gave 0.57 g (two crops) of a solid product. It became clear from the NMR analysis that one diastereomer was recovered by recrystallization. HPLC analysis ($C_{18}$ reverse phase, acetonitrile-50 mmol ammonium acetate, pH 4.5) of the recrystallized product as well as the material recovered from the mother liquor after each had been deprotected by treatment with HCl in glacial acetic acid under standard conditions showed that the former was the long retention time component (isomer A) of the mixture. The material recovered from the mother liquor was rich in the short retention time component (isomer B). The analytical sample was obtained by flash chromatography on silica gel (ethyl acetate-hexane) of the material recovered from the mother liquor. $^1H$ NMR (DMSO-$d_6$, 300 MHz) isomer A $\delta$ 1.35 (d, 18H), 2.2-2.55 (cm, 2H), 2.85-3.25 (cm, 2H), 4.2 (cm, 1H), 4.5 (cm, 1H), 7.08 (d, J=9Hz, 1H), 7.25 (br s, 1H), 7.55 (cm, 2H), 7.67 (d, J=7.5Hz, 1H) 7.83 (d, J=7.5Hz, 1H),8.0-8.2 (cm, 2H). Anal calc for $C_{22}H_{32}N_4O_8$: C 54.98, H 6.72, N 11.66; found: C 54.76, H 6.86, N 11.30.

## Example 20e

### Boc-Leu-Asp-(m-nitro)Phe-$NH_2$ (Isomer A)

Isomer A from example 20d (0.46 g, 0.95 mmol) was treated with excess 1.5N HCl in glacial acetic acid at room temperature. The flask was capped with a drierite filled drying tube and allowed to stir 5 h. The reaction mixture was subsequently frozen and lyophilized. The crude hydrochloride was used without further purification. The hydrochloride (0.95 mmol) was combined with Boc-Leu-OSu (0.31 g,0.95 mmol) in a flask chilled in an ice bath to which was added pre-chilled DMF (15 ml) followed by DIEA (0.36 ml, 2.09 mmol). The flask was capped with a drierite filled drying tube and allowed to stir overnight while slowly warming to room temperature. The reaction mixture was subsequently concentrated in vacuo and poured into dilute aqueous citric acid solution. The crude product was collected by vacuum filtration and water washed. Purification by recrystallization from aqueous ethanol gave 0.32 g of a solid product (two crops). $^1H$ NMR (DMSO-$d_6$, 300 MHz) $\delta$ 0.83 (m, 6H), 1.2-1.65 (cm, 3H), 1.37 (s, 9H), 2.4-2.7 (cm, 2H), 2.85-3.25 (cm, 2H) 3.92 (cm, 1H), 4.45 (cm, 2H), 6.83 (d, J=9Hz, 1H), 7.25 (s, 1H), 7.38 (s, 1H), 7.55 (t, 1H), 7.67 (d, J=7.5Hz, 1H), 7.95-8.2 (cm, 3H). Anal calc for $C_{24}H_{35}N_5O_9$: C 53.61, H 6.57, N 13.03; found: C 53.44, H 6.61, N 12.97.

## Example 20f

### Boc-Trp-Leu-Asp-(m-nitro)Phe-$NH_2$ (Isomer A)

The product from example 20e (0.259 g, 0.48 mmol) was treated with 7 ml of 1.5N HCl in glacial acetic acid at room temperature. The flask was capped with a drierite filled drying tube and allowed to stir for one hour. The reaction mixture was subsequently frozen and lyophilized. The crude hydrochloride was used without further purification. The hydrochloride (0.48 mmol) was combined with Boc-Trp-OSu (0.2 g, 0.48 mmol) in a flask to which was added DMF and the resulting solution chilled in an ice bath. To the cold solution was added DIEA (0.184 ml, 1.06 mmol) and the flask capped with a drierite filled drying tube and allowed to stir overnight while slowly warming to room temperature. The reaction mixture was poured into dilute aqueous citric acid and the product collected by vacuum filtration and water washed. Purification by recrystallization from aqueous ethanol gave 0.30 g of a solid product. MS (FAB+) m/e 724 $(M+H)^+$. $^1H$ NMR (DMSO-$d_6$, 300 MHz) $\delta$ 0.84 (m, 6H), 1.1-1.7 (cm, 12H), 2.4-3.3 (cm, 6H), 4.2 (cm, 1H), 4.33 (cm, 1H), 4.46 (cm, 2H), 6.84 (d, J=9Hz, 1H), 6.96 (t, 1H), 7.05 (t, 1H), 7.1 (s, 1H), 7.26 (br s, 1H), 7.31 (d, J=7.5Hz, 1H),7.38 (br s, 1H), 7.5-7.7 (cm, 3H), 7.89 (d, J=9Hz, 1H), 7.96 (d, J=9Hz, 1H), 8.06 (d, J=7.5Hz, 1H), 8.11 (s, 1H), 8.23 (d, J=7.5Hz, 1H),10.78 (s, 1H), 12.45 (br s, 1H). Anal calc for $C_{35}H_{45}N_7O_{10}.0.5H_2O$: C 57.36,

H 6.34, N 13.38; found: C 57.48, H 6.30, N 13.33. mp 197-198.5 °C (d).

Example 21

Boc-Trp-Leu-Asp-(m-amino)Phe-$NH_2$(Isomer A)

The product from example 20f (0.064 g, 0.089 mmol) was dissolved in DMF (2 ml) and to this solution was added 5% Pd/$BaSO_4$ (15.3 mg). The reaction mixture was subsequently degassed and then pressurized to one atmosphere with hydrogen (balloon) at room temperature. After approximately one hour the solution was filtered to remove catalyst and the crude product purified by preparative $C_{18}$ reverse phase HPLC (acetonitrile-50 mmol ammonium acetate pH4.5) to give after lyophilization 0.048 g of a white solid. MS (FAB-) m/e 692 (M-H)-. $^1$H NMR (DMSO-$d_6$, 300 MHz) $\delta$ 0.85 (m, 6H), 1.1-1.7 (cm, 12H), 2.4-3.2 (cm, 6H), 4.15-4.45 (cm, 3H), 4.5 (cm, 1H), 6.37 (m, 3H), 6.75-7.2 (cm, 5H), 7.26 (br s, 1H), 7.31 (d, J=7.5Hz, 1H), 7.6 (d, J=7.5Hz, 1H), 7.85 (br d, J=9Hz, 1H), 7.95 (d, J=9Hz, 1H), 8.33 (d, J=7.5Hz, 1H), 10.81 (s, 1H). Anal calc for $C_{35}H_{47}N_7O_8.1.5H_2O$: C 58.31, H 7.00, N 13.60; found: C 58.46, H 6.66, N 13.24.

Example 22

Boc-Trp-Leu-Asp-[m-(N-ethyl)amino]Phe-$NH_2$

Example 22a

Boc-(m-(N-ethyl)amino)-Phe-$NH_2$

The product from example 21 (0.16 g, 0.53 mmol) was dissolved in methanol (15 ml) and to this solution was added 5% Pd/$BaSO_4$ (84 mg). The reaction mixture was subsequently degassed and then pressurized to one atmosphere with hydrogen (balloon) at room temperature. After one half hour the reaction mixture was filtered through celite to remove the catalyst and the filtrate concentrated to dryness to give 0.158 g of the crude product suitable for use without further purification. The above product (0.53 mmol) was dissolved in glacial acetic acid (10 ml) and the solution cooled to 10 °C. To this was added solid sodium borohydride (0.22 g, 5.81 mmol) in portions over several minutes. the flask was subsequently capped with a rubber septum and allowed to stir overnight at room temperature. The reaction mixture was frozen and lyophilized. the residue was partitioned between ethyl acetate and 5% sodium bicarbonate solution. The aqueous phase was extracted twice with ethyl acetate and the combined organics dried (magnesium sulfate), filtered and concentrated in vacuo. The crude product was purified by flash chromatography on silica gel (ethyl acetate-hexane-acetic acid then ethyl acetate-acetic acid-methanol) to give 0.117 g product. $^1$H NMR (DMSO-$d_6$, 300 MHz) $\delta$ 1.15 (t, 3H), 1.32 (s, 9H), 2.6 (cm, 1H), 2.82 (cm, 1H), 3.0 (q, 2H), 4.05 (cm, 1H), 5.38 (br s, 1H), 6.4 (m, 3H), 6.7 (d, J=9Hz, 1H), 6.9-7.0 (cm, 2H), 7.3 (s, 1H).

Example 22b

Boc-Asp($\beta$-benzyl)-[m-(N-ethyl)amino]-Phe-$NH_2$

The product from example 22a (0.117 g, 0.381 mmol) was treated with 6 ml of 1.5N HCl in glacial acetic acid at room temperature. The flask was capped with a drierite filled drying tube and allowed to stir for one hour. The reaction mixture was subsequently frozen and lyophilized. The crude hydrochloride isolated in this manner was sufficiently pure for direct use in subsequent steps. To a solution of Boc-Asp(β-benzyl) ester (0.12 g, 0.37 mmol) in THF (15 ml) under nitrogen was added NMM (0.13 ml, 1.17 mmol) and the resulting solution cooled to -10/-15 °C. To this was added in one portion isobutylchloroformate (IBCF) (0.05 ml, 0.37 mmol) and the activation allowed to proceed 5 min. A previously prepared solution of the above hydrochloride (0.37 mmol) in DMF (4 ml) was subsequently added via syringe over 4 min. On completion of the addition the reaction mixture was warmed to 0/+5 °C and allowed to stir while slowly warming to room temperature. After approximately one hour the reaction was quenched by pouring into water and the crude product isolated by extraction with ethyl acetate. The combined organics were dried (magnesium sulfate), filtered and concentrated in vacuo. Purification by flash chromatography on silica gel (ethyl acetate-hexane) gave 0.139 g product. $^1H$ NMR (DMSO-$d_6$, 300 MHz) δ 1.12 (cm, 3H) , 1 .37 (s, 9H), 2.4-3. 1 (cm, 6H), 4.32 (cm, 2H), 5.5 and 5.8 isomers A and B (s, 2H), 5.33 (t, 1H), 6.37 (m, 3H), 6.91 (cm, 1H), 7.1-7.4 (cm, 8H), 7.72 and 7.98 isomers A and B (d, J=9Hz, total 1H).

## Example 22c

### Boc-Leu-Asp(β-benzyl)-[m-(N-ethyl)amino]-Phe-$NH_2$

The product from example 22b (0.139 g, 0.264 mmol) was treated with 6 ml of 1.5N HCl in glacial acetic acid at room temperature. The flask was capped with a drierite filled drying tube and allowed to stir one hour. The reaction mixture was subsequently frozen and lyophilized. The crude hydrochloride was sufficiently pure for use as isolated. The hydrochloride (0.264 mmol) was combined with Boc-Leu-OSu (0.09 g, 0.264 mmol) and to this mixture was added DMF (5 ml). The resulting solution was cooled in an ice bath and to the cold solution was added DIEA (0.10 ml, 0.58 mmol). The flask was capped with a drierite filled drying tube and allowed to stir overnight while slowly warming to room temperature. The reaction mixture was subsequently poured into water and extracted with ethyl acetate. The combined extracts were dried (magnesium sulfate), filtered and concentrated in vacuo. The residue was purified by flash chromatography on silica gel (ethyl acetate-hexane) to give 0.089 g of pure product. $^1H$ NMR (DMSO-$d_6$, 300 MHz) δ 0.83 (m, 6H), 1.12 (q, 3H), 1.25-1.7 (cm, 12H), 2.5-3.05 (cm, 6H), 3.91 (cm, 1H), 4.28 (cm, 1H), 4.45-4.65 (cm, 1H), 5.03 and 5.07 isomers A and B (s, 2H), 5.35 (cm, 1H), 6.36 (cm, 3H), 6.85-7.45 (cm, 8H), 7.8 and 7.87 isomers A and B (d, J=9Hz, total 1H), 8.17 (d, J=7.5 Hz) and 8.31 (d, J=9Hz) isomers A and B total 1H.

## Example 22d

### Boc-Trp-Leu-Asp-[m-(N-ethyl)amino]Phe-$NH_2$

The product from example 22c (0.089 g, 0.142 mmol) was dissolved in methanol (7 ml). To this solution was added 10% Pd/C (10 mg). The reaction mixture was subsequently degassed and then pressurized to one atmosphere with hydrogen (balloon) at room temperature. After 45 min the solution was filtered through celite and the filtrate concentrated to dryness. The residue was treated with 6 ml of 1.5N HCl in glacial acetic acid at room temperature. The flask was capped with a drierite filled drying tube and the contents allowed to stir 45 min. The contents of the flask were subsequently frozen and lyophilized. The crude hydrochloride was combined with Boc-Trp-OSu (0.06 g, 0.142 mmol) under a nitrogen atmosphere and to this was added DMF (2 ml). The solution was cooled in an ice bath and to the cold solution was added DIEA (0.08 ml, 0.47 mmol). The mixture was allowed to stir overnight while slowly warming to room temperature. The reaction mixture was concentrated in vacuo and the product suspended in aqueous acetic acid. The solid was collected by centrifuge, dissolved in glacial acetic acid, frozen and lyophilized. The crude product was purified by preparative $C_{18}$ reverse phase HPLC (acetonitrile-50 mmol ammonium

acetate pH 4.5) to yield after lyophilization 0.028 g of a solid product. MS (FAB-) m/e 720 (M-H)-, (FAB+) m/e 722 (M+H)+. $^1$H NMR (DMSO-$d_6$, 300 MHz) δ 0.85 (m, 6H), 1.15 (t, 3H), 1.2-1.75 (cm, 12H), 2.4-3.15 (cm, 8H), 4.15-4.55 (cm, 4H), 6.3-6.45 (cm, 3H), 6.81 (d, J=7.5Hz, 1H), 6.85-7.2 (cm, 5H), 7.25-7.4 (cm, 2H), 7.6(t, 1H), 7.87 (br d,), 7.95 and 8.06 isomers A and B (d, J=7.5Hz total 2H), 8.3 and 8.37 isomers A and B (d, J=7.5Hz, total of 1H), 10.83 (br s, 1H). Anal calc for $C_{37}H_{51}N_7O_8.2H_2O$: C 58.62, H 7.33, N 12.94; found: C 58.26, H 6.81, N 12.66.

## Example 23

### Ctp-Leu-Asp-(dehydro)Phe-$NH_2$

The product from example 19c (0.099 g, 0.201 mmol) was treated with 6 ml 1.5 N HCl in glacial acetic acid at room temperature and the flask capped with a drierite filled drying tube. After 45 min the contents of the flask were frozen and lyophilized. The crude hydrochloride was sufficiently pure for use as isolated. To a solution of Ctp (0.05 g, 0.191 mmol) prepared according to Yonemitsu et.al., J.Am.Chem.Soc. , 88 (17), 3941 (1966), in DMF (1 ml) and THF (2 ml) under a nitrogen atomosphere was added NMM (0.05 ml, 0.42 mmol) and the resulting solution cooled to -3 °C with an ice-salt bath. To this was added in one portion IBCF (0.026 ml, 0.191 mmol) and activation allowed to proceed 7 min. A previously prepared solution of the above hydrochloride (0.201 mmol) in DMF (2 ml) was subsequently added dropwise over 3 min and the mixture allowed to warm to room temperature. After 2.5 h the reaction mixture was concentrated in vacuo. The residue was dissolved in methanol and then suspended in 10% citric acid solution. the crude product was collected by centrifuge and purified by preparative $C_{18}$ reverse phase HPLC (acetonitrile-50 mmol ammonium acetate, pH 4.5) to give after lyophilization 0.018 g product. MS (FAB+) m/e 617 (M+H)+. $^1$H NMR (DMSO-$d_6$, 300 MHz) δ 0.89 (m, 6H), 1.5 (t, 2H), 1.65 (cm, 2H), 2.6-2.9 (cm, 2H), 4.2 (cm, 2H), 4.35 (cm, 1H), 4.55 (cm, 1H), 6.75 (d, J=6Hz, 1H), 6.9-7.05 (cm, 2H), 7.13 (s, 1H), 7 2-7.45 (cm, 6H), 7.58 (d, J=7.5Hz, 2H), 8.35 (d, J=7.5Hz, 1H), 8.66 (d, J=7.5Hz, 1H), 9.45 (s, 1H), 10.93 (s, 1H). Anal calc for $C_{32}H_{36}N_6O_7 \cdot 2.5H_2O$: C 58.07, H 6.26, N 12.70; found: C 57.75, H 5.55, N 12.44.

## Example 24

### Boc-Trp-Met-Asu-Phe-$NH_2$

To a mixture of Boc-Trp-Met-Asp-Phe-$NH_2$ (0.153 g, 0.220 mmol) prepared according to Morley, et.al., J Chem Soc, 555 (1966) and p-chlorobenzoic acid anhydride (0.135 g, 0.457 mmol) which was prepared as described in J. Amer. Chem Soc. , 40 , 424 (1918) was added, at room temperature, DMF (10 ml), followed by DIEA (0.045 ml, 0.253 mmol). The flask was capped with a nitrogen filled balloon and the contents allowed to stir approximately 3 h. The mixture was subsequently poured into brine solution and the product extracted with ethyl acetate. The combined extracts were dried (magnesium sulfate), filtered and concentrated in vacuo. The crude product was purified by flash chromatography on silica gel (ethyl acetate-hexane-acetic acid) to give 0.083 g of solid product. $^1$H NMR (DMSO-$d_6$, 300 MHz) δ 1.15 and 1.3 (s, total 9H), 2.04 (s, 3H), 1.88 (cm, 2H), 2.35-2.57 (cm, 2H), 2.85-3.15 (cm, 5H), 3.38-3.5 (cm, 1H), 4.22 (cm, 1H), 4.4 (cm, 2H), 4.63 (q, 1H), 6.84 (d, J=7.5Hz, 1H), 6.97 (t, 1H), 7.05 (t, 1H), 7.1-7.4 (cm, 9H), 7.6 (d, J=7.5Hz, 1H), 8.12 (d, J=7.5Hz, 1H), 8.52 (d, J=7.5Hz, 1H), 10.8 (s, 1H). Anal calc for $C_{34}H_{42}N_6O_7S$ $0.5H_2O$: C 59.36, H 6.31, N 12.22; found: C 59.41, H 6.39, N 11.88.

## Example 25

Boc-Trp-Tpp-Asp-(N-Me)Phe-$NH_2$

Example 25a

Boc-Tpp-Asp(β-benzyl)-(N-Me)Phe-$NH_2$

To a solution of Boc-(trans-3-propyl)Proline (1.03 g, 4.00 mmol) prepared as in example 52g, in THF (75 ml) under nitrogen at room temperature was added NMM (0.5 ml, 4.4 mmol) and the resulting solution cooled to -10/-15 °C. To this was added IBCF (0.52 ml, 4.0 mmol) and the activation allowed to proceed 6 min. A freshly prepared solution of Asp(β-benzyl)-(N-Me)Phe-$NH_2$ hydrochloride (1.68 g, 4.0 mmol), prepared as in example 5e, in DMF (40 ml) was subsequently added (rapidly) dropwise over 5 min followed by the (slow) dropwise addition of a solution of NMM (0.5 ml, 4.4 mmol) in THF (75 ml) over one hour while slowly warming the reaction mixture to 0 °C. After stirring an additional 30 min, the mixture was partitioned between ethyl acetate and dilute aqueous HCl. The organic phase was washed once each with water, 5% sodium bicarbonate and water then dried (magnesium sulfate), filtered and concentrated in vacuo. The crude product was purified by flash chromatography on silica gel (ethyl acetate-hexane) to give 2.01 g of the product as a foam. MS m/e 623 (M+H)+, m/e 640 (M+NH4)+. $^1$H NMR (DMSO-$d_6$, 300 MHz) δ 0.82 (cm, 3H), 1.1-1.6 (cm, 14H), 1.85 (cm, 2H), 2.2-3.7 (cm, 12H), 4.75 (cm, 1H), 4.85-5.2 (cm, 4H), 7.0-7.6 (cm, 12H), 8.3-8.6 (cm, 1H). Anal calc for $C_{34}H_{46}N_4O_7 \cdot 0.5H_2O$: C 64.63, H 7.51, N 8.87; found: C 64.46, H 7.51, N 8.68.

Example 25b

Tpp-Asp(β-benzyl)-(N-Me)Phe-$NH_2$ Hydrochloride

The product from example 25a (2.01 g, 3.23 mmol) was treated at room temperature with 10 ml of 1.44N HCl in glacial acetic acid. The flask was capped with a drierite filled drying tube and allowed to stir 40 min. The product was precipitated with ethyl ether (400 ml), collected by vacuum filtration and dried in vacuo at room temperature to give 1.56 g of a white solid product. Anal Calc for $C_{29}H_{38}N_4O_5 \cdot HCl \cdot H_2O$: C 60.34, H 7.00, N 9.71; found: C 60.67, H 6.90, N 9.76.

Example 25c

Boc-Trp-Tpp-Asp(β-benzyl)-(N-Me)Phe-$NH_2$

The product from example 25b (1.56 g, 2.79 mmol) was combined with Boc-Trp-OH (0.85 g, 2.79 mole), HOBt (0.38 g, 2.79 mmol) and EDCI (0.54 g, 2.79 mmol) under nitrogen and to this mixture was added DMF (20 ml). The resulting solution was chilled in an ice bath and to the cold solution was added DIEA (0.53 ml, 3.07 mmol). The reaction mixture was allowed to warm to room temperature. After approximately 3.5 h the mixture was partitioned between ethyl acetate and dilute hydrochloric acid. The organic phase was dried (magnesium sulfate), filtered and concentrated in vacuo. The crude product was purified by flash chromatography on silica gel (ethyl acetate-hexane) to give 1.59 g product. MS (FAB+) m/e 809 (M+H)+, m/e 831 (M+Na)+. $^1$H NMR (DMSO-$d_6$, 300 MHz) δ 0.83 (cm, 3H), 1.1-1.6 (cm, 13H), 2.0 (cm, 2H), 2.2-3.6 (cm, 10H), 3.7-3.95 (cm, 2H), 4.2-4.5 (cm, 1H), 4.7-5.7 (cm, 4H), 6.85-7.6 (cm, 18H), 8.32-8.53 (cm, 1H), 10.75-10.92 (cm, 1H). Anal calc for $C_{45}H_{56}N_6O_8 \cdot 1.5H_2O$: C 64.63, H 7.13, N 10.05;

found: C 64.67, H 6.75, N 10.02.

Example 25d

Boc-Trp-Tpp-Asp-(N-Me)Phe-$NH_2$

The product from example 25c (0.072 g, 0.089 mmol) was dissolved in methanol (5 ml) to which was added 10% Pd/C (0.035 g). The mixture was degassed then subjected to hydrogenolysis in a Parr shaker under 4 atmosphere hydrogen at room temperature. The reaction mixture was filtered through celite and the filtrate concentrated in vacuo. The crude product was purified by preparative $C_{18}$ reverse phase HPLC (acetonitrile-50 mmol ammonium acetate pH 4.5) to give after lyophilization 0.037 g product. MS (FAB+) m/e719 (M+H)+. $^1H$ NMR (DMSO-$d_6$, 300 MHz) δ 0.83 (cm, 2H), 1.1-1.6 (cm, 13H), 1.8-2.1 (cm, 2H), 2.3-3.5 (cm, 10H), 3.7-4.0 (cm, 2H), 4.2-4.5 (cm, 1H), 4.75-5.2 (cm, 3H), 6.9-7.6 (cm, 13H), 8.2-8.5 (cm, 1H). Anal calc for $C_{38}H_{50}N_6O_8 \cdot 2H_2O$: C 60.45, H 7.22, N 11.13; found: C 0.19, H 6.67, N 11 14.

Example 26

Boc-Trp-Nle-Asp-(N-Me)Phe-$NH_2$

Example 26a

Boc-Nle-Asp(β-benzyl)-(N-Me)Phe-$NH_2$

Boc-Nle-OH (1.82 g, 7.87 mmol) was coupled with Asp(β-benzyl)-N-methyl-Phe-$NH_2$ hydrochloride (3.3 g, 7.87 mmol) using methodology as described in example 25a. The crude product was isolated and purified as described to give 3.84 g of the desired product. MS (FAB-) m/e 595 (M-H)-. $^1H$ NMR (DMSO-$d_6$, 300 MHz) δ 0.75-1.6 (cm, 18H), 2.2 (cm, 1H), 2.5 (cm, 1H), 2.7-3.3 (cm, 5H), 3.83 (cm, 1H), 4.65 and 4.85-5.15 (cm, 4H), 6.8 (cm, 1H), 7.0-7.55 (cm, 12H), 8.15 (d, J=9Hz) and 8.44 (d, J=7.5Hz) total 1H. Anal calc for $C_{32}H_{44}N_4O_7$. 0.25 $H_2O$: C 63.91, H 7.47, N 9.32; found: C 63.89, H 7 37, N 9.27.

Example 26b

Nle-Asp(β-benzyl)-(N-Me)Phe-$NH_2$ Hydrochloride

The product from example 26a (3.84 g, 6.43 mmol) was treated with 20 ml of 1.5N HCl in glacial acetic acid at room temperature. The flask was capped with a drierite filled drying tube and the contents allowed to stir one hour. The product was precipitated by the dropwise addition of ethyl ether (300 ml), collected by vacuum filtration and dried in vacuo at room temperature to give 3.16 g of a white solid. Anal calc for $C_{27}H_{36}N_4O_5$.HCl.0.5 $H_2O$. C 59.81, H 7.08, N 10.34; found: C 59.50, H 6.79, N 10.27.

Example 26c

Boc-Trp-Nle-Asp(β-benzyl)-(N-Me)Phe-$NH_2$

The tripeptide hydrochloride from example 26b (1.54 g, 2.89 mmol) was combined under a nitrogen atmosphere with Boc-Trp-OSu (1.16 g, 2.89 mmol) and to this mixture DMF (20 ml) was added. The resulting solution was cooled in an ice bath and DIEA (0.55 ml, 3.18 mmol) was added. The ice bath was removed and the mixture allowed to warm to room temperature. After approximately 2.5 h the reaction mixture was partitioned between ethyl acetate and dilute aqueous hydrochloric acid. The organic phase was washed once with water, dried (magnesium sulfate), filtered and concentrated in vacuo. The crude product was purified by flash chromatography on silica gel (ethyl acetate-hexane) to give 1.91 g of solid product.MS (FAB+) m/e 805 (M+Na)+. Anal calc for $C_{43}H_{54}N_6O_8 \cdot 0.5\ H_2O$: C 65.20, H 7.01, N 10.61; found: C 65.07, H 6.82, N 10.54.

Example 26d

Boc-Trp-Nle-Asp-(N-Me)Phe-$NH_2$

The product from example 26c (0.181 g, 0.23 mmol) was subjected to hydrogenolysis as described in example 25d. Purification by preparative $C_{18}$ reverse phase HPLC (acetonitrile-50 mmol ammonium acetate, pH 4.5) gave after lyophilization 0.086 g of solid product. MS (FAB+) m/e 693 (M+H)+. $^1$H NMR (DMSO-$d_6$, 300 MHz) δ 0.84 (cm, 1H), 1.0-1.6 (cm, 15H), 2.08 and 2.36 (cm, 1H), 2.65-3.5 (cm, 8H), 4.1-4.4 (cm, 2H), 4.65 and 5.0 (cm, 1H), 4.88 and 5.15 (q, 1H), 6.82 (cm, 1H), 6.9-7 7 (cm, 12H), 7.8 and 7.88 (d, J=9Hz, total 1H), 8.26 (d, J=9Hz) and 8.61 (d, J=7.5Hz) total 1H. Anal calc for $C_{36}H_{48}N_6O_8 \cdot 0.5\ H_2O$: C 61.59, H 7.05, N 11.98; found: C 61.50, H 7.07, N 12.06.

Example 27

Boc-Trp-Nle-Asp-(dehydro)Phe-$NH_2$

Example 27a

Boc-Nle-Asp-(dehydro)Phe-$NH_2$

The fully protected dehydrodipeptide (0.78 g, 1.81 mmol) prepared as outlined in example 19c was treated at room temperature under nitrogen with excess 1.4N HCl in glacial acetic acid. After 2.5 h methanol was added until the solution became homogeneous and the product subsequently precipitated by the dropwise addition of ethyl ether (125 ml). Vacuum filtration, followed by drying in vacuo at room temperature, gave the hydrochloride 0.55 g as a white solid suitable for use without further purification. To a solution of Boc-Nle-OH (0.19 g, 0.80 mmol) in THF (10 ml) under nitrogen was added NMM (0.20 ml, 1.75 mmol) and the resulting solution cooled to -15/-20 °C. To this was added in one portion IBCF (0.10 ml, 0.80 mmol). The activation was allowed to proceed 8 min upon which time a previously prepared solution of the above hydrochloride (0.25 g,0.80 mmol) in DMF (7 ml) was aded dropwise over 3 min. The reaction mixture was subsequently warmed to 0/+5 °C. After one hour, the contents of the flask were partitioned between

ethyl acetate and dilute aqueous hydrochloric acid. The organic phase was washed once with water, dried (magnesium sulfate), filtered and concentrated in vacuo. The crude product was purified by recrystallization from ethyl acetate-acetone to give 0.20 g solid product. MS (FAB+) m/e 491 (M+H)+, m/e 513 (M+Na)+. $^1$H NMR (DMSO-$d_6$, 300 MHz) δ 0.82 (cm, 3H), 1.15-1.65 (cm, 15H), 2.6-2.9 (cm, 2H), 3.88 (cm, 1H), 4.57 (cm, 1H), 7.05 (d, J=7.5Hz, 1H), 7.14 (br s, 1H), 7.25-7.40 (cm, 4H), 7.55 (dd, 1H), 8.46 (d, J=7.5Hz,1H), 9.32 (s, 1H), 12.53 (br s, 1H). Anal calc for $C_{24}H_{34}N_4O_7$: C 58.75, H 7.00, N 11.42; found: C 58.46, H 6.92, N 11.08. mp 180-182 °C (d).

## Example 27b

### Boc-Trp-Nle-Asp-(dehydro)Phe-$NH_2$

The product from example 27a (0.184 g, 0.375 mmol) was treated with excess 1.57N HCl in glacial acetic acid. The flask was capped with a drierite filled drying tube and the contents allowed to stir at room temperature. After approximately 1.5 h the contents of the flask were frozen and lyophilized. The crude hydrochloride was sufficiently pure for use as isolated. The hydrochloride (0.375 mmol) and Boc-Trp-OSu (0.15 g, 0.375 mmol) were combined and to this mixture was added, under nitrogen, DMF (5 ml). The resulting solution was cooled in an ice bath and DIEA (0.15 ml, 0.825 mmol) added. The mixture was allowed to stir overnight while slowly warming to room temperature. The reaction mixture was subsequently poured into dilute aqueous hydrochloric acid. The crude product was collected ny vacuum filtration and washed with water. Purification by recrystallization from aqueous ethanol gave 0.184 g solid product. MS (FAB-) m/e 675 (M-H)-. $^1$H NMR (DMSO-$d_6$, 300 MHz) δ 0.82 (cm, 3H), 1.05-1.75 (cm, 15H), 2.55-3.15 (cm, 4H), 4.21 (cm, 1H), 4.31 (cm, 1H), 4.62 (cm, 1H), 6.88 (d, J=7.5Hz, 1H), 6.97 (t, 1H), 7.05 (t, 1H), 7.12 (s, 1H), 7.17 (br s, 1H), 7.25-7.40 (cm, 5H), 7.58 (cm, 3H), 7.97 (d, J=7.5Hz,1H), 8.5 (d, J=7.5Hz,1H), 9.52 (s, 1H), 10.78 (s, 1H), 12.58 (br s, 1H). Anal calc for $C_{35}H_{44}N_6O_8$ 0.5 $H_2O$: C 61.29, H 6.63, N 2.26; found: C 61.40, H 6.60, N 12.37. mp 193.5-194.5 °C (d)

## Example 28

### Boc-Trp-Leu-Asu-Phe-$NH_2$

To a mixture of Boc-Trp-Leu-Asp-Phe-$NH_2$ (0.5 g, 0.74 mmol) prepared according to G.W Kenner, et.al., J. Chem. Soc. C, 761 (1968) and benzoic anhydride (0.19 g, 0.81 mmol) under a nitrogen atmosphere was added DMF (10 ml) followed by DIEA (0.14 ml, 0.81 mmol). The reaction mixture was allowed to stir overnight at room temperature. The contents of the flask was subsequently partitioned between ethyl acetate and dilute hydrochloric acid. The organic phase was washed once (each) with water, 5% sodium bicarbonate and water, then dried (magnesium sulfate), filtered and concentrated in vacuo. The crude product was purified by flash chromatography on silica gel (ethyl acetate-hexane) to yield 0.45 g of a solid product. MS (FAB+) m/e 561 (M+H)+. $^1$H NMR (DMSO-$d_6$, 300 MHz) δ 0.87 (m, 6H), 1.3 (s, 9H), 1.4-1.7 (cm, 3H), 2.38 (m, 1H), 2.85-3.15 (cm, 4H), 3.43 (dd, 1H), 4.22 (cm, 1H), 4.37 (cm, 1H), 4.63 (dd, 1H), 6.78 (d, J=7.5Hz,1H), 6.95-7.4 (cm, 11H), 7.6 (d, J=7.5Hz,1H), 8.02 (d, J=9Hz,1H), 8.57 (d, J=7.5Hz,1H), 10.8 (s, 1H). Anal calc for $C_{35}H_{44}N_6O_7$.0.25 $C_4H_8O_2$ (ethyl acetate): C 63.31, H 6.80, N 12.31; found C 63.05, H 6.84, N 12.27.

## Example 29

### $N^{\alpha}$-Boc-$N^{in}$-formyl-Trp-Leu-Asp-Phe-$NH_2$

## Example 29a

### $N^{\alpha}$-Boc-$N^{in}$-formyl-Trp-succinimide ester

To a solution of $N^{\alpha}$-Boc-$N^{in}$-formyl-Trp (200 mg,0.60 mmol) in 6 ml of anhydrous DME was added N-hydroxysuccinimide (69 mg, 0.60 mmol) and DCC (200 mg, 1.0 mmol) at room temperature and stirred for 48 h under nitrogen atmosphere. Dicyclohexyl urea was removed by filtration, the solvent evaporated and the residue dissolved in EtOAc. The filtration process was repeated. EtOAc was then evaporated to give the product (205 mg, 80%) as a crisp white foam. $^1H$ NMR ($CDCl_3$, 60 MHz) $\delta$ 1.4 (s, 9H), 2.8 (s, 4H), 3.4 (dd, 2H), 5.1 (br s, 1H), 7.2-7.6 (m, 5H), 9.1 (br s, 1H).

## Example 29b

### $N^{\alpha}$-Boc-$N^{in}$-formyl-Trp-Leu-Asp-Phe-$NH_2$

To a solution of Leu-Asp-Phe-$NH_2$•HCl (349 mg, 0.80 mmol) in 6 ml anhydrous DMF was added DIEA (0.28 ml, 1.60 mmol) and a solution of 29a (350 mg, 0.82 mmol) in 6 ml anhydrous DMF at 0 °C. The resulting solution was stirred for 24 h with warming to ambient temperature under nitrogen atmosphere. The reaction mixture was poured into a rapidly stirring solution of cold 10% citric acid causing a white precipitate. The solid was collected and recrystallized from EtOH to give the product (280 mg,50%) as a white solid: mp 215-218 °C., $[\alpha]^{25}D$ = -30.6° (c=0.3, DMF)., MS(FAB+) m/e 707(M + H)$^+$. 1H NMR-(DMSO-$d_6$, 300 MHz) $\delta$ 0.81-0.87 (2d, 6H), 1.29 (s, 9H), 1.13 (m, 1H), 1.33-1.70 (m, 2H), 2.44-2.50 (dd, 1H), 2.65-2.73 (dd, 1H), 2.80-3.08 (m, 4H), 4.26-4.36 (m, 3H), 4.52 (q, 1H), 7.18-7.38 (m, 10H) and six $D_2O$ exchangable protons. Anal Calcd for $C_{36}H_{46}N_6O_9$•0.5 $H_2O$: C 60.42; H 6.34; N 11.75. Found: C 60.62; H 6.54; N 11.60.

## Example 30

### $N^{\alpha}$-Boc-$N^{in}$-Boc-(2,3-dihydro-Trp)-Leu-Asp-Phe-$NH_2$

## Example 30a

### $N^{\alpha}$-Boc-$N^{in}$Boo-2,3-dihydro-Trp succinimide ester

To a solution of $N^{a}$-Boc-$N^{in}$-Boc-2,3-dihydro-Trp (407 mg,1.0 mmol) in 10 ml anhydrous DME was added N-hydroxysuccinimide (115 mg,1.0 mmol) and DCC (309 mg,1.5 mmol). The reaction mixture was stirred at room temperature for 48 h under nitrogen atmosphere. Dicyclohexyl urea was removed by filtration, the solvent evaporated and the residue dissolved in EtOAc. The filtration process was repeated. The EtOAc was then evaporated to give the product (400 mg,80%) as a crisp, slightly yellow foam. $^1$HNMR ($CDCl_3$,60 MHz) $\delta$ 1.5 (d, 18H), 1.9-2.4 (s, 2H), 2.8 (s, 4H), 3.3-5.3 (m, 7H), 6.8-7.8 (m, 4H).

Example 30b

$N^{\alpha}$-Boc-$N^{in}$-Boc-(2,3-dihydro-Trp)-Leu-Asp-Phe-$NH_2$

To a solution of Leu-Asp-Phe-$NH_2$•HCl (420 mg,1.0 mmol) in 8 ml anhydrous DMF was added DIEA (0.35 ml,2.0 mmol) and a solution of 30a (500 mg, 1.0 mmol) in 6 ml anhydrous DMF at 0 °C. The resulting solution was stirred for 24 h at room temperature under nitrogen atmosphere. The reaction mixture was poured into a rapidly stirred solution of cold 10% citric acid, causing a white precipitate which was collected and recrystallized from EtOH/$H_2O$ to give the product (429 mg,55%) as a white solid: mp 193-195 °C., $[a]^{25}_D$ = -30.4° (c=0.5, DMF, mixture of a pair of diastereomers), MS(FAB+) m/e 781 (M+H)., $^1H$ NMR-(DMSO-$d_6$/$D_2O$, 300 MHz) δ 0.85 (2d,6H), 1.4 (s, 9H), 1.5 (s, 9H), 1.55-1.85 (m, 2H), 2.0-2.13 (m, 1H), 2.45-3.12 (m, 7H), 3.25-3.40 (m, 1H), 3.63 (dd, 1H), 4.0-4.15 (m, 2H), 4.18-4.28 (m, 1H), 4.36 (dd, 1H), 4.47 (t, 1H), 6.90 (t, 1H), 7.1-7.30 (m, 9H). Anal Calcd for $C_{40}H_{56}N_6O_{10}$•$1H_2O$: C 60.11; H 7.32; N 10.52. Found: C 60.18; H 7.33; N 10.13.

Example 31

(2,3-Dihydroindole-Trp)-Leu-Asp-Phe-$NH_2$-HCl

The protected tetrapeptide in example 30b (200 mg,0.26 mmol) was treated with 6 ml of 4N HCl/dioxane and stirred under nitrogen at room temperature for 3 h. Anhydrous ether was added to the reaction with vigourous stirring causing a white precipitate The precipitate was collected and recrystallized from EtOH/EtOAc afford the product (150 mg, 90%) as a slightly cream colored solid. $[\alpha]^{25}D$=-0.27° (c=0.75, DMF, mixture of a pair of diastereomers at benzylic carbon of indoline moiety)., mp 175 °C (d), MS(FAB+) m/e 581$(M+H)^+$ for free base., $^1H$ NMR (DMSO-$d_6$/$D_2O$, 300 MHz) δ 0.90 (2d, 6H), 1.34-1.55 (m, 2H), 1.61-1.83 (m, 1H), 1.94-2.11 (m, 1H), 2.20-2.33 (m, 1H), 2.45-2.85 (two dd and one m, 5H), 3.03 (dd, 1H), 3.39 (dd, 1H), 3.56-3.67 (m, 1H), 3.90-4.0 (m, 1H), 4.3-4.45 (m, 1H), 4.45-4.60 (m, 1H), 7.15-7.45 (m, 9H).

Example 32

Boc-5-Amino-2-(3′-indolylmethyl)-pentanoyl-Leu-Asp-Phe-$NH_2$

Example 32a

Benzyl α-(3-indolylmethyl)-α-(triphenylphosphoranylidene)acetate

The compound was prepared in an identical manner to that described by M. Strandtmann, etal, J. Org. Chem. , 33 , 4306 (1968). mp.152-154 °C. MS(CI) m/e 540 $(M+H)^+$. $^1H$ NMR($CDCl_3$, 300 MHz) δ 3.56 (d, J=18Hz, 2H), 4.84 (br s, 1H), 5.15 (br s, 1H), 6.60 (s, 1H), 6.67 (br s, 1H), 6.93 (t, J=7.5Hz, 1H), 7.03 (br s, 1H), 7.08 (t, J=7.5 Hz, 1H), 7.20-7.38 (m, 10H), 7.38-7.57 (m, 10H), 7.73 (s, 1H). Anal calcd for $C_{36}H_{30}NO_2P$•0•$5H_2O$: C 78.80, H 5.71 N, 2.55; found: C 79.05, H 5.80, N 2.36.

Example 32b

Boc-5-amino-2-(3′-indolylmethyl)-2-pentenoic acid benzyl ester

To a solution of ylide from example 32a (10 g,18 mmol) in 100 ml of anhydrous $CH_2Cl_2$ was added to a solution of Boc-3-aminopropanal (E.J. Corey, et al, Tetrahedron Lett . 1979, 399) (2.1 g, 12 mmol) in 30 ml anhydrous $CH_2Cl_2$. After stirring at room temperature for three days, the product was purified by flash silica gel using EtOAC/Hexane (1/3~3/1,v/v) as eluents to afford the product (4.2 g,80%) as a yellow oil. $^1$H NMR ($CDCl_3$, 300 MHz) δ 1.4 (s, 9H), 2.6 & 2.6 (2q, 2H), 3.17 & 3.24 (2q, 2H), 3.777 & 3.83 (2s, 2H), 4.56 & 4.8 (br.,1H), 5.16 (s, 2H), 5.95 (t, 1H), 6.84 (br s, 1H), 6.9 (t, 1H), 7.1 (t, 1H), 7.18 (t, 1H), 7.24-7.38 (m, 6H), 7.6 (d, 1H), 7.96 (br.1H)., m/e calculated for $C_{26}H_{30}N_2O_4$ 434.2205, Found 434.2208.

Example 32c

Boc-5-amino-2-(3′-indolylmethyl)-2-pentanoic acid

The product from example 32b (766 mg, 1.7 mmol) was dissolved in 30 ml EtOAc and to this solution was added 600 mg of 10% Pd/C. The reaction mixture was stirred under hydrogen atmosphere for 6 h. Filtration of the reaction mixture through a celite pad, followed by several washings with MeOH, afforded a filtrate which after concentration was purified by flash silica gel using 1% HOAc/EtOAc (1/2~1/1,v/v) as eluent to provide the product (480 mg,81%) as an oil: $^1$H NMR ($CDCl_3$, 300 MHz) δ 1.4 (s, 9H), 1.5-1.75 (m, 4H), 2.5 & 2.8 (m, 1H), 3.05 (dd, 2H), 3.10-3.28 (m, 2H), 3.8 & 4.6 (2 br s, 1H), 6.9 (br s, 1H), 7.1 (t, 1H), 7.16 (t, 1H), 7.33 (d, 1H), 7.58 (2d,1H), 8.2 (br s, 1H)., m/e calculated for $C_{19}H_{26}N_2O_4$ 346.1892, Found 346.1898.

Example 32d

Boc-5-amino-2-indolemethyl-pentanoic acid 2,4,5-trichlorophenol ester

To a solution of example 32c (233 mg, 0.67 mmol) in 10 ml anhydrous $CH_2Cl_2$ was added 2,4,5-trichlorophenol (133 mg, 0.67 mmol) and EDCI (191 mg, 1.0 mmol). The solution was stirred at room temperature for 24 h under nitrogen atmosphere. The reaction mixture was washed with $H_2O$ (2x) and brine. The organic layer was concentration and the residue was purified by flash silica ge using 1% HOAc/EtOAc/Hexane (1/5~1/3,v/v) as eluents to afford the product (183 mg,52%) as a white, crisp foam: $^1$H NMR($CDCl_3$, 300 MHz) δ 1.44 (s, 9H), 1.6-1.9 (m, 4H), 3.08-3.3 (m, 5H), 4.55 (br.t, 1H), 6.67 (s, 1H), 7.06 (d, 1H), 7.14 (s, 1H), 7.1-7.3 (2t,2H), 7.4 (d, 1H), 7.62 (d, 1H), 8.12 (br,1H).

Example 32e

Boc-5-Amino-2-(3′-indolylmethyl)-2-pentanoyl-Leu-Asp-Phe-$NH_2$

A solution of Leu-Asp-Phe-$NH_2$•HCl (129 mg,0.30 mmol) in 5 ml anhydrous DMF was treated with DIEA (0.12 ml ,0.68 mmol), HOBt (40 mg,0.30 mmol) and a solution of example 32c (158 mg,0.3 mmol) in 5 ml

anhydrous DMF at 0 °C. The resulting solution was stirred with warming to ambient temperature for 24 h under a nitrogen atmosphere. The mixture then was poured into a cold, rapidly stirring solution of 10% citric acid causing a white precipitate which was collected and purified by flash silica gel using EtOAc/Py/HOAc/$H_2O$ (24/4/1.2/2.2) as an eluent and after lyophOlization afforded the product (120 mg,55%) as a white solid: mp 179-181 °C. HPLC analysis ($C_{18}$-ultrasphere ODS with $CH_3CN/H_2O/TFA$ = 40/60/0.1 as eluent) revealed a diastereomeric ratio of 45/55 at the carbon that bears indolemethylene moiety. $[\alpha]^{25}_D$ = -28.6° (c=0.6,DMF), MS(FAB+) m/e 721 $(M+H)^+$, $^1H$ NMR(DMSO-$d_6$,300 MHz) $\delta$ 0.61,0.69,0.81,0.85 (4d,6H), 1.34,1.36 (2s, 9H), 1.11-1.60 (m, 5H), 2.40-2.70 (m, 4H), 2.70-3,10 (m, 5H), 4.13-4.24 (m, 1H), 4.25-4.40 (m, 2H), 4.40-4.50 (m, 1H), 6.90-7.60 (m, 10 Ar-H), and eight $D_2O$ exchangable protons. Anal calcd for $C_{38}H_{52}N_6O_8 \cdot 0.5\ H_2O$: C 62.55, H 7.32, N, 11.52., Found: C 62.80, H 7.43, N 11.63.

Example 33

Boc-5-amino-pentenoyl-Leu-Asp-Phe-$NH_2$

A solution of Leu-Asp-Phe-$NH_2 \cdot HCl$ (300 mg, 0.48 mmol) in 10 ml anhydrous DMF was treated with DIEA (0.17 ml, 0.96 mmol) and Boc-5-amino-pentanoic trichlorophenol ester (190 mg, 0.48 mmol) in 2 ml anhydrous DMF at 0 °C. The resulting solution was stirred with warming to ambient temperature under nitrogen atmosphere for 24 h. The mixture was poured into a rapidly stirring solution of cold 10% citric acid causing a white precipitate which was collected and recrystallized from EtOAc to yield the product (185 mg,50%) as a white solid: mp 189-191 °C, MS(DCI) m/e 795 $(M+NH_4)^+$., $[\alpha]^{25}_D$ = -29.8° (c=0.5,DMF)., $^1H$ NMR (DMSO-$d_6$, 300 MHz) $\delta$ 0.81,0.85 (2d, 6H), 1.36 (s, 9H), 1.20-1.65 (m, 7H), 1.95-2.10 (m, 2H), 2.35-2.63 (m, 2H), 2.75-2.95 (m, 4H), 3.0-3.21 (m, 2H), 4.20-4.36 (m, 2H), 4.42-4.50 (q, 1H), 4.45-4.62 (m, 1H), 6.94 (t, 1H), 7.09 (t, 1H), 7.21 (br s, 1H), 7.32 (d, 1H), 7.57 (d, 1H), 7.15-7.27 (m, 5H) and nine $D_2O$ exchangable protons. Anal calcd for $C_{40}H_{55}N_7O_9 \cdot 2H_2O$: C 59.04, H 7.25, N 12.05., Found: C 58.96, H 7.12, N 11.58.

Example 34

Boc-5-amino-2-(3′-indolylmethyl)-2-pentenoyl-Leu-Asp-Phe-$NH_2$

Example 34a

Boc-5-amino-2-(3′-indolylmethyl)-2-pentenoic acid

To a solution of the compound of example 32b (400 mg, 0.92 mmol) in 18 ml EtOH was treated with 400 mg of 10% Pd on carbon, system flushed with nitrogen and cooled in an ice bath, then 1,4-cyclohexadiene (2.6 ml, 28 mmol) was added to the above reaction mixture. The mixture was stirred at room temperature under nitrogen atmosphere for 4 h. Filtration of the reaction mixture through a celite pad, followed by several washings with EtOH and evaporation, afforded an oily residue which was purified by flash silica gel eluting with EtOAc/hexane (1/1,v/v, with 1% HOAc) to afford the acid (269 mg, 85%): MS m/e 344 (M+)., $^1H$ NMR($CDCl_3$, 300 MHz) $\delta$ 1.4 (s, 9H), 2.5 (q, 2H), 3.20 (q, 2H), 3.77 (br s, 2H), 4.60 (br t, 1H), 6.87 (br s, 1H), 6.96 (t, 1H), 7.16 (t, 1H), 7.18 (t, 1H), 7.32 (d, 1H), 7.62 (d, 1H), 7.90 (br.1H).

Example 34b

Boc-5-amino-2-(3′-indolylmethyl)-2-pent-enoic acid 2,4,5 trichlorophenyl ester

To a solution of acid in example 34a (195 mg,0.57 mmol) in 8 ml anhydrous $CH_2Cl_2$ was treated with 2,4,5-trichlorophenol (112 mg,0.57 mmol) and EDCI (160 mg,0.85 mmol). The reaction mixture was stirred for 24 h at room temperature under nitrogen atmosphere. The mixture was washed with 10% citric acid and brine. The organic layer was dried over $Na_2SO_4$ and concentrated to yield a residue that was purified by flash silica gel. Elution.with EtOAc/Hexane (1/5~1/1,v/v, with 1% HOAc) afforded the product (158 mg,54%) as an oil: $^1H$ NMR($CDCl_3$, 60 MHz) δ 1.45 (s, 9H), 2.58 (q, 2H), 3.28 (q, 2H), 3.85 (q, 2H), 4.80 (br, 1H), 7.2 (s, 1H), 7.5 (s, 1H), 6.8-7.8 (m, 6H), 10.1 (br,1H).

Example 34c

Boc-5-amino-2-(3′-indolylmethyl)-2-pentene-oyl-Leu-Asp-Phe-$NH_2$

A solution of Leu-Asp-Phe-$NH_2$•HCl (124 mg,0.29 mmol) in 5 ml anhydrous DMF was treated with DIEA (0.10 ml, 0.58 mmol), HOBt (30 mg,0.22 mmol) and a solution of the compound of example 34b (153 mg,0.29 mmol) in 5 ml of anhydrous DMF at 0 °C. The resulting solution was stirred at room temperature under nitrogen atmosphere for 24 h. The mixture was then poured into a rapidly stirring solution of cold 10% citric acid causing a white precipitate, which, after collection, was recrystallized from EtOAc to yield the product (124 mg, 60%) as a white solid. mp 179-181 °C., $[\alpha]^{25}_D$ = -13.7° (c=0.58, DMF)., MS(FAB+) m/e 719$(M+H)^+$. $^1H$ NMR(DMSO-$d_6$, 300 MHz) δ 0.72 & 0.72 (2d, 6H), 1.36 (s, 9H), 1.25-1.47 (m, 3H), 2.35 (q, 2H), 2.55 (dd, 2H), 2.88 (dd, 2H), 2.95-3.08 (m, 2H), 3.63 (q, 2H), 4.28-4.40 (m, 2H), 4.48 (q, 1H), 6.21 (t, 1H), 6.93 (t, 1H), 6.95 (d, 1H), 6.97 (br s, 1H), 7.04 (t, 1H), 7.15-7.25 (m, 6H) and eight $D_2O$ exchangable protones., Anal Calcd for $C_{38}H_{50}N_6O_8$: C 63.47, H 7.01, N 11.69., Found: C 63.21, H 7.24, N 11.47.

Example 35

$N^\alpha$-Boc-$N^{in}$-propionyl-Trp-Leu-Asp-Phe-$NH_2$

Example 35a

$N^\alpha$-Boc-$N^{in}$-propionyl-Trp benzyl ester

A solution of BOC-Trp benzyl ester (200 mg,0.5 mmol), $(n\text{-}Bu)_4NHSO_4$ (30 mg, cat.) and 40% NaOH aqueous solution (2 ml) was stirred vigorously in 6 ml benzene. Propionic anhydride (excess) was added in portions to the above mixture and the progress of reaction was monitored by tlc. As soon as the starting material was consumed as indicated by tlc, the reaction mixture was poured into 10 ml distilled water and was extracted with benzene.. The organic layer was dried over $Na_2SO_4$, filtered and the solvent was evaporated. The residue was purified by flash silica using EtOAc/hexane (1/5 ~ 1/3,v/v) as eluents to afford the product 110 mg (48%) as a white solid. $^1H$ NMR($CDCl_3$, 60 MHz) δ 1.24 (t, 3H), 1.20 (s, 9H), 2.77 (q, 2H), 3.22 (d, 2H), 4079 (m, 1H), 5.17 (br s, 2H), 7.0-7.6 (m, 9H), 8.15-8.30 (m, 1H).

Example 35b

$N^{\alpha}$-Boc-$N^{in}$-propionyl-Trp-trichlorophenyl ester

The same procedure as described for example 32b was used to provide the active ester in 63% yield from the compound of example 35a. $^1$H NMR($CDCl_3$,60 MHz) δ 1.32 (t, 3H), 1.38 (s, 9H), 2.90 (q, 2H), 3.30-3.60 (br,2H), 4.90-5.25 (m, 1H), 7.1 (s, 1H), 7.20-7.70 (m, 5H), 8.30-8.70 (m, 1H).

Example 35c

$N^{\alpha}$-Boc-$N^{in}$-propionyl-Trp-Leu-Asp-Phe-$NH_2$

The same procedure as described for example 34c was used to provide the tetrapeptide in 67% yield from the compound of example 35b. mp 207-208 °C, MS(FAB-) m/e 733 $(M-H)^-$, $^1$H NMR(DMSO-$d_6$/300 MHz) δ 0.85 (2d, 6H), 1.18 (t, 3H), 1.30 (s, 9H), 1.27-1.52 (m, 2H), 1.55-1.67 (m, 1H), 2.56 (dd, 2H), 2.50 (br,1H), 2.78-3.15 (m, 5H), 4.25-4.42 (m, 3H), 4.52 (q, 1H), 7.1-7.40 (m, 10H) and seven $D_2O$ exchangable protons. Anal calcd for $C_{38}H_{50}N_6O_9 \cdot 0.5H_2O$: C 61.34, H 6.91, N 11.30. Found: C 61.47, H 7.13, N 11.13.

Example 36

3-(3-Indolyl)propionyl-Nle-Asp-Phe-$NH_2$

Example 36a

3-(3-Indolyl)propionyl-Nle-Asp(OBn)-Phe-$NH_2$

A solution of Nle-Asp-Phe-$NH_2$.TFA [M. Rodriguez, et al, J. Med. Chem. , 30 , 1366 (1987)](202 mg,0.34 mmol) in 1ml THF was treated with DIEA (61 µl, 0.35 mmol), HOBt(cat.) and a solution of 2,4,5-trichlorophenol-(3-indolyl)-propionate (H. Gregory, J.S. Morley, J.Chem.Soc. (C) , 1968, 910) (129 mg, 0.35 mmol) in 1 ml THF at 0 °C. The resulting solution was stirred for 24 h with warming to ambient temperature under a nitrogen atmosphere. The reaction mixture was poured into a rapidly stirred solution of cold 10% citric acid, causing a white precipitate. The solid was collected by filtration and triturated with hot EtOAc to yield (185 mg, 83%). MS(FAB+) m/e 654$(M+H)^+$, $^1$H NMR(DMSO-$d_6$, 300 MHz) δ 0.82 (t, 3H), 1.20 (m, 4H), 1.40-1.60 (m, 2H), 2.50-2.65 (m, 4H), 2.81 (dd, 1H), 2.85-2.93 (m, 2H), 3.02 (dd, 1H), 4.18 (m, 1H), 4.36 (m, 1H), 4.59 (m, 1H), 5.08 (s, 2H), 6.99 (t, 1H), 7.05 (t, 1H), 7.08 (s, 1H), 7.15-7.40 (m, 13H), 7.50 (d, 1H), 7.83 (d, 1H), 8.10 (d, 1H), 8.34 (d, 1H), 10.77 (s, 1H).

Example 36b

3-(3-Indolyl)propionyl-Nle-Asp-Phe-$NH_2$

A solution of the compound of example 36a (138 mg,0.21 mmol) in 8 ml MeOH and 6 ml DMF was treated with 5% Pd/C (69 mg). The reaction mixture was stirred under hydrogen atmosphere for 30 min. Filtration of the catalyst through a celite pad, followed by several washings with MeOH, afforded a filtrate which after concentration was recrystallized from EtOAc to yield the product (70 mg, 59%). mp 213-218 °C, $[\alpha]^{25}_D$ = -41.4° (c=0.5,DMSO), MS(FAB+) m/e 564(M+H)$^+$, $^1H$ NMR(DMSO-$d_6$, 300 MHz) δ 0.81 (t, 3H), 1.1-1.25 (m, 4H), 1.35-1.60 (m, 2H), 2.40-2.66 (m, 4H), 2.80-2.95 (m, 3H), 3.05 (dd, 1H), 4.17 (m, 1H), 4.32 (m, 1H), 4.45 (m, 1H), 6.94 (t, 1H), 7.05 (t, 1H), 7.08 (s, 1H), 7.10=7.30 (m, 7H), 7.31 (d, 1H), 7.51 (d, 1H), 7.88 (d, 1H), 8.11 (d, 1H), 8.30 (d, 1H), 10.78 (s, 1H), Anal cald for $C_{30}H_{37}N_5O_6 \cdot H_2O$: C 61.93, H 6.77, N 12.04.,Found: C 62.13, H 6.53, N 11.82.

## Example 37

### 8-[N-(t-Boc)amino]-2-(3-indolemethyl)-2-octenoyl-Leu-Asp-Phe-$NH_2$

## Example 37a

### Methyl-8-[N-(t-butoxycarbonyl)amino]-2-(3′-indolylmethyl)2-octenoate

A solution of 6-(BOC-amino)hexanol (2.0 g,9.20 mmol) in 5 ml of anydrous $CH_2Cl_2$ was added to a suspension of dried, powdered 4A molecule sieves (10 g) and PDC (16 g,42.5 mmol) in $CH_2Cl_2$ (100 ml) at room temperature and stirred for 1 h. Dilution of the reaction mixture with diethyl ether, filtration through a celite pad and evaporation afforded a brown oil, which was purified by flash silica gel using EtOAc/hexane (1/2) as eluents to give desired aldehyde (1.24 g, 62%) which was immediately treated with methyl α-(3-indolemethyl)-α-(triphenyl phosphoranylidine)acetate (7.5 g,16 mmol) in 50 ml $CH_2Cl_2$ and allowed to reflux for 4 days. The volume of reaction solvent was reduced to 8 ml and chromatographed on flash silica gel using EtOAc/toluene (1/7 ~ 1/4,v/v) as eluents to afford the product (1.6 g,43%) as a pale yellow oil. $^1H$ NMR($CDCl_3$, 60 MHz) δ 1.37 (m, 6H), 1.47 (s, 9H), 2.30 (q, 2H), 3.06 (br d, 2H), 3.71 (s, 3H), 3.81 (s, 2H), 4.50 (br s, 1H), 6.72-7.77 (m, 6H), 8.33 (br s, 1H).

## Example 37b

### Boc-8-amino-2-(3′-indolylmethyl)-2-octenoic acid

A solution of the compound of example 37a (564 mg, 1.41 mmol) in 1.0 M NaOH/MeOH (32 ml) was heated to reflux for 4 h. Additional 1.0 M NaOH (10 ml) was added and the mixture was refluxed for an additional 3 h. The methanol was removed under reduced pressure and the aqueous layer was acidified with 2.0 N HCl (20 ml) and extracted with three portions of EtOAc. The combined extracts were dried over $Na_2SO_4$ and concentrated to afford an orange oil (yield not determined). $^1H$ NMR($CDCL_3$, 60 MHz) δ 1.32 (m, 6H), 1.43 (s, 9H), 2.17-2.50 (m, 1H), 2.75-3.30 (m, 3H), 3.40 (s, 1H), 3.79 (s, 1H), 5.49 (br,s, 1H), 6.77-7.77 (m, 5H), 8.30 (br,d, 1H), 8.87 (br,s, 1H).

## Example 37c

8-[N-(t-Boc)amino]-2-(3′-indolylmethyl)-2-octenoic acid 2,4,5-trichlorophenol ester

A solution of the crude acid of example 37b in 7 ml $CH_2Cl_2$ was treated with 2,4,5-trichlorophenol (330 mg, 1.69 mmol) and EDCI (380 mg, 1.98 mmol) and allowed to stir for 5 days at room temperature. The mixture was washed with 10% citric acid solution (2x), then brine. After drying, the solvent was evaporated and the resulting residue purified by flash silica gel using EtOAc/hexane (1/10~1/5,v/v) as eluents to afford a pale yellow oil (562 mg,70% from example 37a). MS(DCI) m/e 565$(M+H)^+$, $^1H$ NMR($CDCl_3$, 300 MHz) δ 1.3-1.4 (m, 4H), 1.45 (s, 9H), 1.47 (s, 2H), 2.0-2.1 (m, 1H), 2.42 (q, 1H), 3.0-3.15 (m, 3H), 3.90 (s, 1H), 4.47 (br s, 1H), 6.95-7.0 (m, 1H), 7.05-7.30 (m, 4H), 7.23 (s, 1H), 7.38 (t, 1H), 7.51 (d, 1H), 7.63 (d, 1H).

Example 37d

8-[N-(t-Boc)amino]-2-(3′-indolylmethyl)-2-octenoyl-Leu-Asp-Phe-$NH_2$

A solution of Leu-Asp-Phe-$NH_2$ (86 mg, 0.22 mmol) in 1 ml anhydrous DMF was treated with DIEA (38 µl, 0.22 mmol), HOBt (cat.) and a solution of the compound of example 37c (125 mg, 0.22 mmol) in 1 ml anhydrous DMF at 0 °C. The solution was heated to 40 °C under nitrogen atmosphere over night. After cooling to room temperature, the mixture was poured into a rapidly stirring solution of cold 10% citric acid causing formation of a white precipitate which was collected and recrystallized from EtOAc give the product (94 mg, 56%) as a white solid. Analytical HPLC [Altex $C_{18}$; 50 mM $NH_4OAc$, (pH 4.5)/ACN linear gradient: 30% for 5 min, then 30-45% over 10 min; flow rate 1.0 ml/min] revealed a isomer ratio about 1/1. mp 166-169 °C, MS(FAB+) m/e 761$(M+H)^+$. $^1H$ NMR(DMSO-$d_6$, 300 MHz) δ 0.63-0.88 (m, 6H), 1.10-1.46 (m, 9H), 1.36 (s, 9H), 2.60-2.76 (m, 2H), 2.76-2.93 (m, 3H), 2.96-3.07 (m, 2H), 3.68 (dt, 1H), 4.27-4.52 (m, 2H), 5.38-5.47 and 6.29 (m & t, 1H) , 6.9-7.08 (m, 3H), 7.11-7.27 (m, 5H), 7.29 (d, 1H), 7.49 (d, 1H), 7.72 (d, 1H), 7.79 (d, 1H), 7.95 (dd, 1H), 10.74 (br,s, 1H), Anal cald for $C_{41}H_{56}N_6O_8 \cdot 0.5H_2O$: C 63.95, H 7.48, N 10.92, Found: C 63.81, H 7.58, N 10.77

Example 38

3-(3-Indolyl)propionyl-Leu-Asp-Phe-$NH_2$

Example 38a

N-Methoxy-N-methyl-3-(3-Indolyl)propionamide

A solution of 3-(3-indolyl)propionic acid (950 mg,5.0 mmol) in 50 ml $CH_2Cl_2$ was treated with N,O-dimethylhydroxylamine•HCl (580 mg,5.9 mmol), DIEA (1.03 ml, 5.9 mmol), DCC (1.44 g, 7.0 mmol) and DMAP (300 mg,2.4 mmol) at 0 °C. The reaction mixture was allowed to warm to ambient temperature and stirred for 4 h. The precipitated dicyclohexyl urea was removed by filtration. The filtrate was washed with 10% aqueous citric acid solution (2x), dried over $Na_2SO_4$ and concentrated to yield a brown oil. The crude was purified by flash silica gel using EtOAc/hexane (1/15~1/2,v/v) as eluents to give corresponding dimethylhydroxyamide as a clear yellow oil (1.14 g,98%). $^1H$ NMR($CDCl_3$, 60 MHz) δ 2.70-3.33 (m, 4H), 3.18 (s, 3H), 3.58 (s, 3H), 6.93-7.80 (m, 5H), 8.25 (br s, 1H).

Example 38b

3-(3-Indolyl)propionyl-Leu-Asp(OBn)-Phe-$NH_2$

LAH (190 mg, 5.0 mmol) was added to a solution of dimethyl hydroxyamide of example 38a (580 mg, 2.5 mmol) in 20 ml THF at -40 °C. The reaction mixture was allowed to stir, warming to -20 °C over 1 h at which time tlc indicated completion. The reaction was quenched with MeOH (3.5 ml) then 10% citric acid solution (50 ml) and extracted with $CHCl_3$(3x). The combined extracts were dried over $Na_2SO_4$ and concentrated to yield a brown oil. The crude product was purified on flash silica gel, eluting with EtOAc/Hexane (1/1,v/v), to afford a pale yellow oil (390 mg, 91%) which was used immediately for the next reaction. A solution of Leu-Asp(OBn)-Phe-$NH_2$•TFA (1.24 g, 2.08 mmol) in 3 ml absolute EtOH was treated with TEA (0.29 ml, 2.08 mmol) and a solution the 3-(3-indolyl)propionaldehyde (0.39 g, 2.25 mmol) in 4 ml EtOH. The reaction mixture was heated gently until a homogeneous solution was obtained. Acetic acid (119 µl, 2.08 mmol) was added and the solution was allowed to stir at room temperature for 10 min. $NaBH_3CN$ (0.26 g, 4.14 mmol) was added slowly in one portion and stirred overnight at room temperature. The solvent was removed under reduced pressure and the residue was treated with 1.0 N NaOH (25 ml) at 0 °C and extracted with EtOAc (4X). The extracts were washed with brine, dried over $Na_2SO_4$, and concentrated to yield a white foam which was purified by the Chromatotron on silica gel (chromatatron), eluting with 1~6% EtOH/$CHCl_3$ (v/v) on 225 mg portions, to produce the product as a pale yellow solid (484 mg, 31.5%). MS-(FAB+) m/e 640$(M+H)^+$, $^1H$ NMR($CDCl_3$, 300 MHz) δ 0.81-1.0 (t, 6H), 1.20-1.35 (m, 2H), 1.40-1.71 (m, 5H), 1.78-1.91 (m, 2H), 2.28-2.56 (m, 2H), 2.71-2.88 (m, 3H), 2.88-3.10 (m, 3H), 4.50 (q, 1H), 4.62-4.73 (m, 1H), 5.10 (s, 2H), 5.31 (s, 1H), 6.13 (s, 1H), 6.95 (s, 2H), 7.03-7.41 (m, 12H), 7.60 (d, 1H), 8.06 (s, 1H), 8.13 (d, 1H).

Example 38c

3-(3-Indolyl)propionyl-Leu-Asp-Phe-$NH_2$

A solution of the peptide of example 38b (68 mg, 0.1 mmol) in 1 ml MeOH was treated with 10% Pd/C (7 mg) and the resulting mixture was stirred overnight under a hydrogen atmosphere. The catalyst was removed by filtration and the filtrate was concentrated under reduced pressure. The residue was triturated with hot EtOAc to yield the product (55%). mp 133-136 °C, $[\alpha]^{25}_D$ = -24.8° (c=0.025,MeOH), MS(DCl) m/e 550$(M+H)^+$, $^1H$ NMR($CD_3OD$,300 MHz) δ 0.82-0.99 (m, 6H), 1.29 (s, 1H), 1.50 (br s, 2H), 1.67 (br s, 2H), 2.05 (br s, 2H), 2.57 (dd, 1H), 2.71-3.01 (m, 6H), 3.13-3.25 (m, 1H), 3.63-3.72 (m, 1H), 4.53 (dd, 1H, 4.71 (t, 1H), 6.97-7.36 (m, 11H), 7.44-7.59 (m, 2H), Anal cald for $C_{30}H_{39}N_5O_5$•$1.5H_2O$: C 62.47, H 7.36, N 12.14, Found: C 62.41, H 6.87, N 11.91.

Example 39

Boc-Gly-ψ[CH=CH]-Trp-Leu-Asp-Phe-$NH_2$

Example 39a

4-[N-(t-Boc)amino]-3-hydroxy-1-butene

To a suspension of dried, powdered 4A molecular sieves (18 g) and PDC (45 mg, 120 mmol) in 150 ml $CH_2Cl_2$ was added a solution of 2-[N-(t-butyoxycarbonyl)amino]ethanol (4.83 g, 30 mmol) in $CH_2Cl_2$. The mixture was allowed to stir at room temperature for 1.5 h, then diluted with ether (200 ml) and filtered through a silica gel pad, rinsing throughly with ether. The filtrate was concentrated to afford a clear pale yellow oil (3.46 g, 72%). The aldehyde was used immediately for the next reaction without purification. To a cooled solution of the freshly prepared Boc-Glycinal (410 mg, 2.6 mmol) in 8 ml THF was slowly added 1.0 M vinyl magnesium bromide in THF (18ml) via syringe. The reaction mixture was allowed to stir for 1 h with warming to -10 °C. The reaction was quenched with sat. $NH_4Cl$ (20 ml) and was extracted with $CHCl_3$ (x4). The extracts were dried and concentrated. The crude oil was purified by flash silica gel, eluting with EtOAc/hexane (1/5~1/1,v/v) to give a clear pale yellow oil (380 mg,78%). MS(DCI) m/e 205$(M+NH_4)^+$. $^1H$ NMR($CDCl_3$, 300 MHZ) δ 1.45 (s, 9H), 2.54 (br s, 1H), 3.10 (dt, 1H), 3.30-3.43 (m, 1H), 4.24 (br s, 1H), 4.92 (br s, 1H), 5.21 (d, 1H), 5.35 (d, 1H), 5.86 (ddd, 1H), Anal cald for $C_9H_{17}NO_3$: C 57.72, H 9.17, N 7.48. Found: C 57.85, H 9.19, N 7.42.

## Example 39b

### 4-[N-(t-Boc)amino]-3-acetoxy-1-butene

A solution of the allylic alcohol of example 39a (1.94 g, 10.4 mmol) in acetic anhydride (22 ml, 230 mmol) was treated with pyridine (2 ml) and DMAP (50 mg) at 0 °C. The reaction mixture was warmed to ambient temperature, then stirred overnight. The mixture was poured into cold aqueous $NaHCO_3$ solution and extracted with $CHCl_3$ (4X). The extracts were dried over $Na_2SO_4$ and concentrated. The crude oil was purified by flash silica gel, eluting with EtOAc/hexane (1/10~1/3,v/v), to give the product as a colorless oil (1.92 g, 81%). MS(DCI) m/e 247$(M+NH_4)^+$, $^1H$ NMR($CDCl_3$, 300 MHz) δ 1.45 (s, 9H), 2.10 (s, 3H), 3.28 (dt, 1H), 3.43 (dt, 1H), 4.72 (br s, 1H), 5.26 (d, 1H), 5.32 (d, 1H), 5.78 (ddd, 1H).

## Example 39c

### Benzyl 4-acetoxy-5-[N-(t-Boc)amino]-2-(3-indolenemethyl)-*trans*-2-pentenoate

A solution of the compound of example 39b (1.15 g, 5.02 mmol) in 60 ml acetone was treated with $OsO_4$ (5 ml of 2.5% in t-BuOH) and N-methylmorpholine N-oxide (1.48 g,10.9 mmol) at room temperature. After stirring for 5 min, a solution of $NaIO_4$ (5.0 g, 23.4 mmol) in 37 ml $H_2O$ was added to the reaction mixture and allowed to stir at room temperature for 3.5 h. The mixture was poured into water (60 ml) and extracted with $CHCl_3$ (4X). The extracts were dried over $Na_2SO_4$ and concentrated. The crude product was purified by flash silica gel, eluting with EtOAc/hexane (1/3~2/1,v/v), to yield a pale yellow oil (1.02 g, 88%). To a solution of the freshly prepared aldehyde (1.02 g, 4.41 mmol) and phosphorane from example 32a (4.80 g, 8.9 mmol) in 22 ml $CH_2Cl_2$ was stirred at room temperature over night. The solvent was evaporated and the crude oil was purified by flash silica gel, eluting with EtOAc/hexane (1/10~1/3,v/v), to yield the product as a pale yellow oil (1.74 g, 80%). MS(DCI) m/e 510 $(M+NH_4)^+$, $^1H$ NMR($CDCl_3$, 300 MHz) δ 1.40 (s, 9H), 2.04 (s, 3H), 3.32 (t, 2H), 3.94 (s, 2H), 4.66 (br s, 1H), 5.12 (s, 2H), 5.72 (dt, 1H), 6.67 (d, 1H), 6.97 (s, 1H), 7.10 (t, 1H), 7.14-7.22 (m, 3H), 7.24-7.30 (m, 3H), 7.33 (d, 1H), 7.64 (d, 1H), 8.0 (s, 1H).

## Example 39d

Benzyl 4-hydroxy-5-[N-(t-Boc)amino]-2-(3-indolenemethyl)-*trans*-2-pentenoate

To a solution of the compound of example 39c (1.51 g, 3.06 mmol) in 75 ml MeOH was added $Na_2CO_3$ (26.0 g) and stirred at room temperature for 1 h. $Na_2CO_3$ was removed by filtration, rinsing throughly with $CH_2Cl_2$. The filtrate was poured into saturated aqueous $NH_4Cl$ (100 ml), the layers were separated and the aqueous layer was extracted further with $CHCl_3$ (3x). The extracts were dried over $Na_2SO_4$ and concentrated. The crude oil was purified by flash silica gel, eluting with EtOAc/Hexane (1/5~1/1,v/v), to give the product as a pale yellow oil (1.03 g, 75%). MS(FAB) m/e 451$(M+H)^+$, $^1H$ NMR($CDCl_3$, 300 MHz) $\delta$ 1.43 (s, 9H), 2.97 (s, 1H), 3.10-3.32 (m, 2H), 3.87 (s, 2H), 4.65-4.76 (m, 1H), 4.85 (br s, 1H), 5.16 (s, 2H), 6.79 (d, 1H), 6.88 (s, 1H), 7.11 (t, 1H), 7.19 (t, 1H), 7.22-7.31 (m, 6H), 7.33 (d, 1H), 7.61 (d, 1H), 7.99 (s, 1H).

Example 39e

Benzyl 4-bromo-5-[N-(t-Boc)amino]-2-(3-indolenemethyl)-*trans*-2-pentenoate

A solution of $CBr_4$ (5.50 g, 16.6 mmol) in 72 ml THF was treated with $PPh_3$ (4.12 g, 15.7 mmol) at 0 °C and allowed to stir for 15 min. A solution of the compound of example 39d in 10 ml THF was added to the above complex via syringe. The reaction mixture was stirred for 2 h with warming to room temperature then poured into cold 10% citric acid solution (150 ml) and extracted with $CHCl_3$(x4). The combined extracts were washed with 10% aqueous sodium thiosulfate (2x). The thiosulfate aqueous layer was back extracted with $CHCl_3$ (2x), the extracts combined, dried over $Na_2SO_4$ and concentrated. The crude oil was purified by flash silica gel, eluting with EtOAc/hexane (1/5~1/1,v/v), to give the product as a yellow oil (0.75 g, 71%). MS(DCI) m/e 532,530$(M+NH_4)^+$, $^1H$ NMR($CDCl_3$, 300 MHz) $\delta$ 1.42 (s, 9H), 3.54 (t, 2H), 3.88 (s, 2H), 4.90 (br s, 1H), 5.01 (dt, 1H), 5.15 (s, 2H), 6.92 (s, 1H), 6.93 (d, 1H), 7.13 (t, 1H), 7.17-7.25 (m, 3H), 7.25-7.31 (m, 3H), 7.35 (d, 1H), 7.63 (d, 1H), 7.98 (br s, 1H).

Example 39f

Benzyl 4-bromo-5-[N-(t-Boc)amino]-2-(3-indolenemethyl)-*trans*-2-pentenoate

A solution of the compound of example 39e (0.75 g, 1.46 mmol) in 36 ml glacial acetic acid was treated with zinc dust (3.8 g) and stirred at room temperature for 50 min. The mixture was diluted with $Et_2O$ (50 ml) and the zinc dust was removed by filtration and rinsed throughly with $Et_2O$. The filtrate was washed with $H_2O$ (2x) and the aqueous extracts were backwashed with $Et_2O$ (2x). The organic phase was washed with brine, dried over $Na_2SO_4$ and concentrated. The crude oil was purified by flash silica gel, eluting with EtOAc/hexane (1/5~1/3,v/v), to give the product as a colorless oil (0.61 g, 97%). MS(DCI) m/e 452$(M+NH_4)^+$, m/e calculated for $C_{26}H_{30}N_2O_4$: 435.2284, Found: 435.2280., $^1H$ NMR($CDCl_3$, 300 MHz) $\delta$ 1.44 (s, 9H), 2.99 (dd, 1H), 3.25 (dd, 1H), 3.48 (q, 1H), 3.68 (br.t, 2H), 4.40 (br s, 1H), 5.05 (s, 2H), 5.51 (dt, J=6 and 15Hz, 1H), 5.73 (dd, J=8 and 15Hz, 1H), 6.89 (s, 1H), 7.11 (t, 1H), 7.14-7.22 (m, 3H), 7.26-7.32 (m, 3H), 7.34 (d, 1H), 7.56 (d, 1H), 7.96 (s, 1H).

Example 39g

Boc-Gly$\psi$[CH=CH]-Trp-OH

To a suspension of 10% Pd/C (380 mg) in 1,4-cyclohexadiene (16.4 ml) was added a solution of the compound of example 39f (380 mg, 0.87 mmol) in 17 ml MeOH and stirred at room temperature for 5 h. The catalyst was removed by filtration and the filtrate was concentrated under reduced pressure. The oily residue was taken up in $Et_2O$ (20 ml) and washed with 0.5N NaOH (5x20 ml). The aqueous layers were backwashed with $Et_2O$ (2x25 ml). The combined organic extracts were washed with brine, dried over $Na_2SO_4$, and concentrated to recover the unreacted starting substrate (277 mg,73%). The aqueous extracts were acidified with cold 2 N HCl (30 ml) until pH 2 was attained, then extracted with EtOAc (4x100 ml). These extracts were washed with brine, dried over $Na_2SO_4$ and concentrated to yield the product as a yellow oil (43 mg, 14%). MS(FAB) m/e 345$(M+H)^+$, $^1H$ NMR($CDCl_3$, 300 MHz) δ 1.44 (s, 9H), 2.99 (dd, 1H), 3.26 (dd, 1H), 3.43 (t, 1H), 3.68 (br s, 2H), 4.48 (br s, 1H), 5.54 (dt, J=5 and 15Hz, 1H), 5.71 (dd, J=11, and 15Hz, 1H), 6.99 (s, 1H), 7.11 (t, 1H), 7.19 (t, 1H), 7.34 (d, 1H), 7.58 (d, 1H), 8.07 (s, 1H).

Example 39h

Boc-Gly-ψ[CH=CH]-Trp-2,4,5-trichlorophenol ester

The same procedure as described in example 32d was used to provide the desired active ester in 63% yield from the comound of example 39. MS m/e 522, 524(M+), $^1H$ NMR ($CDCl_3$, 300 MHz) δ 1.46 (s, 9H), 3.12 (dd, 1H), 3.40 (dd, 1H), 3.65-3.81 (m, 1H), 4.50 (br s, 1H), 5.67 (dt, J=5 and 15Hz, 1H), 5.83 (dd, J=8 and 15Hz, 1H), 6.89 (s, 1H), 7.07 (s, 1H), 7.15 (t, 1H), 7.22 (t, 1H), 7.38 (d, 1H), 7.49 (s, 1H), 7.61 (d, 1H), 8.10 (s, 1H).

Example 39i

Boc-Gly-ψ[CH=CH]-Trp-Leu-Asp-Phe-$NH_2$

The same procedure as described in example 34c was used to provide the product in 59% yield from the compound of example 39h. mp 194-196 °C, MS(DCI) m/e 719$(M+H)^+$, m/e cald for $C_{30}H_{50}N_6O_8$: 719.3768, Found: 719.3749, $^1H$ NMR ($CD_3OD$, 300 MHz) δ 0.62 (dd, 3H), 0.84 (dd, 3H), 1.15-1.23 (m, 1H), 1.35-1.57 (m, 2H), 1.42 (d, 9H), 2.50-2.84 (m, 2H), 2.84-3.01 (m, 2H), 3.08-3.28 (m, 2H), 3.35-3.48 (m, 1H), 3.59 (dd, 2H), 4.18-4.29 (m, 1H), 4.48-4.59 (m, 2H), 5.47-5.80 (m, 2H), 6.94-7.03 (m, 2H), 7.06 (t, 1H), 7.12-7.27 (m, 5H), 7.30 (d, 1H), 7.54 (d, 1H), Anal Cald for $C_{38}H_{50}N_6O_8 \cdot 1.5H_2O$: C 71.17, H 7.18, N 11.27, Found: C 61.33, H 7.05, N 11.18.

Example 40

Boc-Trp-Leu-Asp-Phe-$OCH_3$

Example 40

Boc-Trp-Leu-Asp-Phe-$OCH_3$

Z-Leu-Asp-Phe-$OCH_3$ (Agarwal, K. L., Kenner, G. W., and Sheppard, R. C. J. Chem. Soc. (C) 1968 1384-1391), (3 g, 5.5 mmol) was dissolved in 30 mL of MeOH and 10 mL of 88% $HCO_2H$, then 0.5 g of moist 20% $Pd(OH)_2$/C and 3 drops of conc. $NH_4OH$ were added. After stirring for 12 h at room temp., the mixture was filtered, the filtrant was washed with MeOH and $H_2O$, and the filtrate was concentrated under reduced pressure. The residue in 40 mL of $H_2O$ was washed twice with $CH_2Cl_2$, once with EtOAc, then the aqueous solution was adjusted to ca. pH 6 with conc. $NH_4OH$. The solution was chilled on ice, and the precipitate (0.72 g) was collected and washed with EtOH. Concentration of the mother liquors and recrystallization of the residue from EtOH afforded an additional 0.31 g of product. A solution of 0.6 g (1.48 mmol) of tripeptide in 3 mL of DMF containing 5 drops of $H_2O$ was added to a solution of the mixed anhydride prepared from Boc-Trp-OH (450 mg, 1.48 mmol), isobutyl chloroformate (191 µL, 201 mg, 1.48 mmol), and N-methylmorpholine (163 µL, 150 mg, 1.48 mmol) in THF at -15 °C, followed by $NEt_3$ (205 µL, 150 mg, 1.48 mmol). The mixture was allowed to warm to room temp. and stir for 18 h, then was concentrated at reduced pressure. The residue was partitioned between EtOAc and dil. aq. $KHSO_4$, and the separated aqueous phase was extracted with a fresh portion of EtOAc. The combined organic phases were washed successively with $H_2O$ and brine, then dried ($MgSO_4$) and concentrated. The residue was chromatographed on silica gel, eluting with $CHCl_3$/MeOH/HOAc (96:2:2, then 94:3:3). Pure fractions were combined and concentrated, and the residue was crystallized from $Et_2O$/hexane to afford 390 mg of pure product: mp 164-167 °C (decomp.). $^1H$-NMR (DMSO-$d_6$): δ 0.85 (t, J=6 Hz, 6H); 1.30 (s, 9H); 1.43 (m, 2H); 1.63 (m, 1H); 2.5 (m, 1H); 2.65 (dd, J=4.5,16 Hz, 1H); 2.85-3.0 (m, 3H); 3.08 (dd, J=4.5, 15 Hz, 1H); 3.56 (s, 3H); 4.21 (m, 1H); 4.30-4.48 (m, 2H); 4.57 (m, 1H); 6.82 (d, J=8 Hz, 1H); 6.96 (t, J=7.5 Hz); 7.05 (t, J=7.5 Hz, 1H); 7.31 (s, 1H); 7.16-7.35 (cm, 6H); 7.58 (d, J=7.5 Hz, 1H); 7.92 (d, J=7.5 Hz); 8.18 (m, 2H); 10.77 (s, 1H). MS ($FAB^+$): m/e 694 $(M+H)^+$; 594 $(M+H\text{-Boc})^+$. Anal calc. for $C_{36}H_{47}N_5O_9 \cdot 0.5\ H_2O$: C,61.50; H,6.89; N,9.97. Found: C,61.39; H,6.77; N,9.96.

## Example 41

### Ctp-Leu-Asp-Phe-$NHNH_2$

## Example 41a

### Boc-Phe-NHNH-Z

To a solution of Boc-Phe-OH (3.0 g, 11.2 mmol) and Z-$NHNH_2 \cdot$HCl (Boeshagen, H. and Ullrich, J. Chem. Ber. **1959**, *92*, 1478-80) (2.3 g, 11.2 mmol) in 100 mL of DMF at 0 °C were added diisopropylethylamine (1.95 mL, 1.45 g, 11.2 mmol) followed by 2-ethoxy-1-ethoxycarbonyl-1,2-dihydroquinoline (2.77 g, 11.2 mmol). The ice bath was removed and the solution was allowed to stir for 46 h, and then the solvent was evaporated under reduced pressure. The residue was taken up in EtOAc and washed successively with 1M $KHSO_4$, $H_2O$, sat. aq. $NaHCO_3$, and brine, dried ($Na_2SO_4$) and evaporated to give 4.0 g of a foam. Recrystallization from EtOAc/hexane provided 3.2 g (86%) of white crystalline product: mp 114-115.5 °C. $^1H$-NMR (DMSO): δ 1.27 (s, 9H), 2.76 (br d, J=12 Hz, 1H), 2.97 (br d, J=12 Hz, 1H), 4.18 (m, 1H), 5.09 (br s, 2H), 7.01 (br d, J=9 Hz, 1H), 7.15-7.40 (cm, 10 H), 9.29 (s, 1H), 9.95 (s, 1H). MS (EI) m/e 413 (M+), 357 $(M\text{-}C4H8)^+$. Anal. calc. for $C_{22}H_{27}N_3O_5$: C, 63.89; H, 6.58; N, 10.17. Found: C, 63.86; H, 6.54; N, 9.99.

## Example 41b

### Boc-Asp(Bn)-Phe-NHNH-Z

The product of example 41a (2.7 g, 13.0 mmol) was N-deprotected with 1:1 $CF_3CO_2H/CH_2Cl_2$ for 1 h in the usual manner. The crude salt from evaporation of the volatile components was dissolved in 20 mL of DMF, cooled on dry ice, and added to a solution of mixed anhydride prepared in the usual manner from Boc-Asp(Bn)-OH (1.96 g, 6.06 mmol), N-methylmorpholine (667 µL, 0.61 g, 6.06 mmol) and isobutyl chloroformate (786 µL, 0.83 g, 6.06 mmol) in 40 mL of THF at -15 °C. After addition of diisopropylethylamine (1.06 mL, 0.78 g, 6.06 mmol), the mixture was allowed to warm to room temp. and stir overnight. The reaction mixture was concentrated under reduced pressure, and the residue was taken up in EtOAc and washed with 1M $KHSO_4$, $H_2O$, sat. aq. $NaHCO_3$, and brine, dried ($Na_2SO_4$) and evaporated to 3.8 g of light yellow solid, which was recrystallized from EtOAc/hexane to afford 3.0 g (80%) of white crystalline solid, mp 140-141 °C. $^1$H-NMR (DMSO-$d_6$). δ 1.30 (s, 9H), 2.65 (dd, $J_1$ = 4.5 Hz, $J_2$ = 15 Hz, 1H), 2.82 (m, 1H), 3.02 (br d, J = 16 Hz, 1H), 4.33 (m, 1H), 4.52 (m, 1H), 5.08 (d, J = 5 Hz, 2H), 7.10 (cm, 15H), 7.90 (d, J = 7.5 Hz, 1H), 9.32 (s, 1H), 9.99 (s, 1H). MS ($FAB^+$): m/e 619 $(M+H)^+$, 563, 519, 429, 327. Anal. calc. for $C_{33}H_{38}N_4O_8.H_2O$: C,62.23; H,6.01; N,8.80. Found: C, 62.32; H, 5.62; N, 8.65.

## Example 41c

### Boc-Leu-Asp(Bn)-Phe-NHNH-Z

The product of example 41b (430 mg, 0.69 mmol) was N-deprotected with 1:1 $CF_3CO_2H/CH_2Cl_2$ for 1 h. After evaporation of the volatile components, the residue was taken up in $Et_2O$ and treated with 4N HCl/dioxane to precipitate 246 mg of the product as the hydrochloride salt. A solution of 203 mg (0.36 mmol) of the above salt in 2 mL of DMF at 0 °C was treated with Boc-Leu-N-hydroxysuccinimide ester (148 mg, 0.47 mmol) and diisopropylethylamine (69 µL, 51 mg, 0.40 mmol), then the mixture was allowed to warm to room temp. and stir for 12 h. The solution was concentrated, then diluted with EtOAc and washed with 1M $KHSO_4$, $H_2O$, sat. aq. $NaHCO_3$, and brine, dried ($Na_2SO_4$) and evaporated to 270 mg of yellow solid. Trituration with $Et_2O$ afforded 191 mg (73%) of product as a white solid, mp 122.5-124.5 °C. $^1$H-NMR (DMSO-$d_6$): δ 0.85 (m, 6H), 1.35 (m, 11H, includes 1.35, s, 9H), 1.57 (m, 1H), 2.55 (m, 1H), 2.78 (m, 2H), 3.0 (m, 1H), 3.9 (m, 1H), 4.48 (m, 1H), 4.65 (m, 1H), 5.07 (s, 2H), 5.08 (s, 2H), 6.90 (d, J = 9 Hz, 1H), 7.15-7.43 (cm, 15 H), 8.05 (m, 2H), 9.32 (s, 1H), 9.94 (s, 1H). MS ($FAB^+$): 754 $(M+Na)^+$, 732 $(M+H)^+$, 632, 566, 542, 510, 363, 319.

## Example 41d

### Ctp-Leu-Asp-Phe-$NHNH_2$

To a solution of HCl•H-Leu-Asp(Bn)-Phe-NHNH-Z (100 mg, 0.15 mmol, obtained by deprotection of the product of example 41c with HCl/dioxane in the usual manner), Ctp-OH (38 mg, 0.15 mmol), and N-methylmorpholine (20 µL, 18 mg, 0.18 mmol) in 2 mL of DMF at 0 °C under N2 was added diphenylphosphoryl azide (DPPA) (39 µL, 50 mg, 0.18 mmol). The reaction was allowed to proceed for 18 h at 0 °C and at room temp. for 2.5 days. Additional DPPA and NMM were added in aliquots until consumption of the amine component was complete, whereupon the product was precipitated by addition of $H_2O$, and collected by filtration to afford 120 mg of tan powder. The crude product was chromatographed over silica gel eluting with $MeOH/CHCl_3$ (3:97, then 5:95) to provide 75 mg (60%) of product. MS ($FAB^+$): m/e 880 $(M + Na)^+$, 858 $(M + H)^+$. A total of 57 mg of the above material in 2 mL of DMF was stirred with 25 mg of 5% Pd on $BaSO_4$ under a hydrogen atmosphere for 1.5 h at room temp., then the mixture was filtered and concentrated. The crude product was purified by preparative HPLC (Dynamax $C_{18}$, elution with $CH_3CN$/0.05% aq. $NH_3$ in a linear gradient from 10% to 40% $CH_3CN$ over 30 min.) Combination and lyophilization of fractions judged pure by analytical HPLC afforded 11 mg of product, mp ca. 205 °C (d).

$^1$H-NMR (DMSO-$d_6$): δ 0.85 (t, J=7 Hz, 6H), 1.45 (m, 2H), 1.62 (m, 1H), 2.4 (m, obscured by solvent), 2.8 (dd, J=9 Hz and 15 Hz, 1H), 2.95 (m), 3.0-3.7 [obscured by HDO, spectrum rerun in presence of DCl shows for this region 2.7 (dd, J=6 and 18 Hz, 1H), 2.90 (m, 1H), 2.98 (m, 1H), 3.25 (m, 1H), 3.37 (m, 1H)], 4.20 (m, 2H), 4.33 (m, 1H), 4.47 (m, 1H), 6.75 (d, J=7.5 Hz, 1H), 6.90-7.05 (cm, 3H), 7.10-7.30 (cm, 8H), 8.05-8.30 (cm, 3H, includes 8.04, d, J=7.5 Hz), 9.22 (s, 1H), 10.94 (s, 1H). MS ($FAB^+$): m/e 634 $(M + H)^+$, 602, 455, 340, 227.

## Example 42

## Boc-Trp-Leu-Asp-Trp-$NH_2$

Boc-Trp-Leu-Asp(phenacyl)-Phe-O-polystyrene resin was assembled using a Biosearch SAM 2 automatic solid phase peptide synthesizer starting with 1.50 g of Boc-Trp-O-resin (0.26 mmol/g, 1% crosslinked polystyrene, 200-400 mesh) using the following protocol: two cycles of deblock ($CF_3CO_2H/CH_2Cl_2$/anisole/dimethyl phosphite, 50: 45.5: 2.5: 2); two cycles of alternating $CH_2Cl_2$ wash and DMF wash; two cycles of base wash (10% diisopropylethylamine in $CH_2Cl_2$); single 1 h coupling of Boc-amino acid (3.5 fold excess, concentration 0.4M in $CH_2Cl_2$), activated by in-line mixing with an equimolar quantity of 0.4 M diisopropylcarbodiimide in DMF; capping step with 0.3 M acetylimidazole in DMF. All reagents and materials were commercially available except Boc-Asp(phenacyl)-OH (Yang, C. C. and Merrifield, R. B. *J. Org. Chem.* **1976**, *41*, 1032-1041). The crude peptide resin was gently rotated in the presence of 10 mL of 2 M benzeneselenol in DMF for 72 h under $N_2$, nitrogen atmosphere then the resin was filtered and washed three times with fresh portions of DMF, three times with $CH_2Cl_2$, then dried under reduced pressure. Using a pressure vessel, the peptide was cleaved from the resin by ammonolysis at room temp. for 72 h with 2,2,2-trifluoroethanol saturated at -20 °C with gaseous ammonia, to afford 59 mg of crude peptide. The recovered resin was resubjected to the above conditions to provide an additional 62 mg of crude peptide. The combined fractions of crude peptide were purified by preparative HPLC (Dynamax $C_{18}$, elution with $CH_3CN$/0.1% aq. $NH_3$, gradient from 10% to 40% $CH_3CN$ over 30 min). Pure fractions were combined and lyophilized to afford 69 mg of pure product, mp 186-191 °C. $^1$H-NMR (DMSO-$d_6$). δ 0.82 (t, J=7 Hz, 6H), 1.15 (br s), 1.3 (s, 9H), 1.4 (m, 2H), 1.6 (m, 1H), 2.41 (dd, J=6Hz and 16 Hz, 1H), 2.56, (partially obscured by solvent peak), 2.84-3.2 (cm, 6H), 4.21 (m, 1H), 4.35 (m, 2H), 4.47 (m, 1H), 6.73 (d, J=8 Hz, 1H), 6.95 (t, J=7.5 Hz,2H), 7.0-7.5 (cm, 5H), 7.30 (d, J=8 Hz, 2H), 7.45 (br s, 1H), 7.55 (d, J=8 Hz, 1H), 7.58 (d, J=8 Hz, 1H), 7.98 (d, J=8 Hz, 1H), 8.10, (br d, J=7.5 Hz, 1H), 8.26 (d, J=8 Hz, 1H), 10.77 (s, 1H), 10.92 (s, 1H). MS (FAB): m/e 716 $(M+H)^-$, 642, 586, 412, 341. Amino acid analysis (hydrolysis in presence of thioglycolic acid): Leu, 1.0; Trp, 1.76; Asp, 0.81.

## Example 43

## Boc-Trp-Leu-Asp-Cha-$NH_2$

## Example 43a

## HCl Asp-Cha-$NH_2$

A solution of $CCK_2$, Asp-Phe-$NH_2$, (500 mg, 1.79 mmol) in HOAc (83 mL) and 2N HCl (17 mL) was hydrogenated over Pt black (100 mg) for 2.25 h at room temp and 4 Atm. The mixture was filtered and evaporated to dryness to afford 590 mg of product. $^1$H NMR(DMSO-$D_6$) δ 0.89 (m, 2H), 1.16 (m, 3H), 1.35

(m, 1H), 1.50 (m, 2H), 1.65 (m, 5H), 2.75 (dd, J=8 and 18 Hz, 1H), 2.91 (dd, J=4.5 and 5 Hz, 1H), 4.10 (m, 1H), 4.23 (m, 1H), 7.05 (s, 1H), 7.38 (s, 1H), 8.58 (d, J=7.5 Hz, 1H). MS ($FAB^+$): m/e 286 $(M+H)^+$, 269, 241.

Example 43b

Boc-Trp-Leu-Asp-Cha-$NH_2$

The mixed anhydride from Boc-Trp-Leu-OH (372 mg ,0.89 mmol) and isobutyl chloroformate was formed in 4 mL of THF at -15 °C in the usual manner, then a pre-chilled solution of HCl•H-Asp-Cha-$NH_2$ - (286 mg, 0.89 mmol) and $NEt_3$ (246 µL, 1.76 mmol) in 6 mL of DMF was added. The mixture was stirred at -15 °C for 20 min., then the cooling bath was removed and stirring was continued for an additional 90 min. The reaction mixture was concentrated, the product was precipitated by addition of $H_2O$ abd sat. aq. $KHSO_4$, collected by filtration, washed with $H_2O$, and dried in vacuo over $P_2O_5$, mp 195-197 °C. $[\alpha]_D$= -44.2 (c=0.5, DMF). $^1$H-NMR (DMSO-$d_6$): δ 0.85 (t, J=7 Hz, 6H), 1.05-1.75 (cm, 25 H, includes 1.28 [s, Boc]), 2.50 (1H, plus solvent), 2.75 (dd, J=8 and 16 Hz, 1H), 2.90 (dd, J=8 and 15 Hz, 1H), 3.07 (dd, J=4 and 14 Hz, 1H), 4.19 (m, 2H), 4.35 (m, 1H), 4.53 (q, J=7 Hz, 1H), 6.81 (d, J=8 Hz, 1H), 6.95 (t, J=7 Hz, 1H), 7.04 (m, 2H), 7.10 (s, 1H), 7.15 (s, 1H), 7.30 (d, J=8 Hz, 1H), 7.57 (d, J=8 Hz, 1H), 7.76 (d, J=9 Hz, 1H), 7.92 (d, J=8 Hz, 1H), 8.34 (d, J=7.5 Hz, 1H). MS ($FAB^+$): m/e 685 $(M+H)^+$, 585, 171. Calc. for $C_{35}H_{52}N_6O_8$: C, 61.37; H, 7.66; N, 12.28. Found: C, 61.15; H, 7.62; N, 12.15.

Example 44

Boc-Trp-Leu-Asp-β-Nal-$NH_2$

Example 44a

Boc-β-Nal-$NH_2$

The product was prepared in 96% yield by reaction of the mixed anhydride from Boc-β-Nal-OH (682 mg, 2.17 mmol) with conc. $NH_4OH$ according to the procedure of Rzeszotarska, B., Makowski, M., and Kubica, Z. Org. Prep. Proc. Int. **1984**, *16*, 136-139. $^1$H-NMR ($CDCl_3$): δ 1.40 (s, 9H), 3.24 (m, 2H), 4.47 (m, 1H), 5.10 (br s, 1H), 5.37 (br s, 1H), 5.75 (br s, 1H), 7.49 (dd, J=2 Hz and 9 Hz, 1H), 7.47 (m, 2H), 7.69 (s, 1H), 7.80 (m, 3H). MS (DCI): m/e 315 $(M+H)^+$, 259, 215.

Example 44b

HCl Asp(Bn)-β-Nal-$NH_2$

The product of example 44a (700 mg, 2.23 mmol) was N-deprotected with 4N HCl/dioxane in the usual manner. The mixed anhydride from Boc-Asp(Bn)-OH (720 mg, 2.23 mmol) and isobutyl chloroformate was formed at -15 °C in THF in the usual manner, then a prechilled solution of amine salt and an equimolar

amount of NEt3 in DMF was added. The reaction mixture was stirred for 30 min. at -15 °C then the cooling bath was removed and stirring was continued. After several hours, the reaction mixture was concentrated, the crude product was precipitated by addition of $H_2O$ and sat. aq. $KHSO_4$ and collected by filtration, then dried under vacuum to provide 900 mg of crude product which was N-deprotected with 4N HCl/dioxane in the normal manner. Trituration of the crude salt with $Et_2O$/hexane provided the pure salt. $^1$H-NMR (DMSO-$d_6$): δ 2.83 (dd, J=9 and 17 Hz, 1H), 2.99 (m, 2H), 3.22 (dd, J=4.5 and 15 Hz, 1H), 4.09 (dd, J=3 and 11 Hz, 1H), 4.58 (m, 1H), 5.15 (d, J=2 Hz, 2H), 7.20 (s, 1H), 7.39 (m, 4H), 7.48 (m, 3H), 7.61 (s, 1H), 7.75 (s, 1H), 7.85 (m, 3H), 8.12 (br s, 3H), 8.73 (d, J=9 Hz, 1H) MS (DCI): m/e 420 $(M+H)^+$, 329, 312, 286, 260, 243, 217, 195, 178.

## Example 44c

### Boc-Trp-Leu-Asp(Bn)-β-Nal-$NH_2$

The mixed anhydride from Boc-Trp-Leu-OH (459 mg, 1.1 mmol) and isobutyl chloroformate was formed at -15 °C in THF in the usual manner, then a prechilled solution of the product of Example 44b (500 mg,1.1 mmol) and an equimolar amount of NEt3 in DMF was added. The reaction mixture was stirred for 30 min. at -15 °C,then the cooling bath was removed and stirring was continued for 1 h. The reaction mixture was concentrated, then partitioned between EtOAc and dil. aq. $KHSO_4$. The separated aqueous phase was extracted with a fresh portion of EtOAc, then the combined organic extracts were washed with $H_2O$, sat. aq. $NaHCO_3$, $H_2O$, and brine, then dried ($Na_2SO_4$), filtered and evaporated to afford 770 mg of crude product. Recrystallization from EtOH/$H_2O$ provided 660 mg (73%) of pure product. $^1$H-NMR (DMSO-$d_6$). δ 0.81 (m, 6H), 1.12 (br s), 1.29 (s, 9H), 1.39 (m, 2H), 1.60 (m, 1H), 2.62 (dd, J=8 and 17 Hz, 1H), 2.80 (dd, J=7 Hz and 16 Hz, 1H), 2.90 (dd, J=10 and 14 Hz, 1H), 3.05 (m, 2H), 3.17 (dd, J=4 and 17 Hz), 4.22 (m, 1H), 4.32 (m, 1H), 4.48 (m, 1H), 4.61 (m, 1H), 5.06 (s, 2H), 6.87 (d, J=8 Hz, 1H), 6.93 (t, J=8 Hz, 1H), 7.04 (t, J=7 Hz, 1H), 7.12 (s, 1H), 7.18 (s, 1H), 7.33 (m, 9H), 7.45 (m, 2H), 7.58 (d, J=7.5 Hz, 1 H), 7.69 (s, 1H), 7.79 (d, J=9 Hz, 1H), 7.83 (m, 1H), 7.90 (d, J=8 Hz, 1H), 7.95 (d, J=8 Hz, 1H), 8.33 (d, J=7.5 Hz, 1H), 10.79 (s, 1H). MS ($FAB^+$): m/e 831, 819 $(M+H)^+$, 803, 746, 719, 701, 505, 420, 375, 344, 300.

## Example 44d

### Boc-Trp-Leu-Asp-β-Nal-$NH_2$

The product of Example 44c (300 mg, 0.37 mmol) in DMF was stirred under an $H_2$ atmosphere in the presence of 75 mg of 5% Pd-C until disappearance of starting material was complete by TLC. The catalyst was removed by filtration, and the filtrate was concentrated. The residue was twice chromatographed over silica gel, eluting with 9:1 EtOAc/S2 (S2=8:1:1 MeOH/HOAc/$H_2O$), pure fractions were combined and concentrated to a small volume of HOAc, then $H_2O$ was added to precipitate 105 mg of the product, mp 205 °C (dec). $^1$H-NMR (DMSO-$d_6$). δ 0.80 (m, 6H), 1.23-1.50 (cm, 11H, includes 1.30, s, 9H), 1.57 (m, 1H), 2.50 (1H, obscured by solvent), 2.67 (dd, J=7.5 and 13.5 Hz, 1H), 2.90 (m, 1H), 3.11 (m, 2H), 3.20 (dd, J=4 and 11.5 Hz, 1H), 4.21 (m, 1H), 4.33 (m, 1H), 4.50 (m, 2H), 6.81 (d, J=2.5 Hz, 1H), 6.95 (t, J=6 Hz, 1H), 7.04 (t, J=6 Hz, 1H), 7.10 (s, 1H), 7.17 (s, 1H), 7.31 (m, 2H), 7.38 (d, J=7 Hz, 1H), 7.45 (m, 3H), 7.57 (d, J=7 Hz, 1H), 7.70 (s, 1H), 7.78 (d, J=7 Hz, 1H), 7.84 (m, 2H), 7.90 (m, 1H), 8.29 (m, 1H), 10.76 (s, 1H), 12.4 (br s, 1H). MS (DCI): m/e 729 $(M+H)^+$, 712, 685, 629, 611, 430. Anal. calc. for $C_{39}H_{48}N_6O_8$.0.3 HOAc: C, 63.61; H, 6.64; N, 11.23. Found: C, 63.95; H, 6.77; N, 10.90.

## Example 45

Boc-Trp-Leu-Asp-α-Nal-$NH_2$

## Example 45a

Boc-α-Nal-$NH_2$

In the same manner as that described in Example 44a the title compound was prepared from Boc-(β-1-naphthyl)alanine (682 mg, 2.17 mmol) in 98% yield. ¹H-NMR ($CDCl_3$): δ 1.40 (s, 9H), 3.52 (m, 2H), 4.52 (m, 1H), 5.20 (br s, 1H), 5.26 (br s, 1H), 5.46 (br s, 1H), 7.38 (m, 2H), 7.55 (m, 2H), 7.28 (d, J=8 Hz, 1H), 7.38 (d, J=8 Hz, 1H), 8.20 (d, J=8 Hz, 1H).MS (DCI): 315 $(M+H)^+$, 215.

## Example 45b

$CF_3COOH$.Asp(Bn)-α-Nal-$NH_2$

In the same manner as that described in Example 44b the product of example 45a (700 mg, 2.23 mmol) was deprotected and coupled to Boc-Asp(Bn)-OH to afford 950 mg of crude protected dipeptide, which was N-deprotected with 1:1 $CF_3CO_2H/CH_2Cl_2$ to provide the title salt. ¹H-NMR (DMSO-$d_6$): δ 2.83 (dd, J=9 and 18 Hz, 1H), 3.03 (dd, J=4 and 17 Hz, 1H), 3.27 (dd, J=10 and 15 Hz, 1H), 3.53 (dd, J=6 and 14 Hz), 4.09 (dd, J=4 and 9 Hz, 1H), 4.61 (m, 1H), 5.16 (d, J=2 Hz, 2H), 7.22 (s, 1H), 7,40 (m, 6H), 7.55 (m, 3H), 7.82 (d, J=7.5 Hz, 1H), 7.93 (d, J=7.5 Hz, 1H), 8.15 (br s, 3H), 8.29 (d, J=8 Hz, 1H), 8.82 (d, J=8 Hz, 1H). MS (DCI): m/e 420 $(M+H)^+$, 329, 312, 286, 260, 243, 217, 195, 178.

## Example 45c

Boc-Trp-Leu-Asp(Bn)-α-Nal-$NH_2$

In the same manner as that described in example 44c the product of Example 45b (1.1 mmol) was converted to the title compound. Upon treatment of the concentrated reaction mixture with $H_2O$ and sat. aq. $KHSO_4$, the product precipitated as a solid (750 mg) and was recrystallized to afford 630 mg (70%) of pure product. ¹H-NMR (DMSO-$d_6$): δ 0.83 (m, 6H), 1.29 (s, 9H), 1.40 (m, 2H), 1.62 (m, 1H), 2.61 (dd, J=8 and 16 Hz, 1H), 2.80 (dd, J=5 and 16 Hz, 1H), 2.90 (dd, J=4 and 15 Hz, 1H), 3.25 (m, 1H), 3.51 (m, 1H), 4.20 (m, 1H), 4.32 (m, 1H), 4.50 (m, 1H), 4.60 (m, 1H), 5.07 (s, 2H), 6.86 (d, J=8 Hz, 1H), 6.93 (t, J=7.5 Hz, 1H), 7.03 (t, J=7 Hz, 1H), 7.11 (br s, 1H), 7.20 (br s, 1H), 7.25-7.40 (cm, 9H), 7.48-7.61 (cm, 3H), 7.77 (d, J=8 Hz, 1H), 7.93 (m, 2H), 8.06 (d, J=8 Hz, 1H), 8.20 (d, J=8 Hz, 1H), 8.31 (d, J=8 Hz, 1H), 10.79 (s, 1H). MS ($FAB^+$): m/e 831, 819 $(M+H)^+$, 803, 746, 719, 701, 505, 420, 375, 344, 319, 300.

## Example 45d

Boc-Trp-Leu-Asp-α-Nal-$NH_2$

The product of Example 45c (100 mg, 0.12 mmol) was hydrogenolyzed as described in Example 44d. The crude product was purified by chromatography over silica gel, eluting with 6:1 EtOAc/S1 (S1 = 20:11:6 pyridine/HOAc/$H_2O$). Pure fractions were combined and concentrated to a small volume, then $H_2O$ was added to precipitate 63 mg (71%) of the product, mp 213 °C (decomp.) $^1$H-NMR (DMSO-$d_6$): δ 0.84 (m, 6H), 1.30 (m, 11H, includes 1.30, s, 9H), 1.60 (m, 1H), 2.45 (1H, obscured by solvent), 2.67 (dd, J=7 and 16.5 Hz, 1H): 2.90 (m, 1H), 3.08 (dd, J= 4 and 14 Hz, 1H), 3.25 (m, partially obscured by $H_2O$), 3.59 (m, 1H), 4.20 (m, 1H), 4.32 (m, 1H), 4.48 (m, 2H), 6.82 (d, J=8 Hz, 1H), 6.95 (t, J=7.5 Hz, 1H), 7.05 (t, J=7.5 Hz, 1H), 7.10 (s, 1H), 7.23 (m, 2H), 7.30-7.41 (m, 3H), 7.48-7.61 (m, 3H), 7.77 (d, J=8 Hz, 1H), 7.90 (d, J=8 Hz, 1H), 8.08 (d, J=8 Hz, 1H), 8.21 (d, J=8 Hz, 1H), 8.27 (d, J=7.5 Hz, 1H), 10.77 (s, 1H). MS (FAB+): 729 $(M+H)^+$, 713, 629, 611, 596. Anal. calc. for $C_{39}H_{48}N_6O_8$•0.3 HOAc: C, 63.61, H, 6.64, N, 11.23. Found: C, 63.90, H, 6.76, N, 11.18.

Example 46

Boc-Trp-Pro-Asp-Phe-$NH_2$

Example 46a

Boc-Pro-Asp(Bn)-Phe-$NH_2$

Boc-Pro-OH (189 mg, 0.88 mmol) was coupled to TFA•Asp(Bn)-Phe-$NH_2$ (425 mg, 0.88 mmol) using the DCC/HOBt method in THF in the usual manner to provide after work-up 469 mg of crude product. Recrystallization from EtOAc provided 326 mg (65%) of the product as a white solid. $^1$H-NMR (DMSO-$d_6$) (2 conformers, 1.5:1): δ 1.28 (s, 5.4 H), 1.38 (s, 3.6H), 1.71 (m, 3H), 2.0 (m, 1H), 2.58 (d, J=7.5 Hz, 0.4H), 2.63 (d, J=7.5 Hz, 0.6 H), 2.80 (m, 2H), 3.02 (m, 1H), 3.25 (1H, obscured by $H_2O$), 4.05 (m, 1H), 4.39 (m, 1H), 4.60 (m, 1H), 5.07 (s, 2H), 7.20 (m, 7H), 7.35 (m, 5H), 7.74 (d, J=8 Hz, 0.4 H), 7.86 (d, J=7.5 Hz, 0.6H), 8.14 (d, J=7.5 Hz, 0.6 H), 8.24 (d, J=7.5 Hz, 0.4H). MS (EI): m/e 566 (M+), 522, 465, 458, 402, 385, 358, 314.

Example 46b

Boc-Trp-Pro-Asp-Phe-$NH_2$

The product of Example 46a was N-deprotected with 4N HCl/dioxane in the usual manner. The resulting hydrochloride salt (177 mg, 0.35 mmol) in DMF at 0 °C was neutralized with N-methylmorpholine (38 µL, 0.35 mmol), then treated with a solution of the symmetrical anhydride formed from Boc-Trp-OH (322 mg, 1.06 mmol) and DCC (109 mg, 0.53 mmol) in $CH_2Cl_2$. After stirring at 0 °C for 68 h, the mixture was treated with several drops of 1-(2-aminoethyl)piperazine, then concentrated. The residue was partitioned between EtOAc and dil. aq. $KHSO_4$, and the separated aqueous layer was extracted twice with fresh portions of EtOAc, then the combined organic extracts were washed with $H_2O$, sat. aq. $NaHCO_3$, $H_2O$, and brine, then dried ($Na_2SO_4$), filtered and evaporated to 330 mg of crude product. Chromatography over silica gel, eluting with 83:17:2 EtOAc/hexane/HOAc, then 98:2 EtOAc/HOAc, afforded 281 mg of pure product which was hydrogenolyzed in MeOH over 100 mg of 20% $Pd(OH)_2$-C for 0.5 h. The mixture was filtered and concentrated, and the residue was chromatographed on silica gel, eluting with 85:15 EtOAc/S2 (S2=8:1:1 MeOH/HOAc/$H_2O$). Pure fractions were combined and concentrated to 240 mg, which was recrystallized from EtOH/$H_2O$ to provide 174 mg of the product as a white solid, mp 155.5-157 °C

(decomp.). $^1$H-NMR (DMSO-$d_6$) (2 conformers, 4:1) major conformer: δ 1.30 (s, 9H), 1.7-2.1 (cm, 4H), 2.48 (1H, obscured by solvent), 2.67 (dd, J=6 and 16 Hz, 1H), 2.84-3.16 (cm, 4H), 3.55 (m, 2H), 4.27-4.51 (cm, 4H), 6.92-7.10 (m, 3H), 7.15-7.28 (m, 7H), 7.35 (d, J=7.5 Hz, 1H), 7.52 (d, J=7.5 Hz, 1H), 7.81 (d, J=8 Hz, 1H), 8.18 (d, J=7.5 Hz, 1H), 10.83 (s, 1H). MS ($FAB^+$): m/e 663 $(M+H)^+$, 647, 563, 377. Anal. calc. for $C_{34}H_{42}N_6O_8 \cdot 0.5\ H_2O$: C, 60.78, H, 6.46, N, 12.51. Found: C, 60.90, H, 6.44, N, 12.46.

## Example 47

### Boc-Trp-Tpp-Asp-Phe-$NH_2$

## Example 47a

### Boc-Tpp-Asp(Bn)-Phe-$NH_2$

Boc-Tpp-OH (65 mg, 0.25 mmol) was coupled to TFA•Asp(Bn)-Phe-$NH_2$ (244 mg, 0.5 mmol) by the DCC/HOBt procedure in THF in the usual manner. On completion of the reaction, the mixture was treated with several drops of sat. aq. $KHSO_4$, filtered, and the filtrate was diluted with EtOAc and washed with sat. aq. $KHSO_4$, $H_2O$, sat. aq. $NaHCO_3$, $H_2O$, and brine, then dried ($Na_2SO_4$), filtered and evaporated to provide 180 mg of crude product. The product was purified on silica gel (chromatotron, 1 mm plate) eluting with 2% MeOH/$CHCl_3$ to provide 105 mg (72%) of pure product. $^1$H-NMR ($CDCl_3$): δ 0.93 (t, J=7 Hz, 3H), 1.24-1.6 (cm, 14H, includes 1.45, s, 9H), 2.0 (m, 1H), 2.19 (m, 1H), 2.75 (dd, J= 6 and 18 Hz, 1H), 2.94-3.21 (cm, 2H), 3.37 (m, 1H), 3.47 (dd, J= 4.5 and 15 Hz, 1H), 3.58 (m, 1H), 3.77 (d, J=7 Hz, 1H), 4.57 (m, 1H), 4.75 (m, 1H), 4.91 (d, J=12 Hz, 1H), 5.08 (d, J=12 Hz, 1H), 5.30 (br s, 1H), 6.70 (br s, 1H), 7.12-7.43 (cm, 10 H), 7.50 (d, J=7 Hz, 1H), 7.58 (d, J=7.5 Hz, 1H). MS (DEI/DIP): m/e 609 $(M+H)^+$, 507, 464, 445, 400, 356, 212.

## Example 47b

### Boc-Trp-Tpp-Asp(Bn)-Phe-$NH_2$

The product of Example 47a (101 mg, 0.17 mmol) was N-deprotected with 1:1 $CF_3CO_2H/CH_2Cl_2$ in the usual manner. The resultant salt in 3 mL of DMF at 0 °C was treated with a solution of the symmetrical anhydride prepared from Boc- Trp-OH (155 mg, 0.51 mmol) and DCC (53 mg, 0.26 mmol) in 5 mL of $CH_2Cl_2$ at 0 °C, followed by addition of NEt*i*-Pr2 (78 μL, 0.45 mmol). Solvent was removed under reduced pressure (air bath) and the solution was stirred for 1 h at 0 °C, and then allowed to warm to room temp. The solution was concentrated, and the residue was dissolved in EtOAc, then washed with sat. aq. $KHSO_4$, $H_2O$, sat. aq. $NaHCO_3$, $H_2O$, and brine, then dried ($Na_2SO_4$), filtered and evaporated. The crude product was chromatographed over silica gel, eluting with EtOAc/hexane (10:1, then 7:1), then EtOAc, to afford 112 mg (83%) of pure product. $^1$H-NMR ($CDCl_3$): δ 0.89 (t, J=4.5 Hz, 3H), 1.15-1.35 (cm, 5H), 1.41 (s, 9H, shoulder at 1.38), 1.83 (m, 1H), 1.98 (m, 1H), 2.64 (dd, J=5 and 3.5 Hz, 1H), 2.81 (dd, J= 3 and 10 Hz, 1H), 2.98 (m, 1H), 3.15 (m, 3H), 3.44 (dd, J=2 and 9 Hz, 1H), 3.56 (m, 1H), 3.90 (d, J=5 Hz, 1H), 4.60 (q, J=3H, 1H), 4.70 (q, J=4 Hz, 1H), 4.75 (m, 1H), 5.02 (d, J=7.5 Hz, 1H), 5.12 (2H, includes d, J=7.5 Hz, 1H), 5.36 (s, 1H), 6.61 (s, 1H), 6.96 (m, 2H), 7.13 (t, J=4 Hz, 1H), 7.20 (m, 4H), 7.25-7.35 (cm, 7H), 7.47 (d, J=5 Hz, 1H), 7.62 (d, J=5 Hz, 1H), 8.20 (s, 1H). MS (DCI): m/e 795 $(M+H)^+$, 695, 687, 587, 569.

Example 47c

Boc-Trp-Tpp-Asp-Phe-$NH_2$

The product of example 47b (105 mg, 0.13 mmol) in 1.5 mL of MeOH was stirred under a $H_2$ atmosphere in the presence of 5% Pd/C for 2.5 h, then the mixture was filtered through Celite and the filtrate was concentrated. The residue was chromatographed over silica gel, eluting with 9:1 EtOAc/S2 (S2 = 8:1:1 MeOH/HOAc/$H_2O$) to afford 63 mg of product, mp 145-147 °C. $[\alpha]^{25}_D$=-30.1° (c=0.5, DMF). $^1$H-NMR (DMSO-$d_6$): δ 0.85 (m, 3H), 1.05-1.40 (cm, 14 H, includes 1.44, s, 9H), 1.46 (m, 1H), 2.03 (m, 1H), 2.53 (1H, obscured by solvent), 2.67 (m, 1H), 2.93 (m, 2H), 3.0-3.18 (cm, 2H), 3.73 (m, 1H), 3.90 (m, 1H), 4.25-4.50 (cm, 4H), 6.97 (m, 1H), 7.11 (m, 2H), 7.22 (m, 8H), 7.44 (d, J=7.5 Hz, 1H), 7.50 (d, J=7.5 Hz, 1H), 7.90 (br d, J=7.5 Hz, 1H), 8.30 (d, J=7.5 Hz, 1H), 10.87 (br s, 1H). MS (FAB$^+$) : m/e 727 $(M+Na)^+$, 705 $(M+H)^+$, 689, 605, 587, 419. Anal. calc. for $C_{37}H_{48}N_6O_8$•0.5 $H_2O$: C, 62.24; H, 6.92; N, 11.77. Found: C, 62.37; H, 6.83; N, 11.52.

Example 48

Ctp-Tpp-Asp-Phe-$NH_2$

Example 48a

Ctp-Tpp-Asp(Bn)-Phe-$NH_2$

The product of example 47a (68 mg, 0.11 mmol) was N-deprotected as described in Example 47a. The resultant salt and Ctp-OH (0.12 mmol) were dissolved in 1:1 $CH_2Cl_2$/DMF and treated at 0 °C with diisopropylethylamine (57 μL, 0.33 mmol) and BOP-Cl (31 mg, 0.12 mmol). The solution was kept at 4 °C for 69 h, more Bop-Cl was added, and the reaction was allowed to continue at the same temperature for an additional 48 h, then the mixture was concentrated. The residue was dissolved in EtOAc, washed with sat. aq. $KHSO_4$, $H_2O$, sat. aq. $NaHCO_3$, $H_2O$, and brine, then dried ($Na_2SO_4$), filtered and evaporated to afford 66 mg of crude product. This material was purified by chromatography over silica gel, eluting with $CHCl_3$/MeOH (98:2, then 90:10), to afford 38 mg (47%) of pure product. HRMS Calc.for $(M+H)^+$ = $C_{41}H_{47}N_6O_7$: m/e 735.3506. Found: 735.3507.

Example 48b

Ctp-Tpp-Asp-Phe-$NH_2$

The product of Example 48a (36 mg, 0.049 mmol) in MeOH was stirred under an $H_2$ atmosphere in the presence of 5% Pd/$BaSO_4$ for 0.5 h, after which TLC showed complete consumption of starting material. The mixture was filtered through Celite, the filtrate was concentrated, and the residue was chromatographed over silica gel, eluting with 3:1 EtOAc/S1 (S1 = 20:11:6 pyridine/$H_2O$/HOAc) to afford 29 mg of pure product, mp 192 °C (dec). $^1$H-NMR (DMSO-$d_6$): δ 0.88 (m, 3H), 1.25 (m, 4H), 1.55 (br, 1H), 2.05 (m, 2H), 2.22 (m, 2H), 2.80 (dd, J=10 and 14 Hz, 1H), 3.12 (dd, J=4 and 14 Hz, 1H), 3.20-3.72 (cm, 5H), 3.87 (m,

1H), 3.93 (d; J=4 Hz, 1H), 4.08 (m, 1H), 4.22 (m, 1H), 4.58 (m, 1H), 6.77 (m, 1H), 6.95 (m, 3H), 7.10-7.25 (cm, 7H), 11.0 (br s, 1H). HRMS: Calc. for $(M+H)^+$ = $C_{34}H_{41}N_6O_7$: 645.3037. Found: 645.3036

Example 49

Boc-α-Nal-Leu-Asp-Phe-$NH_2$

Boc-α-Nal-OH (200 mg, 0.63 mmol) was coupled to TFA•Leu-Asp(Bn)-Phe-$NH_2$ (376 mg, 0.63 mmol) by the mixed anhydride method in the normal fashion using isobutyl chloroformate as the activating agent. Upon completion of the reaction, the mixture was concentrated, and the crude product (390 mg) was caused to precipitate by addition of dil. aq. $KHSO_4$. A solution of 150 mg of this product in DMF was stirred under an $H_2$ atmosphere in the presence of 5% Pd-C until consumption of starting material was complete by TLC. The mixture was filtered, the filtrate was concentrated, and the residue was chromatographed over silica gel, eluting with 6:1 EtOAc/S1. Pure fractions were combined and concentrated to a small volume, then water was added and the precipitated product (120 mg) was collected by filtration and dried in vacuo. mp 231 °C (dec). $^1$H-NMR (DMSO-$d_6$) δ 0.87 (m, 6H), 1.24 (s, 9H), 1.44 (m, 2H), 1.65 (m, 1H), 2.48 (1H, obscured by solvent), 2.70 (dd, J=6 and 20 Hz, 1H), 2.85 (dd, J=9 and 14 Hz, 1H), 3.05 (dd, J=4.5 and 14 Hz, 1H), 3.15 (dd, J=10 and 14 Hz, 1H), 3.53 (dd, J=4 and 14 Hz, 1H), 4.35 (m, 3H), 4.52 (m, 1H), 7.05 (d, J=9 Hz, 1H), 7.15-7.32 (cm, 7H), 7.40 (m, 2H), 7.53 (m, 2H), 7.77 (m, 1H), 7.90 (m, 3H), 8.15 (d, J=8 Hz, 1H), 8.30 (d, J=7.5 Hz, 1H). MS (FAB+): m/e 690 $(M+H)^+$, 674, 590, 307. Calc. for $C_{37}H_{47}N_5O_8$•0.5 $H_2O$: C, 63.57, H, 6.93, N, 10.03. Found: C, 63.64; H, 6.70; N, 9.69.

Example 50

Boc-β-Nal-Leu-Asp-Phe-$NH_2$

In a procedure analogous to that described in Example 49, Boc-β-Nal-OH (200 mg, 0.63 mmol) was coupled to TFA•Leu-Asp(Bn)-Phe-$NH_2$ to afford 430 mg of crude protected tetrapeptide as a solid. Hydrogenolysis of 150 mg of this product followed by work-up and purification as in Example 49 afforded 73 mg of the title compound, m.p 204 °C (dec). $^1$H-NMR (DMSO-$d_6$). δ 0.85 (t, J=7.5 Hz, 6H), 1.25 (s, 9H), 1.40 (m, 2H), 1.63 (m, 1H), 2.43 (dd, J=7 and 17 Hz, 1H), 2.64 (dd, J=7 and 17 Hz, 1H), 2.79-2.91 (cm, 2H), 3.05 (dd, J=4.5 and 14 Hz, 1H), 3.15 (dd, J=3 and 14 Hz, 1H), 4.25-4.40 (cm, 3H), 4.50 (m, 1H), 6.97 (d, J=8 Hz), 7.10-7.28 (cm, 6H), 7.35 (s, 1H), 7.45 (m, 3H), 7.73 (s, 1H), 7.76-7.88 (cm, 3H), 7.96 (m, 2H), 8.28 (d, J=7.5 Hz, 1H). MS ($FAB^+$): m/e 690 $(M+H)^+$, 674, 590, 575, 307. Anal. calc. for $C_{37}H_{47}N_5O_8.H_2O$: C, 62.77; H, 6.98; N, 9.90. Found: C, 62.40; H, 6.55; N, 9.58.

Example 51

Boc-Trp-Leu-Asp-3(S)-benzyl-2-oxo-4-piperazine

Example 51a

Z-Phe-allylamide

Under a nitrogen atmosphere, Z-Phe-OH (1.00 g, 3.35 mmol) was dissolved in 10 mL of anhydrous methylene chloride containing 1-hydroxybenzotriazole monohydrate (0.57 g, 3.69 mmol) and 1-(3-dimethylaminopropyl)ethylcarbodiimide hydrochoride (0.71 g, 3.69 mmol) and stirred at ice bath temperature for 1.5 h. Allylamine (0.19 g, 3.35 mmol) was added and the solution was stirred at 0 °C for 5 h and then allowed to warm to room temperature overnight. The solvent was evaporated, and the residue was dissolved in ethyl acetate and washed with sat. aq. $KHSO_4$, $H_2O$, sat. aq. $NaHCO_3$, $H_2O$, and brine, prior to drying and evaporation. Recrystallization from ethyl acetate/hexane afforded 0 56 g (50%) of the white solid product: $^1H$ NMR ($CDCl_3$): δ 3.03 (dd, J = 15 and 7.5 Hz, 1H), 3.14 (dd, J = 15 and 7.5Hz, 1H), 3.76-3.82 (m, 2H), 4.33-4.42 (m, 1H), 4.96-5.08 (m, 2H), 5.10 (s, 2H), 5.33 (br s, 1H), 5.59-5.74 (m, 2H), 7.15-7.40 (m, 10H), MS (DCI) m/e 356 $(M+NH_4)^+$, 339$(M+H)^+$.

Example 51b

3(S)-Benzyl-4-benzyloxycarbonyl-5-hydroxy-2-oxo-4-piperazine

To a stirred solution of the product of example 51a (376 mg, 1.1 mmol) in 27 mL of $CH_3CN/H_2O$ (2:1) at room temperature was added 4-methylmorpholine N-oxide (180 mg, 1.3 mmol) and osmium tetroxide (2.5% in t-butanol, 2.9 mg, 0.01 mmol). After 5 min, sodium periodate (949 mg, 4.4 mmol) was added and the mixture was stirred at room temperature for 3.5 h. The acetonitrile was removed in vacuo and the resulting aqueous solution was extracted with ethyl acetate. The separated organic phase was washed with sat. aq. $KHSO_4$, $H_2O$, sat. aq. $NaHCO_3$, $H_2O$, and brine prior to drying. After solvent evaporation, the crude material was purified by flash chromotography over silica gel, eluting with 2% methanol/chloroform to afford 212 mg (56%) of the lactol: $^1H$ NMR ($CDCl_3$): δ 3.02-3.17 (m, 2H), 4.06-4.19 (m, 2H), 4.43-4.55 (m, 1H), 5.09 (s, 2H), 5.30 (br s, 1H), 6.47 (br s, 1H), 7.16-7.39 (m, 10H), MS (DCI) : m/e 358 $(M+NH_4)^+$, 341$(M+H)^+$, 316, 299.

Example 51c

3(S)-βenzyl-2-oxopiperazine

The product of example 51b (183 mg, 0.54 mmol) in MeOH was stirred under a $H_2$ atmosphere (4 atm) in the presence of 10% palladium on activated carbon (183 mg) at room temperature overnight. Removal of the catalyst by filtration followed by solvent evaporation gave 77 mg (75%) of crude product. Purification by flash chromotography on silica gel eluting with 3% $MeOH/CHI_3$ afforded 26 mg (25%) of the piperazine: $^1H$ NMR (DMSO-$d_6$) δ 1.24 (br s, 1H), 2.61-3.21 (m, 6H), 4.03-4.07 (m, 1H), 7.13-7.32 (m, 5H), 7.64 (br s, 1H). MS (DCI): m/e 208$(M+NH_4)^+$, 191 $(M+H)^+$

Example 51d

Boc-Asp(Bn)-3(S)-benzyl-2-oxo-4-piperazine

A solution of Boc-Asp(Bn)-OH (476 mg, 1.47 mmol) in 4 mL of anhydrous $CH_2Cl_2$ under a nitrogen atmosphere was cooled to ice bath temperature and 1-(3-dimethylaminopropyl)-3-ethylcarbodiimide hydrochloride (142 mg, 0.74 mmol) was added. After 40 min the solution was added directly to a pre-cooled solution of the product of Example 51c (128 mg, 0.67 mmol) in 2 mL of anhydrous dimethylformamide at

ice bath temperature. The reaction mixture was stirred at 0 °C for 3 h and at room temperature for 48 h. The solvents were removed in vacuo, then the residue was dissolved in ethyl acetate and washed with sat. aq. $KHSO_4$, $H_2O$, sat. aq. $NaHCO_3$, $H_2O$, and brine. After drying and solvent evaporation, the crude material was purified by flash chromotography on silica gel eluted with 43% acetone/hexane affording 213 mg (64%) of product: $^1$H NMR ($CDCl_3$) 3.02-3.17 (m, 2H), 4.06-4.19 (m, 2H), 4.43- 4.55 (m, 1H), 5.09 (s, 2H), 5.30(br s, 1H), 6.47 (br s, 1H), 7.16-7.39 (m,10H). MS (DCI): m/e 358 $(M+NH_4)^+$, 341$(M+H)^+$, 316, 299.

Example 51e

Boc-Leu-Asp(Bn)-3(S)-benzy-2-oxo-4-piperazine

The product of example 51d (182 mg, 0.37 mmol) was treated with a solution of 4.5 M HCl/dioxane (3 mL) for 1 h at room temperature after which the solvent was removed in vacuo to give a foamy residue. Diethyl ether was repeatedly added to the residue and evaporated, leaving the deprotected dipeptide as a tan solid which was then employed in the subsequent coupling step without purification. A solution of Boc-Leu-OH monohydrate (203 mg,0.81 mmol) in 3 mL of anhydrous 1:10 DMF/$CH_2Cl_2$ under a nitrogen atmosphere was cooled in an ice bath and EDCI was added. After 40 min the solution was added directly to a pre-cooled solution of the above HCl salt (160 mg, 0.37 mmol) in 1 mL of anhydrous DMF at ice bath temperature. Triethylamine (0.052 mL, 0.37 mmol) was then added and the reaction was stirred for 3 h at 0 °C and allowed to warm to room temperature overnight. The solvents were removed in vacuo and the residue was dissolved in ethyl acetate and washed with sat. aq. $KHSO_4$, $H_2O$, sat. ag. $NaHCO_3$, $H_2O$, and brine. After drying and solvent evaporation the crude material was purified by flash chromotography on silica gel eluting with 3% MeOH/$CHCl_3$ to afford 195 mg (86%) of the protected tripeptide. $^1$H NMR ($CDCl_3$): δ 0.85-1.00 (m, 6H), 1.46 (s, 9H), 1.57-1.60 (m, 2H), 2.55- 2.67 (m, 2H), 2.89-3.00 (m, 2H), 3.09-3.50 (m, 4H), 3.62-3.90 (m, 2H), 4.11 (br s, 1H),4.99-5.26 (m, 3H), 5.86-6.00 (m, 1H), 6.85 (br s, 1H), 7.12-7.40 (m, 10H). MS (DCI): m/e 626 $(M+NH_4)^+$, 609 $(M+H)^+$, 509, 419, 381, 231, 131.

Example 51f

Boc-Trp-Leu-Asp(Bn)-3(S)-benzyl-2-oxo-4-piperazine

The product of example 51e (173 mg, 0.28 mmol) was deprotected with 4N HCl/dioxane in the usual manner. To a stirred solution of the resulting salt (152 mg, 0.28 mmol) in 1.8 mL of anhydrous DMF under a nitrogen atmosphere at room temperature was added N,N-diisopropylethylamine (0.049 mL, 0.28 mmol) and Boc-Trp-N-hydroxysuccinimide ester (124 mg, 0.31 mmol). The mixture was stirred for 48 h, then the solvent was evaporated and the residue was dissolved in ethyl acetate and washed with saturated potassium hydrogen sulfate solution, water, saturated sodium bicarbonate solution, and brine. After drying and solvent evaporation, the crude material was purified by flash chromotography on silica gel eluting with acetic acid/ethyl acetate/hexane (3:25:72, then 3:50:47) to afford 188 mg (84%) of the tetrapeptide: $^1$H NMR ($CDCl_3$) δ 0.74-0.89 (m, 6H), 1.45 (s, 9H), 1.57-1.78 (m, 3H), 2.66-3.53 (m, 10H), 3.88-3.99 (m, 1H), 4.18-4.60 (m, 3H), 4.94-5.28 (m, 3H), 5.84-5.91 (m, 1H), 6.02-6.18 (m, 1H), 6.42-6.49 (m, 1H), 7.07- 7.43 (m, 14H), 7.65-7.72 (m, 1H), 8.67-8.80 (m, 1H). MS ($FAB^+$): m/e, 795 $(M+H)^+$, 779, 695, 678, 605, 505, 489, 396.

Example 51g

Boc-Trp-Leu-Asp-3(S)-benzyl-2-oxo-4-piperazine

To a stirred suspension of 10% Pd-C (32 mg) at room temperature in 3 mL of 10% formic acid/methanol under nitrogen atmosphere was added the product of Example 51f (76 mg, 0.096 mmol). After 24 h, the mixture was filtered using methanol to rinse. The crude material was subjected to flash chromotography (elution with 9:1 EtOAc/S1 followed by RP-HPLC purification (flow rate 14 mL/min, linear gradient from 20 to 55% $CH_3CN/H_2O$ over 40 min). The pure fractions were combined and lyopholized twice, yielding 13 mg (19%) of pure product isolated as the trihydrate. $^1H$ NMR (DMSO-$d_6$): δ 0.78-0.95 (m, 6H), 1.12-1.72 (m, 3H), 2.19-3.30 (m, 10H), 3.79-3.90 (m, 1H), 4.12-4.52 (m, 3H), 4.69-4.76 (m, 1H), 4.86-5.05 (m, 1H), 6.72 (br s, 1H), 6.92-7.34 (m, 8H), 7.53-7.62 (m, 1H), 7.88-7.98 (m, 1H), 8.08 (br s, 1H), 8.47-8.62 (m, 1H), 10.82-10.97 (m, 1H). MS(FAB$^+$): m/e 705 $(M+1)^+$, 605, 185, 130, 93. Anal. calcd. for $C_{37}H_{48}N_6O_8 \cdot 3\ H_2O$: C,58.52; H,7.17; N,11.07. Found: C, 58.49; H, 6.52; N, 10.90; $[\alpha]^D 24.5 = +8.57\ ^\circ C$ (c=0.105, MeOH).

## Example 52

## Ctp-Tpp-Asp-(NMe)Phe-$NH_2$

### Example 52a

### Diethyl 1-Acetyl-5-hydroxy-3-n-propylpyrrolidine-2,2-dicarboxylate

Sodium (3.48 g, 0.15 mol) was dissolved in a stirred solution of diethyl acetamidomalonate (201.6 g, 0.93 mol) in anhydrous ethanol (1200 mL) at room temperature under nitrogen. The reaction mixture was cooled to 0 °C and trans-2-hexenal (100.0 g, 1.02 mol) was then added dropwise. The resulting mixture was allowed to warm to room temperature. After stirring for 3 h at 23 °C, the reaction was quenched with 24 mL of acetic acid. The solution was concentrated in vacuo and the resulting residue was taken up in EtOAc and washed with saturated aqueous $NaHCO_3$ (2x) and brine. Dried over $MgSO_4$, concentration and recrystallization of the residue from EtOAc/Hexane gave 271.4 g (93%) of the product as fine needles. mp 105-106 °C. TLC, $R_f$= 0.43 (9:1 $CHCl_3$/MeOH). MS(Cl) m/e 316 $(M+1)^+$, 298 $(MH-H_2O)^+$. $^1H$ NMR ($CD_3OD$, 300 MHz) δ 0.94 (t, J=7.2 Hz, 3H), 1.47-1.11 (m, 9H), 1.90-1.78 (m, 1H), 1.94 (dd, J=12.8, 5.2 Hz, 1H), 2.08 (dd, J=12.8, 6.4 Hz, 1H), 2.21 (s, 3H), 2.83 (m, 1H), 4.10-4.26 (m, 4H), 5.64 (d, J=5.2 Hz, 1H). IR ($CDCl_3$) 3460, 1750, 1665 $cm^{-1}$. Anal. calcd for $C_{15}H_{25}NO_6$: C, 57.13; H, 7.99; N, 4.44. Found: C, 57.28; H, 8.08; N, 4.42.

### Example 52b

### Diethyl 1-Acetyl-3-n-propylpyrrolidine-2,2-dicarboxylate

To a solution of diethyl 1-acetyl-5-hydroxy-3-propylpyrrolidine-2,2-dicarboxylate (271.0 g, 0.86 mol) and triethylsilane (206 mL, 1.29 mol) in $CH_2Cl_2$ (3 L) was added trifluoroacetic acid (663 mL, 8.6 mol) dropwise with stirring while controlling the internal temperature at 25-30° C by means of an ice bath. After stirring for 3 h at 23 °C, the solution was concentrated in vacuo and the residue was diluted with $CH_2Cl_2$ and washed with saturated aqueous $NaHCO_3$ solution until all the TFA are neutralized. The organic phase was dried ($Na_2SO_4$) and concentrated to give 350 g of oil. The oily product containing silicon impurities was used directly in the subsequent step. An analytically pure sample was obtained as an oil after flash chromatography (1:2 -> 1.1 EtOAc/hexane). TLC, $R_f$ = 0.55 (9:1 $CHCl_3$/MeOH). MS(CI) m/e 300 $(M+H)^+$. IR ($CDCl_3$) 1745, 1655 $cm^{-1}$. $^1H$ NMR ($CDCl_3$, 300 MHz) $\delta$ 0.89-93 (t, J=7.0 Hz, 3H), 1.15-1.42 (m, 9H), 1.70-1.85 (m, 2H), 2.01-2.16 (m, 4H), 2.40-2.51 (m, 1H), 3.57 (ddd, J=10.7, 9.6, 6.2 Hz, 1H), 3.76 ('dt', J=9.6, 1.1 Hz, 1H), 4.17-4.28 (m, 4H). Anal. calcd. for $C_{15}H_{25}NO_5$: C, 60.18; H, 8.42; N, 4.68. Found: C, 59.90; H, 8.34; N, 4.65.

## Example 52c

## *Trans*- and *cis*-3-n-propylproline hydrochlorides

The crude ester of example 52b (350 g) was suspended in 6 N HCl (2 L) and acetic acid (500 mL) and heated at reflux for 17 h. The reaction mixture was extracted with EtOAc (2x) and the aqueous phase was concentrated on a rotary evaporator. The residue was then triturated with ether to crystallize the product The solid was collected by filtration, washed with ether and dried in a vacuum oven to give 152.3 g of the hydrochloride salt. An analytically pure sample was obtained by recrystallization from acetone/ether. mp 131-133 °C. TLC, $R_f$= 0.26 (10:4:1 $CHCl_3$/MeOH/$NH_4$OH). IR (KBr) 3420, 1735 $cm^{-1}$. MS(CI) m/e 158 (free base MH+). $^1H$ NMR ($D_2O$, 300 MHz) major isomer: $\delta$ 0.91 (t, J=7.2 Hz, 3H), 1.18-1.50 (m, 3H), 1.68-1.90 (m, 2H), 2.16-2.32 (m, 1H), 2.43-2.50 (m, 1H), 3.29-3.58 (m, 2H), 3.93 (d, J=7.4 Hz, 1H); Minor isomer: $\delta$ 0.89 (t, J=6.8 Hz, 3H), 1.18-1.50 (m, 3H), 1.68-1.90 (m, 2H), 2.16- 2.32 (m, 1H), 2.60-2.66 (m, 1H), 3.29-3.58 (m, 2H), 4.32 (d, J=8.1 Hz, 1H). Anal. calcd. for $C_8H_{16}NO_2Cl$: C, 49.61; H, 8.33; N, 7.23. Found: C, 49.35; H, 8.17; N, 7.18.

## Example 52d

## Methyl N-tert-butoxycarbonyl-3-n-propylproline

The hydrochloride salt of example 52c (152.3 g) was dissolved in MeOH (1.5 L) and the solution was charged with HCl gas until it was saturated. After stirring overnight, the reaction mixture was concentrated to give an oil. This was taken up in 1 N HCl and extracted with EtOAc (2x). The aqueous layer was carefully basified with $K_2CO_3$ and extracted with $CHCl_3$ exhaustively. The combined organic phases were dried ($MgSO_4$), filtered and concentrated to give 122 g of oil. TLC, $R_f$= 0.70 (90:10:0.1 $CHCl_3$/MeOH/$NH_4$OH). MS (CI) m/e 272 $(M+H)^+$. Partial $^1H$ NMR ($CDCl_3$, 300 MHz) Major isomer: $\delta$ 3.36 (d, J=6.3 Hz, 1H), 3.74 (s, 3H). Minor isomer: $\delta$ 3.72 (s, 3H), 3.83 (d, J=8.1 Hz, 1H). The oil (122 g) was taken up in MeOH (1 L) and then $NaHCO_3$ (180 g) and di-t-butyldicarbonate (171.4 g, 0.79 mol) were added slowly. After stirring overnight at 23 °C, the mixture was filtered and the filtrate concentrated. The residue was triturated with EtOAc, filtered again and concentrated to give 186.1 g of oil as a mixture of cis and trans isomers. TLC, $R_f$= 0.31 (1:6 EtOAc/hexane). IR ($CDCl_3$) 1700, 1745 $cm^{-1}$. MS(CI) m/e 272 $(M+H)^+$. Partial $^1H$ NMR ($CDCl_3$, 300 MHz) Cis isomer (2 conformers): $\delta$ 0.91 (t, J=7.0 Hz, 3H), 3.71 (s, 1.8H), 3.72 (s, 1.2H), 4.23 (d, J=8.5 Hz, 0.6H), 4.32 (d, J=8.1 Hz, 0.4H). Trans isomer (2 conformers): $\delta$ 0.92 (t, J=7.0 Hz, 3H), 1.06-1.78 (m, 14H), 1.92-2.03 (m, 1H), 2.13-2.25 (m, 1H), 3.42-3.50 (m, 1H), 3.56-3.68 (m, 1H), 3.73 (s, 1.8H), 3.74 (s, 1.2 H), 3.81 (d, J=6.2 Hz, 0.6H), 3.94 (d, J=5.5 Hz, 0.4H). Anal. calcd for $C_{14}H_{25}NO_4$: C, 61.97; H, 9.29; N, 5.16. Found: C, 61.93; H, 9.30; N, 5.11.

## Example 52e

## N-t-Boc-*trans*-3-n-propylproline and Methyl N-t-Boc-*cis*-3-n-propyloroline

To a solution of the esters of example 52d (186.0 g, 0.685 mol), in MeOH (685 mL) was added 1 N NaOH (685 mL) at 23 °C. After stirring for 20 h, the solution was concentrated to remove MeOH and then extracted with EtOAc (3x). The extracts were dried ($MgSO_4$), filtered and concentrated to give 71.4 g (38%) of cis ester. TLC, $R_f$= 0.54 (1:4 EtOAc/hexane). IR ($CDCl_3$) 1745, 1700 $cm^{-1}$. High resolution MS(CI) m/e 272.1869 ($(M+H)^+$, for $C_{14}H_{26}NO_4$, calcd 272.1862). $^1H$ NMR ($CDCl_3$, 300 MHz) Cis isomer (2 conformers). δ 0.91 (t, J=7.0 Hz, 3H), 1.07-1.20 (m, 1H), 1.23-1.45 (m, 12H), 1.64-1.78 (m, 1H), 1.90-2.12 (m, 1H), 2.25-2.41 (m, 1H), 3.25-3.34 (m, 1H), 3.56-3.68 (m, 1H), 3.71 (s, 1.8H), 3.72 (s, 1.2H), 4.23 (d, J=8.5 Hz, 0.6H), 4.32 (d, J=8.1 Hz, 0.4H). The aqueous phase was acidified with solid citric acid and extracted twice with EtOAc. The combined extract was washed with $H_2O$ and brine, dried ($MgSO_4$), filtered and concentrated to 94.2 g (53%) of trans acid. TLC, $R_f$= 0.45 (9:1 $CHCl_3$/MeOH). IR ($CDCl_3$) 3050, 1720, 1690 $cm^{-1}$. MS(CI) m/e 258 $(M+H)^+$. $^1H$ NMR ($CDCl_3$, 300 MHz) 2 conformers: δ 0.93 (m, 3H), 1.28-1.69 (m, 14H), 1.97-2.12 (m, 1H), 2.30 (m, 0.4H), 2.50 (m, 0.6H), 3.34-3.64 (m, 2H), 3.84 (br d, J=6.2 Hz, 0.4H), 3.98 (br d, J=4.4 Hz, 0.6H). Anal. calcd. for $C_{13}H_{23}NO_4$: C, 60.68; H, 9.01; N, 5.44. Found: C, 60.66; H, 8.91; N, 5.44.

## Example 52f

## N-tert-Butoxycarbonyl-*trans*-3-n-propyl-proline, (s)-methylbenzylamides

To a solution of trans acid of example 52e (94.2 g, 0.37 mol), (S)-(-)methylbenzylamine (44.8 g, 0.37 mol) and 1-hydroxybenzotriazole hydrate (54.4 g, 0.40 mol) in $CH_2Cl_2$ (1 L) was added 1-ethyl-3-(3-dimethylaminopropyl)carbodiimide.HCl (76.9 g, 0.40 mol) under nitrogen at 0 °C. After stirring overnight at 23 °C, the reaction mixture was diluted with EtOAc, washed with 10% aqueous citric acid, saturated aqueous $NaHCO_3$ and brine, then dried over $MgSO_4$, filtered and concentrated. The residue was dissolved in minimum amount of ether and the S-isomer (with respect to proline), preferentially crystallized upon standing (34.0 g, 90% S-isomer). The mother liquor was concentrated and the resulting residue was chromatographed on silica gel using 35% EtOAc/Hexane as elutant to give 46.5 g of the R-isomer (35%, contains <5% S-isomer) 6.9 g of mixture and 16.3 g of S-isomer (90% S-isomer). The 34.0 g of 90% pure S-isomer, 6.9 g of mixture and 16.3 g of 90% pure S-isomer were combined and recrystallized three time from ether/hexane to give a total of 40.2 g (30%) of S-isomer (99%+ pure). Alternatively, the mixture could also be separated by HPLC with about 80% recovery S-isomer: mp 112-114 °C ($Et_2O$/hexane). TLC, $R_f$=0.39 (EtOAc:hexane=1:2). $[\alpha]^{23}_D$= -56.30 (c=0.75, MeOH). $^1H$ NMR (300 MHz, DMSO-$d_6$, 138 °C) δ 0.88 (br t, J=7.0 Hz, 3H), 1.40 (s, 9H), 1.43 (d, J=7.1Hz, 3H), 1.25-1.55 (m, 5H), 1.97 (m, 1H), 2.14(m, 1H), 3.30-3.55 (m, 2H), 3.77 (d, J=5.1 Hz, 1H), 4.99 (q, J=7.1Hz, 1H), 7.65-7.15 (m, 5H). MS (CI) m/e 361 ($MH^+$, base), 305, 261, 112. Anal. Calcd. For C21H32N2O3. C, 69.97; H, 8.95; N, 7.77. Found: C, 69.99; H, 9.05; N, 7.73. R-isomer: mp 104-105 °C ($Et_2O$/Hexane). TLC, $R_f$=0.45 (EtOAc:Hexane=1:2). $[\alpha]_D$=-65.8° (c=0.76, MeOH). $^1H$ NMR (300 MHz, DMSO-$d_6$, 138 °C) δ 0.91 (br t, J=7.0 Hz, 3H), 1.36 (s, 9H), 1.28-1.42 (m, 3H), 1.43 (d, J=7.1 Hz, 3H), 1.45-1.58 (m, 2H), 2.00 (m, 1H), 2.18 (m, 1H), 3.33-3.51 (m, 2H), 3.79 (d, J=4.9 Hz, 1H), 4.98 (q, J=7.1 Hz, 1H), 7.60-7.17 (m, 5H). MS (CI) m/e 361 ($MH^+$, base), 305, 261, 112. Anal. Calcd for $C_{21}H_{32}N_2O_3$: C, 69.97; H, 8.95; N, 7.77. Found: C, 70.27; H, 9.25; N, 7.73.

## Example 52g

## N-t-Boc-*trans*-3-n-propyl-(L)-proline

A solution of the S-isomer of example 52f (40.2 g, 0.11 mol) in 8 N HCl (870 mL) and glacial acetic acid (220 mL) was heated at reflux overnight. The solution was concentrated on a rotary evaporator and the residue taken up into $H_2O$ and extracted with ether. The aqueous phase was concentrated and azeotroped 3x with toluene to give 43.0 g of trans-3-n-propyl-(L)-proline and (s)-methylbenzylamine hydrochlorides. The salts were taken up in dioxane/$H_2O$ (1:1, 400 mL) and then treated carefully with N,N-diisopropylethylamine (35.5 g, 0.275 mol) and di-t-butyl dicarbonate (60.0 g, 0.275 mol) sequentially at 0 °C. After stirring

overnight at 23 °C, the mixture was diluted with EtOAc and the two layers were separated. The organic layer was extracted with 0.5 N NaOH (2x). The combined aqueous phase was extracted with EtOAc once and again back-extracted EtOAc was washed with 0.5 N NaOH. The combined basic $H_2O$ phase was cooled to 0 °C-5 °C and acidified to pH 1.0 with cold 4 N HCl and extracted immediately with EtOAc (2x). The combined EtOAc extract was washed with brine, dried ($MgSO_4$) and concentrated. The residue was dried in vacuous desicator over $P_2O_5$ to give 26.1 g (92%) of desired product as a white solid. mp 88-89 °C (Hexane). TLC, $R_f$=0.15 ($CHCl_3$:MeOH=9:1). $[\alpha]^{24}_D$= -38.3° (c=1.0, $CHCl_3$); $[\alpha]^{25}_D$=-42.5° (c 0.095, $CHCl_3$). $^1$H NMR (360 MHz, DMSO-$d_6$, 100 °C) $\delta$ 0.90 (t, J=7.0 Hz, 3H), 1.20-1.55 (m, 15H), 1.98 (m, 1H), 2.20 (m, 1H), 3.30 (m, 1H), 3.40 (m, 1H), 3.67 (d, J=4.0 Hz, 1H). MS (Cl) m/e 258 $(MH)^+$, 219 (base), 202, 158. Anal. Calcd. for $C_{13}H_{23}NO_4$: C, 60.68; H, 9.01; N, 5.44. Found: C, 60.85; H, 8.97; N, 5.44. The (+)-isomer was obtained similarly: mp 90-92 °C (Hexane). $[\alpha]^{24}_D$= +43.2° (c 1.0, $CHCl_3$).

## Example 52h

### Ctp-Tpp-Asp-(NMe)Phe-$NH_2$

To a solution of the hydrochloride salt of Tpp-Asp(OBn)-N-Me-Ph-$NH_2$ (18.2 mg, 0.032 mmol), prepared as in examples 25α-25b Ctp acid (8 7 mg, 0.035 mmol) and BOP-Cl (10 mg, 0.039 mmol) in dimethylformamide (0.5 mL) and methylene chloride (1.5 mL) was added diisopropylethylamine (10.1 mg, 0.078 mmol) at 0 °C under nitrogen. The mixture was stirred overnight at 0 °C and then poured into 3% citric acid and extracted with EtOAc (2x). The combined organics was washed with saturated $NaHCO_3$ aqueous solution and brine. After drying with $Na_2SO_4$, the solvent was removed in vacuo and the residue was subjected to flash chromatography (2.5% to 10% MeOH/$CHCl_3$) to give 14 mg (57%) of Ctp-Tpp-Asp(OBn)(NMe)Phe-$NH_2$ as a solid. This material was then taken up in MeOH (2 mL) and hydrogenated under one atmosphere of hydrogen in the presence of 5% Pd/$BaSO_4$ (20 mg) for 1.5 h at room temperature. After filtration of the reaction mixture through Celite and concentration of the filtrate, the resulting residue was chromatographed on a preparative reverse phase HPLC using 0.1% aqueous TFA and acetonitrile as eluants. The product was lyophilized to yield 12 mg of a pink flocculent powder. mp 185-190 °C (dec). MS(Cl) m/e 659 $(M+H)^+$, 691 $(MH+CH_3OH)^+$. $^1$H NMR (DMSO-$d_6$, 360 MHz) $\delta$ 0.90 (m, 3H), 1.20-1.65 (m, 5H), 1.19-2.15 (m, 2H), 2.15-2.45 (m, 1H), 2.45-2.60 (m, 3H), 2.75-3.00 (m, 5H), 3.40-3.25 (m, 3H), 3.50 (m, 1H), 3.60 (m, 1H), 3.69 (m, 1H), 3.81 (m, 1H), 3.95-4.00 (m, 1H), 4.05-4.20 (m, 2H), 4.52 (m, 1H), 4.94 (m, 1H), 5.10 (m, 1H), 6.77 (d, J=7.2 Hz, 1H), 7.05-6.90 (m, 2H), 7.30-7.05 (m, 7H). Anal calcd for $C_{35}H_{42}N_6O_7$ 0.3$CH_3OH$ 0.9$CF_3CO_2H$: C 57.80, H 5.77, N 10.90; Found: C 57.71, H 6.09, N 11.24.

## Example 53

### Ctp-Cpp-Asp-Phe-$NH_2$ (isomer A)

## Example 53a

### Cpp-Asp(OBn)-Phe-$NH_2$, HCl

To solution of N-Boc-*cis*-3-n-propyl-proline (61 mg, 0.23 mmol) (prepared from the corresponding methyl ester (example 52e) via acid hydrolysis (6 N HCl/100 °C/6 h) and treating the resulting amino acid with Boc-ON and diisopropyl ethylamine in aqueous dioxane), TFA.Asp(OBn)-Phe-$NH_2$ (141 mg, 0.29 mmol), 1-hydroxybenzotriazole hydrate (44 mg, 0.29 mmol) and diisopropylethylamine (37 mg, 0.29 mmol)

in THF (3 mL) was added 1-(3-dimethylaminopropyl)-3-ethylcarbodiimide hydrochloride (EDCI) (55 mg, 0.29 mmol) at 0 °C under nitrogen. After stirring for 4 days at room temperature, the mixture was partitioned between a 10% aqueous citric acid solution and EtOAc. The organic phase was further washed with a saturated $NaHCO_3$ aqueous solution and brine and dried ($Na_2SO_4$). Concentration in vacuo gave 167 mg of crude Boc-(L)-Cpp-Asp(OBn)-Phe-$NH_2$ and Boc-(D)-Cpp-Asp(OBn)-Phe-$NH_2$: MS (CI) m/e 609 $(M+H)^+$. This crude product was then treated with 10 mL of 1.5 N HCl/HOAc (prepared from 12.5 mL of conc. HCl diluted to 100 mL with AcOH) for 1 h at room temperature. The solvents were removed in vacuo and the solid residue was washed with ether and filtered. The product was lyophilized to yield 126 mg (97%) of the free amino tripeptide hydrochloride salt as a white solid. MS (CI) m/e 509 $(M+H)^+$.

## Example 53b

### Ctp-Cpp-Asp-Phe-$NH_2$ (isomer A)

To a solution of the tripeptide hydrochloride salt of example 53a (124 mg, 0.22 mmol) and BOP-Cl (69 mg, 0.27 mmol) in methylene chloride (2 mL) was added a solution of Ctp-OH (61 mg, 0.25 mmol) in methylene chloride (1 mL)/DMF (2 mL) and diisopropylethylamine (71 mg, 0.55 mmol) at 0 °C under nitrogen. After stirring overnight at room temperature, 0.4 equivalent of BOP-Cl and 0.4 equivalent of diisopropylethylamine were added and let stirred for 24 h. The reaction mixture was diluted with EtOAc, washed with 10% aqueous citric acid, saturated aqueous $NaHCO_3$ solution and brine, and dried ($Na_2SO_4$). Purification by flash chromatography (4% -> 10% MeOH in $CHCl_3$) gave 43 mg of pure faster-eluting diastereomer, 16 mg of mixture, and 36 mg of pure slower-eluting diastereomer. The faster-eluting diastereomer (43 mg, 0.059 mmol) was hydrogenated under one atmosphere of hydrogen at ambient temperature for 6 h using 10% Pd/C (15 mg) in MeOH (5 mL). The reaction mixture was filtered through Celite and the solvent was removed in vacuo. The product was lyophilized to give 37 mg of white solid. mp 175-180 °C. MS (FAB+) m/e 645 $(M+H)^+$, 667 $(M+Na)^+$. $^1H$ NMR (DMSO-$d_6$, 300 MHz) $\delta$ 0.81 (m, 3H), 1.0 (m, 1H), 1.45-1.15 (m, 4H), 1.85-1.50 (m, 1H), 2.12-1.85 (m, 1H), 2.50- 2.12 (m, 1H), 3.00-2.55 (m, 6H), 3.07 (m, 1H), 3.50-3.30 (m, 2H), 3.78-3.58 (m, 2H), 3.85 (m, 1H), 4.63-4.10 (m, 8H), 6.78 (d, J=7.2 Hz, 1H), 6.96 (t, J=8.4 Hz, 1H), 7.30-7.10 (m, 9H). Anal calcd for $C_{34}H_{40}N_6O_7$ 0.6$CF_3CO_2H$. C 59.07, H 5.74, N 11.79; Found: C 59.07, H 6.02, N 12.13.

## Example 54

### Boc-Trp-Tpp-Asp-Trp-$NH_2$

## Example 54a

### Tpp-Asp(OBn)-Trp-$NH_2$ TFA

To solution of N-Boc-*trans*-3-n-propyl-(L)-proline (61 mg, 0.23 mmol), TFA•Asp(OBn)-Phe-$NH_2$ (149 mg, 0.28 mmol), 1-hydroxybenzotriazole hydrate (44 mg, 0.28 mmol) and 1-(3-dimethylaminopropyl)-3-ethylcarbodiimide hydrochloride (EDCI) (55 mg, 0.28 mmol) in THF (2.5 mL) was added diisopropyl ethylamine (37 mg, 0.28 mmol) at 0 °C under nitrogen. After stirring for 2 days at room temperature, the mixture was partitioned between a 10% aqueous Citric acid solution and EtOAc. The organic phase was further washed with saturated $NaHCO_3$ solution and brine. After drying with $Na_2SO_4$, the solvent was removed in vacuo and the residue was taken up in methylene chloride (5 mL) and treated with TFA (5 mL)

for 35 min at room temperature. The reaction mixture was concentrated and the resulting residue was flash chromatographed (10% MeOH/$CHCl_3$) to give 50 mg of the desired product as a yellow powder after lyopholization.

Example 54b

Boc-Trp-Tpp-Asp-Trp-$NH_2$

To a solution of Boc-Trp-OH (25 mg, 0.083 mmol), TFA•Tpp-Asp(OBn)-Trp-$NH_2$ (50 mg, 0.076 mmol), EDCI (18 mg, 0.091 mmol), and HOBt hydrate (14 mg, 0.091 mmol) in methylene chloride (1 mL) was added diisopropylethylamine (12 mg, 0.091 mmol) at 0 °C. After stirring overnight, the reaction mixture was diluted with EtOAc and washed with 10% aqueous citric acid, saturated aqueous NaHCO3 and brine, then dried with $Na_2SO_4$ and concentration in vacuo to yield 70 mg of yellow solid. This material was taken up in MeOH (15 mL) and hydrogenated at one atmosphere of hydrogen in the presence of 5% Pd/$BaSO_4$ at room temperature for 2 h. After filtration of the reaction mixture through Celite and concentration of the filtrate, the resulting residue was chromatographed on a preparative reverse phase HPLC using 0.1% aqueous TFA and acetonitrile as eluants. The product was lyophilized to yield 21 mg of white powder. mp 195-200 °C (decomp.). MS (FAB+) m/e 744 $(M+H)^+$. $^1H$ NMR (DMSO-$d_6$, 300 MHz) δ 0.81 (m, 3H), 1.10-1.50 (m, 14 H), 2.10 (m, 2H), 2.52-2.67 (m, 1H), 2.70-2.81 (m, 1H), 2.82-2.97 (m, 1H), 2.98-3.10 (m, 1H), 3.15-3.30 (m, 1H), 3.35-3.55 (m, 2H), 3.75 (m, 1H), 3.88 (d, J=6.0 Hz, 1H), 4.39 (m, 2H), 4.48 (dd, J=13.5 and 7.5 Hz, 1H), 6.90-7.00 (m, 3H, 1H $D_2O$ exchangeable), 7.01-7.10 (m, 3H, 1H $D_2O$ exchangeable), 7.10-7.20 (m, 3H, 1H, $D_2O$ exchangeable), 7.31 (d, J=8.4 Hz, 2H), 7.44-7.60 (m, 2H), 7.78 (d, J=8.7 Hz, 1H, $D_2O$, exchangeable), 8.32 (d, J=7.5 Hz, 1H, $D_2O$ exchangeable), 10.81 (m, 2H, $D_2O$ exchangeable). Anal calcd for $C_{39}H_{48}N_7O_8$ $CF_3CO_2H$: C 57.40, H 5.87, N 11.43; Found: C 57.75, H 6.03, N 11.72.

Example 55

Boc-β-Nal-Tpp-Asp-α-Nal-$NH_2$

Example 55a

Tpp-Asp(OBn)-α-Nal-$NH_2$.TFA

The TFA salt of Asp(OBn)-α-Nal-$NH_2$ (151 mg, 0.28 mmol), N-Boc-trans-3-n-propyl-(L)-proline (61 mg, 0.24 mmol), 1-hydroxybenzotriazole hydrate (44 mg, 0.28 mmol), 1-(3-dimethylaminopropyl)-3-ethylcarbodiimide hydrochloride (EDCI) (55 mg, 0.28 mmol) and diisopropylethylamine (37 mg, 0.28 mmol) in methylene chloride (3 mL) were reacted under similar conditions to those described in example 4. The product was deprotected with TFA and isolated in a similar manner as in example 54a to yield the desired product as a white solid.

Example 55b

Boc-β-Nal-Tpp-Asp-α-Nal-$NH_2$

Boc-$\beta$-Nal-OH (25 mg, 0.08 mmol), TFA•Tpp-Asp(OBn)-$\alpha$-Nal-$NH_2$ (54 mg, 0.08 mmol), 1-hydroxybenzotriazole hydrate (15 mg, 0.096 mmol), 1-(3-dimethylaminopropyl)-3-ethylcarbodiimide hydrochloride (EDCI) (19 mg, 0.096 mmol), diisopropylethylamine (13 mg, 0.096 mmol) and methylene chloride (2 mL) were reacted under similar conditions to those described in example 54b. The product was hydrogenated and purified in a similar manner as those described in example 54b to give 35 mg of the desired product as a white solid. mp 190-200 °C (dec). MS (FAB+) m/e 766 $(M+H)^+$, 788 $(M+Na)^+$, 666 ($MH^+$-Boc). $^1H$ NMR (DMSO-$d_6$, 300 MHz) $\delta$ 0.80 (m, 3H), 1.10-1.35 (m, 12H), 1.45 (m, 1H), 1.56 (m, 1H), 2.33 (m, 2H), 2.55 (m, 1H), 2.72 (m, 1H), 2.90 (m, 1H), 3.11 (m, 1H), 3.30 (m, 1H), 3.55-3.70 (m, 2H), 3.80-3.95 (m, 2H), 4.45-4.60 (m, 3H), 7.05-7.60 (m, 10H, 4H $D_2O$ exchangeable), 7.70-8.40 (m, 9H, 1H $D_2O$ exchangeable), 12.50 (br s, 1H, $D_2O$ exchangeable). Anal calcd for $C_{43}H_{51}N_5O_8$ $0.3CF_3CO_2H$: C 65.45, H 6.46, N 8.75; Found: C 65.37, H 6.42, N 8.72.

## Example 56

### Boc-$\beta$-Nal-Tpp-Asp-$\beta$-Nal-$NH_2$

## Example 56a

### Tpp-Asp(OBn)-Nal-$\beta$-$NH_2$.TFA

The TFA salt of Asp(OBn)-$\beta$-Nal-$NH_2$ (151 mg, 0.28 mmol), N-Boc-trans-3-n-propyl-(L)-proline (61 mg, 0.24 mmol), 1-hydroxybenzotriazole hydrate (44 mg, 0.28 mmol), 1-(3-dimethylaminopropyl)-3-ethylcarbodiimide hydrochloride (EDCI) (55 mg, 0.28 mmol) and diisopropyl ethylamine (37 mg, 0.28 mmol) in methylene chloride (3 mL) were reacted under similar conditions to those described in example 54a. The product was deprotected with TFA and isolated in a similar manner as in example 54a to yield the desired product as a white solid.

## Example 56b

### Boc-$\beta$-Nal-Tpp-Asp-$\beta$-Nal-$NH_2$

Boc-$\beta$-Nal-OH (25 mg, 0.08 mmol), TFA•Tpp-Asp(OBn)-$\beta$-Nal-$NH_2$ (54 mg, 0.08 mmol), 1-hydroxybenzotriazole hydrate (15 mg, 0.096 mmol), 1-(3-dimethylaminopropyl)-3-ethylcarbodiimide hydrochloride (EDCI) (19 mg, 0.096 mmol), diisopropyl ethylamine (13 mg, 0.096 mmol) and methylene chloride (2 mL) were reacted under similar conditions to those described in example 54a. The product was hydrogenated and purified in a similar manner as those described in example 54b to give 34 mg of the desired product as a white solid. mp 190-200 °C (dec). MS (FAB+) m/e 766 $(M+H)^+$, 788 $(M+Na)^+$, 666 ($MH^+$-Boc). $^1H$ NMR ($D_2O$, 300 MHz) $\delta$ 0.78 (m, 3H), 1.00-1.32 (m, 12H), 1.39 (m, 1H), 1.52 (m, 1H), 1.99 (m, 2H), 2.55 (m, 1H), 2.72 (m, 1H), 2.90 (m, 1H), 3.05-3.18 (m, 2H), 3.27 (m, 1H), 3.56 (m, 1H), 3.77-3.95 (m, 2H), 4.45-4.60 (m, 3H), 7.10-7.55 (m, 8H, 2H $D_2O$, exchangeable), 7.65-8.10 (m, 10H, 2H $D_2O$, exchangeable), 8.36 (d, J=7.2 Hz, 1H $D_2O$, exchangeable), 12.44 (br s, 1H, $D_2O$ exchangeable). Anal calcd for $C_{43}H_{51}N_5O_8$ $0.8CF_3CO_2H$: C 62.50, H 6.09, N 8.17; Found: C 62.30, H 6.01, N 8.40.

Example 57

Ctp-Tpp-Asp-α-Nal-$NH_2$

To a mixture of Ctp-OH (24 mg, 0.10 mmol), TFA•Tpp-Asp(OBn)-α-Nal-$NH_2$ (67 mg, 0.10 mmol) and Bop-Cl (31 mg, 0.12 mmol) in methylene chloride (2 mL)/DMF (0.5 mL) was added diisopropylethylamine (31 mg, 0.24 mmol) at 0 °C under nitrogen. After stirring overnight at room temperature, the reaction mixture was worked up in a similar manner to those described in example 54b. The product was hydrogenated and purified in a similar manner as those described in example 54b to give 32 mg of the desired product as a white solid. mp 185-190 °C (dec). MS (FAB+) m/e 695 $(M+H)^+$, 713 $(MH+H_2O)^+$. High resolution MS(CI) m/e 695.3198 ($(M+H)^+$, for $C_{38}H_{43}N_6O_7$, calcd 695.3193). $^1H$ NMR (DMSO-$d_6$, 300 MHz) δ 0.81 (m, 3H), 1.27 (m, 3H), 1.36-1.65 (m, 2H), 2.05 (m, 2H), 2.58 (m, 2H), 2.79 (dd, J=16.5, 6.3 Hz, 1H), 3.15-3.48 (m, 3H), 3.50-3.70 (m, 2H), 3.88 (m, 1H), 3.91 (d, J=6.0 Hz, 1H), 4.11 (m, 1H), 4.40 (m, 2H), 4.52 (m, 1H), 6.77 (d, J=7.5 Hz, 1H), 6.96 (t, J=7.5 Hz, 1H), 6.97 (d, J=6.0 Hz, 1H, $D_2O$ exchangeable), 7.15 (2H, 1H $D_2O$ exchangeable), 7.20-7.40 (m, 5H, 2H $D_2O$ exchangeable), 7.51 (m, 2H), 7.77 (d, J=7.8 4Hz, 1H), 7.91 (m, 1H), 7.97 (d, J=8.4 Hz, 1H, $D_2O$ exchangeable), 8.17 (m, 1H), 8.44 (m, 1H, $D_2O$ exchangeable), 10.95 (m, 1H, $D_2O$ exchangeable), 12.50 (br s, 1H, $D_2O$ exchangeable). Anal calcd for $C_{38}H_{42}N_6O_7$ $2CF_3CO_2H$ $1HCON(CH_3)_2$ $1H_2O$: C 53.31, H 5.27, N 9.67; Found: C 53.42, H 4.95, N 9.67.

Example 58

Ctp-Tpp-Asp-β-Nal-$NH_2$

To a mixture of Ctp-OH (24 mg, 0.10 mmol), TFA•Tpp-Asp(OBn)-β-Nal-$NH_2$ (67 mg, 0.10 mmol) and Bop-Cl (31 mg, 0.12 mmol) in methylene chloride (2 mL)/DMF (0.5 mL) was added diisopropyl ethylamine (31 mg, 0.24 mmol) at 0 °C under nitrogen. After stirring overnight at room temperature, the reaction mixture was worked up in a similar manner to those described in example 54b. The product was hydrogenated and purified in a similar manner as those described in example 54b to give 23 mg of the desired product as a white solid. mp 185-190 °C (dec). MS (FAB+) m/e 695 $(M+H)^+$, 717 $(M+Na)^+$. $^1H$ NMR (DMSO-$d_6$, 300 MHz) δ 0.80 (m, 3H), 1.19 (m, 3H), 1.32-1.62 (m, 2H), 1.98 (m, 2H), 2.50-2.75 (m, 3H), 3.03 (m, 2H), 3.20-3.45 (m, 2H), 3.50-3.70 (m, 3H), 3.82 (m, 1H), 3.91 (m, 1H), 4.12 (m, 1H), 4.40 (m,2H), 4.52 (m, 1H), 6.77 (d, J=7.2 Hz, 1H), 6.95 (t, J=7.2 Hz, 1H), 6.96 (m, 1H, $D_2O$ exchangeable), 7.17 (m, 2H, 1H $D_2O$ exchangeable), 7.22 (d, J=8.1 Hz, 1H), 7.30-7.55 (m, 4H, 1H $D_2O$ exchangeable), 7.65-7.89 (m, 5H, 1H $D_2O$ exchangeable), 7.93 (m, 1H, $D_2O$ exchangeable), 8.47-8.52 (m, 1H, $D_2O$ exchangeable), 10.9-11.0 (m, 1H, $D_2O$ exchangeable) . Anal calcd for $C_{38}H_{42}N_6O_7$ $0.9CF_3CO_2H$: C 59.95, H 5.42, N 10.54; Found: C 59.59, H 5.75, N 10.86.

Example 59

Ctp-Tpp-Asp-Cha-$NH_2$

Example 59a

Tpp-Asp(OBN)-Cha-$NH_2$.TFA

The TFA salt of Asp(OBn)-Cha-$NH_2$ (95 mg, 0.19 mmol), N-Boc-trans-3-n-propyl-(L)-proline (50 mg, 0.19 mmol), 1-hydroxybenzotriazole hydrate (45 mg, 0.23 mmol), 1-(3-dimethylaminopropyl)-3-ethylcarbodiimide hydrochloride (EDCI) (45 mg, 0.23 mmol) and diisopropylethylamine (30 mg, 0.23 mmol) in methylene chloride (7 mL) were reacted under similar conditions to those described in example 54a. The product was deprotected with TFA and isolated in a similar manner as in example 54a to yield 66 mg of product as a white solid.

## Example 59b

### Ctp-Tpp-Asp-Cha-$NH_2$

To a mixture of Ctp-OH (27 mg, 0.11 mmol), TFA•Tpp-Asp(OBn)-Cha-$NH_2$ (66 mg, 0.10 mmol) and Bop-Cl (32 mg, 0.13 mmol) in methylene chloride (5 mL)/DMF (1.5 mL) was added diisopropyl ethylamine (33 mg, 0.25 mmol) at 0 °C under nitrogen. After stirring overnight at room temperature, the reaction mixture was worked up in a similar manner to those described in example 54b. The product was hydrogenated and purified in a similar manner as those described in example 54b to give 22 mg of the desired product as a white solid. mp 145-150 °C (dec). MS (FAB+) m/e 651 $(M+H)^+$. $^1H$ NMR ($CD_3OD$, 300 MHz) δ 0.91 (m, 3H), 1.25-1.65 (m, 17H), 1.96-2.20 (m, 2H), 2.70-3.10 (m, 2H), 3.25 (m, 1H), 3.41 (m, 1H), 3.53 (m, 1H), 3.72 (d, J=7.5 Hz, 1H), 4.29 (q, J=7.8 Hz, 1H), 4.58- 4.70 (m, 1H), 4.74-4.95 (m, 2H), 5.13 (s, 2H), 7.28-7.39 (m, 5H). Anal calcd for $C_{34}H_{46}N_6O_7$ 1.5$CF_3CO_2H$ 1.3 $H_2O$: C 52.58, H 5.98, N 9.94; Found: C 52.39, H 5.73, N 10.33.

## Example 60

### Ctp-1,4-thiazane-3-carbonyl-Asp-Phe-$NH_2$

## Example 60a

### BOC-1,4-Thiazane-3-Carboxylic Acid

A heterogeneous suspension of thiazane-3-carboxylic acid (J. F. Carson, F F. Wong, J. Org. Chem. , 29 , 2203 (1964)) (0.28 g, 1.88 mmol) and BOC-ON [2-(*tert*-butoxycarbonyloxyimino)-2-phenylacetronitrile] (0.70 g, 2.84 mmol) and TEA (0.29 g, 2.87 mmol) in acetone (5 mL) and water (5 mL) was rapidly stirred at ambient temperature. The mixture was diluted with water, washed several times with ether then acidified with 1M HCl and extracted several times with methylene chloride. The organic phase was washed with brine and dried over sodium sulfate. Evaporation of the solvent in vacuo yielded 0.39 g (84%) of a white solid. MS (EI) m/e 247 M+. $^1H$ NMR(300 MHz,$CDCl_3$) δ 1.47 (br s, 9H), 2.46 (br t, 1H), 2.72 (br t, 1H), 2.92 (dd, J=14Hz, 5Hz, 1H), 3.05-3.37 (m, 2H), 4.19-4.42 (m, 1H), 5.10,5.32 (s, 1H).

## Example 60b

BOC-1,4-Thiazane-3-carbonyl-Asp(OBn)-Phe-$NH_2$

To a solution of example 60a ( 0.13 g. 0.27 mmol) in methylene chloride (5 mL) was added N-methylmorpholine (30 mg, 0.29 mmol), Asp(OBn)-Phe$NH_2$ hydrochloride (50 mg, 0.20 mmol) and HOBT (30 mg, 0.22 mmol). The solution was cooled to 0 °C and DCC (45 mg, 0.22 mmol) was added to form a milky suspension. The bath was allowed to expire and the reaction allowed to stand at ambient temperature for 18 h. The mixture was diluted with ethyl acetate, filtered and the solution was washed with 1M HCl, saturated bicarbonate solution and brine. After drying over sodium sulfate, the solvent was removed in vacuo and the residue chromatographed (silica gel, ethyl acetate:hexane) to yield 104 mg (87%) of a white solid. MS(FAB) m/e 599 $(M+H)^+$. NMR(DMSO-$d_6$, 300 MHz) δ 1.49 (s, 9H), 2.34-2.45 (m, 1H), 2.60-2.86 (m, 4H), 2.92-3.24 (m, 5H), 4.63 (q, 1H), 4.72-4.81 (m, 1H), 4.88 (br s, 1H), 5.08 (dd, 2H), 5.28 (br s, 1H), 6.98 (br d, 1H), 7.08-7.42 (m, 9H).

Example 60c

1,4-Thiazane-3-Carbonyl-Asp(OBn)-Phe-$NH_2$ Hydrochloride

A solution of compound of example 60b (450 mg, 0.75 mmol) in HCl in acetic acid (20 mL) was stirred at ambient temperature for 4h. The addition of ether precipitated the salt which was collected, washed with fresh ether and dried to yield 390 mg (100%) of a white solid. MS(FAB+) m/e 499 $(M+H)^+$. $^1H$ NMR-(DMSO-$d_6$, 300 MHz) δ2.56-3.22 (m, 10H), 3.93 (br s, 1H), 4.35-4.44 (m, 1H), 4.60-4.68 (m, 1H), 5.11 (s, 2H), 7.15-7.46 (m, 10H).

Example 60d

Ctp-1,4-thiazane-3-carbonyl-Asp(OBn)-Phe-$NH_2$

To a homogeneous solution of Ctp-OH (66 mg, 0.27 mmol) in DMF (1 mL) and methylene chloride (7 mL) cooled to 0 °C were added compound of example 60c (173 mg, 0.32 mmol), diisopropylethylamine (119 mg, 0.92 mmol) and BOP-Cl (76 mg, 0.29 mmol). The solution was allowed to stand at 0 °C for 18 h then diluted with 25% isopropanol in chloroform, washed with 1M HCl, saturated bicarbonate solution and brine. After drying over sodium sulfate, the solvent was removed in vacuo to yield a sludge that was chromatographed on silica (Chromatatron) using methanol in chloroform to yield a white solid. MS (FAB+) m/e 725 $(M+H)^+$. $^1H$ NMR(DMSO-$d_6$,300 MHz,partial spectrum due to $H_2O$ interference) δ 2.57-2.90 (m, 3H), 2.90-3.07 (m, 4H), 3.70-3.87 (m, 2H), 3.96-4.05 (m, 1H), 4.12-4.20 (m, 1H), 4.36-4.44 (m, 1H), 4.65-4.84 (m, 3H), 4.96-5.09 (m, 2H), 6.89 (d, 1H), 6.97 (t, J=7Hz, 1H), 7.04 (d, 1H), 7.07-7.42 (m, ca.12H), 7.85 (d, 1H), 8.18 (d, 1H).

Example 60e

Ctp-1,4-thiazane-3-carbonyl-Asp-Phe-$NH_2$

The compound of example 60d (55 mg) was treated with 10% Pd-C (50 mg) in methanol (5 mL) in the presence of cyclohexadiene (0.15 mL). The suspension was stirred at ambient temperature for 24 h at which time an additional quantity of catalyst (50 mg) and cyclohexadiene were added (0.15 mL). After

stirring an additional 24 h, the catalyst was filtered, the solvent removed in vacuo and the residue chromatographed on reverse phase HPLC using acetonitrile and 0.1% TFA. The fractions were collected and lyopholyzed to yield a white, flocculent solid. MS(FAB-) m/e 633 $(M-H)^-$. Anal. calcd for $C_{31}H_{34}N_6O_7S \cdot 3CF_3CO_2H$: C 45.50, H 3.82, N 8.60; found: C 45.46, H 3.02, N 9.17.

## Example 61

### Ctp-Pip-Asp-Phe-$NH_2$

## Example 61a

### Cbz-Asp(O-t-Bu)-Phe-$NH_2$

To a -10 °C solution of Cbz-Asp(O-t-Bu) (2.5 g, 4.95 mmol) in THF (40 mL) were added N-methylmorpholine (0.52 g, 5.18 mmol) followed by isobutylchloroformate (0.705 g, 5,16 mmol) to form a white precipitate. The suspension was stirred for 4 min and a solution of Phe-$NH_2$ (0.81 g, 4.94 mmol) in DMF (10 mL) and water (5 mL) were added. The solution was stirred at -15 °C for additional 10 min then allowed to warm to ambient temperature. After 6 h, the solution was diluted with ethyl acetate, washed with 1M HCl, saturated bicarbonate solution and brine. After drying over sodium sulfate, the solvent was removed in vacuo to yield 2.07 g (89%) of a white solid which was recrystallied from ethyl acetate. MS-(FAB+) m/e 470 (M+H+). $^1H$ NMR (300 MHz,$CDCl_3$) $\delta$ 1.41 (s, 9H), 2.67 (dd, J=6 and 15Hz, 1H), 2.80 (dd, 1H), 3.03-3.22 (m, 2H), 4.42 (m, 2H), 4.64 (m, (2H), 5.17 (dd, 2H), 7.15-7.40 (m, 10H).

## Example 61b

### Asp(O-t-Bu)-Phe-$NH_2$ Acetate

A mixture of compound of example 61a (1.20 g, 2.56 mmol) and 0.60 g 10% Pd-C in methanol (60 mL) was stirred under one atmosphere of hydrogen gas for one hour. The catalyst was filtered, washed with fresh methanol followed by acetic acid then evaporated to a yield 0.89 g (100%) of a white solid. MS-(FAB+) m/e 336 $(M+H)^+$. $^1H$ NMR (300 MHz, DMSO-$d_6$) $\delta$ 1.38 (s, 9H), 1.95 (s, 3H), 2.18 (dd, J=8Hz and 15Hz, 1H), 2.45 (dd, 1H), 2.84 (dd, 1H), 3.00 (dd, 1H), 3.44 (m, 1H), 4.42 (m, 1H), 7.12-7.29 (m, 5H).

## Example 61c

### Cbz-Pip

To a 0 °C suspension of pipecolic acid (0.15 g, 1.16 mmol) and potassium carbonate (4.64 mmol) in water (5 mL) and THF (5 mL) was added benzyl chloroformate (0.24 g, 1.41 mmol) The mixture was rapidly stirred for one hour then allowed to warm to ambient temperature. After 6 h, the reaction was diluted with water and washed with ether. The aqueous layer was acidified and extracted with methylene chloride. The organic phase was washed with brine then dried over sodium sulfate. The solvent was evaporated and the

residue recrystallized from hexane-ethyl acetate to yield 0.23 g (75%) of a white solid. MS(CI) m/e 264 $(M+H)^+$. $^1H$ NMR (300 MHz, $CDCl_3$) δ 1.24-1.80 (m, 5H), 2.26 (br t, 1H), 2.93-3.14 (m, 1H), 4.00-4.19 (m, 1H), 4.87-5.05 (m, 1H), 5.15 (br s, 2H), 7.27-7.41 (m, 5H).

## Example 61d

### Cbz-Pip-Asp(tBu)-Phe-$NH_2$

To a solution of Cbz-Pip (0.18 g, 0.68 mmol) in THF (20 mL) at -10 °C were added N-methylmorpholine (73 mg, 0.72 mmol), followed by isobutyl chloroformate (98 mg, 0.72 mmol) to form a white suspension that was stirred for 3 min. To the suspension was added a solution of the compound of example 61b (284 mg, 0.72 mmol) and N-methylmorpholine (73 mg) in THF (3 mL) and DMF (2 mL). The solution was stirred at -10 °C for 15 min, then allowed to warm to ambient temperature. After 4 h, the THF was removed in vacuo and the residue diluted with ethyl acetate, washed with 1M HCl, saturated bicarbonate solution and brine. The solvent was dried over sodium sulfate then evaporated. The resulting solid was chromarographed (silica, methanolchloroform) to yield 400 mg of a white solid. MS(FAB+) m/e 581 $(M+H)^+$. $^1H$ NMR (300 MHz, $CDCl_3$) δ 1.25-1.70 (m, 6H), 1.40 (s, 9H), 2.13 (br d, 1H), 2.62-2.73 (m, 2H), 3.13 (br s, 2H), 4.57-4.68 (br m, 3H), 5.16 (s, 2H), 7.16-7.40 (m, 5H).

## Example 61e

### Pip-Asp(t-Bu)-Phe-$NH_2$ Acetate

A mixture of the compound of example 61d (400 mg) and 10% Pd-C (200 mg) in methanol (15 mL) was hydrogenated under an atmosphere of hydrogen gas. After one hour, the catalyst was filtered, rinsed with fresh methanol and acetic acid and evaporated in vacuo to yield 328 mg of a white solid. MS(CI) m/e 447 $(M+H)^+$. $^1H$ NMR(300 MHz,DMSO-$d_6$) δ 1.20-1.58 (m, 2H), 1.34 (s, 9H), 1.63-1.73 (m, 2H), 2.39-2.62 (m, 2H), 2.77-3.11 (m, 4H), 4.31-4.41 (m, 1H), 4.49-4.58 (m, 1H), 7.08-7.42 (m, 4H), 7.81-7.93 (m, 1H).

## Example 61f

### Ctp-Pip-Asp(O-t-Bu)-Phe-$NH_2$

A solution of Ctp (50 mg, 0.21 mmol), compound of example 61e (115 mg, 0.23 mmol), diisopropylethylamine (89 mg, 0.69 mmol), and BOP-Cl (58 mg, 0.23 mmol) in methylene chloride (6 mL) and DMF (2 mL) was stirred at 0 °C for 36 h. The mixutre was diluted with ethyl acetate, washed with 1M HCl, saturated sodium bicarbonate solution and brine. The solvent was dried over sodium sulfate then evaporated in vacuo. The residue was chormatographed (silica, methanolchloroform) to yield 72 mg (52%) of a white solid. MS(FAB-) m/e 671 (M-H)-. $^1H$ NMR (300 MHz, $CDCl_3$) δ 1.35-1.76 (m, 6H), 1.43 (s, 9H), 2.12 (br d, 1H), 2.66 (dd, 1H), 2.87 (dd, 1H), 3.04-3.22 (m, 2H), 3.25-3.49 (m, 2H), 3.50 (dd, 1H), 3.71-3.85 (m, 3H), 4.22 (d, 1H), 4.60-4.70 (m, 2H), 5.04 (br s, 1H), 6.89 (d, 1H), 7.03-7.34 (m, 9H), 8.25 (br s, 1H).

## Example 61g

Ctp-Pip-Asp-Phe-$NH_2$

To a solution of the compound of example 61f (37 mg) in acetic acid (5 mL) was bubbled in a stream of HCl gas for 30 min. The acetic acid was removed in vacuo, dissolved in water and acetone and lyopholyzed. The resulting solid was chromatographed (silica gel, acetic acid-water-pyridine-ethyl acetate) to yield 28 mg of a white flocculent solid after lyopholyzation. MS(FAB+) m/e 617 $(M+H)^+$. $^1H$ NMR (300 MHz, DMSO-$d_6$) δ 1.23-1.67 (m, 6H), 2.14 (br d, 1H), 2.65 (dd, 1H), 2.77-2.90 (m, 2H), 3.06 (dd, 1H), 3.24-3.40 (m, 2H), 3.75-4.05 (m, 2H), 4.31-4.40 (br m, 1H), 4.50-4.86 (m, 3H), 5.08 (br s, 1H), 6.83 (d, 1H), 7.00 (t, J=7Hz, 1H), 7,18-7.32 (m, 8H). Anal calc for $C_{32}H_{36}N_6O_7 \cdot 1.5H_2O$: C 59.71, H 6.11, N 13.06; found: C 59.62, H 5.76, N 12.90.

Example 62

BOC-Trp-(N-1,4-thiazepinyl-2-carbonyl)-Asp-Phe-$NH_2$

Example 62a

S-(3-hydroxypropyl)-Cys

The compound was prepared in a similar manner to that described by J. F. Carson, et al, J. Org. Chem. , 29 , 2203 (1964). To a solution of sodium (6.44 g, 0,281 mol) in liquid ammonia (300 mL) was added Cys (15.0 g, 62.5 mmol) over a 30 min period until permanent discharge of blue. Bromopropanol (15,8 mL, 0.175 mol) was added dropwise over 1 h. The ammonia was allowed to evaporate and the resulting solid was dissolved in 1 M HCl and passed through a column of Dowex 50 (H+) (5X30 cm) eluting with 1N ammonium hydroxide. Lyopholyzation yielded 21.3 g of a white solid. MS(Cl) m/e 180 $(M+H)^+$. $^1H$ NMR (DMSO-$d_6$, 300 MHz) δ 1.70-2.00 (m, 3H), 2.70 (t, J=7.5Hz, 2H), 3.00-3.21 (m, 3H), 3.58-3.84 (m, 3H), 3.93 (dd, 1H).

Example 62b

BOC-1,4-Thiazepine-3-carboxylic acid

The compound from example 62a (5.0 g, 27.9 mmol) was dissolved in concentrated HCl (400 mL) and heated at 90 °C for 16 h. The mixture was evaporated to a white solid to which a solution of sodium bicarbonate (5.8 g, 70 mmol) in 1:1 ethanol:water (200 mL) was added then refluxed for 17 h. Di-t-butyl-dicarbonate (9.6 mL, 42 mmol) was added to the cooled solution and allowed to stand for 16 h. The volume of the reaction was then reduced to 100 mL, acidified with 1 M $H_3PO_4$ and extracted with 25% isopropanol-chloroform. The combined organic phase was washed with brine, dried over sodium sulfate and evaporated to a residue, which was chromatographed (silica, methanol-chloroform) to yield 0.91 g of a white solid. MS-(Cl) m/e 262 $(M+H)^+$. $^1H$ NMR (DMSO-$d_6$, 300 MHz) δ 1.46-1.51 (s, 9H), 1.78-2.05 (m, 2H), 2.50-2.62 (m, 2H), 2.68-2.78 (m, 1H), 2.80-3.00 (m, 1H), 3.20-3.25 (m, 1H), 3.75-3.80 (m, 1H), 4.68-4.85 (m, 1H).

Example 62c

BOC-1,4-Thiazepine-3-carbonyl-Asp-(OBn)-Phe-$NH_2$

The compound was prepared in a similar manner to that described for example 60b. MS(CI) m/e 613 $(M+H)^+$. $^1H$ NMR (DMSO-$d_6$, 300 MHz) δ 1.34-1.45 (m, 9H), 1.62-1.80 (m, 1H), 1.88-1.96 (m, 1H), 2 40-2.52 (m, 2H), 2.58-2.65 (m, 3H), 2.78-3.18 (m, 3H), 3.42-3.47 (m, 1H), 3.63-3.84 (m, 1H), 4.31-4.45 (m, 1H), 4.48-4.50 (m, 1H), 4.58-4.66 (m, 1H), 5.07 (s, 2H), 7.13-7.38 (m, 10H), 7.78-7.84 (dd, 1H), 8.15 (d, 1H), 8.54-8.65 (dd, 1H).

Example 62d

BOC-Trp-(N-1,4-thiazepinyl-2-carbonyl)-Asp(OBn)-Phe-$NH_2$

The peptide of example 62c (120 mg, 0.20 mmol) was treated with a solution of HCl in acetic acid (3 mL) for 1 h. The solution was then frozen and lyopholyzed to yield 108 mg of a white solid that was then allowed to react in a manner similar to that described for example 60d. Chromatography (silica gel, methanol-chloroform) yield 58 mg of a white solid. MS(FAB+) m/e 799 $(M+H)^+$. $^1H$ NMR (DMSO-$d_6$, 300 MHz) δ 1.14 (s, 2H), 1.26-1.34 (s, 9H), 1.71-2.12 (m, 3H), 2.45-3.27 (m, 8H), 3.41-3.50 (m, 1H), 4.32-4.70 (m, 3H), 4.90-5.18 (m, 3H), 6.95-7.40 (m, 15H), 7.50 (d, 1H), 7.90 (t, 1H), 8.15 (d, 1H), 10.83 (m, 1H).

Example 62e

BOC-Trp-(N-1,4-thiazepinyl-2-carbonyl)-Asp-Phe-$NH_2$

A solution of the peptide of example 31 (50 mg, 0.063 mmol), 10% palladium on barium sulfate (80 mg) and 1,4-cyclohexadiene (2 mL) in methanol (3 mL) was stirred at 50 °C for 24 h. The catalyst was filtered, washed with fresh methanol and the filtrate was evaporated in vacuo to a residue. Chromatography (silica gel, pyridine-acetic acid-water-ethyl acetate) followed by lyopholyzation of the residue upon evaporation yielded 21 mg of a white solid. MS(FAB+) m/e 704 $(M+H)^+$. $^1H$ NMR (DMSO-$d_6$, 300 MHz) δ 1.30 (s, 9H), 1.80-2.10 (m, 3H), 2.39-3.30 (m, 10H), 3.62-3.75 (m, 1H), 3.98-4.50 (m, 2H), 4.65-4.80 (m, 1H), 4.90-5.00 (m, 1H), 6.05-6.13 (m, 1H), 6.75-7.25 (m, 9H), 7.35 (d, 1H), 7.55 (d, 1H), 7.80-7.96 (m, 1H), 10.55-10.61 (m, 1H). Anal calcd for $C_{35}H_{44}N_6O_8S \cdot 1.5\ H_2O$: C 57.12, H, 6.44, N 11.42; found: C 56.92, H, 6.12, N 11.05.

Example 63

BOC-Trp-Cha-Asp-Phe-$NH_2$

Example 63a

BOC-Cha-Asp(OBn)-Phe-$NH_2$

The compound was prepared in a manner similar to that described for example 60b. MS(FAB+) m/e

623 $(M+H)^+$. $^1H$ NMR (DMSO-$d_6$, 300 MHz) δ 0.74-0.94 (m, 2H), 1.05-1.39 (m, 2H), 1.37 (s, 10H), 1.56-1.78 (m, 6H), 2.40-2.67 (m, 2H), 2.77-2.86 (m, 2H), 2.98-3.07 (m, 1H), 3.41 (m, 1H), 3.96-4.04 (m, 1H), 4.28-4.36 (m, 1H), 4.41-4.50 (m, 1H), 5.05 (s, 2H), 6.88 (d, 1H), 7.13-7.40 (m, 10H), 7.94 (d, 1H), 8.05 (d, 1H).

Example 63b

Boc-Trp-Cha-Asp(OBn)-Phe-$NH_2$

The compound of example 63a (112 mg, 0.02 mmol) was reacted with HCl in acetic acid for 1 h then frozen and lyopholyzed to yield a white solid which was reacted in a manner similar to that described for example 60d. MS(FAB+) m/e 809 $(M+H)^+$. $^1H$ NMR (DMSO-$d_6$, 300 MHz) δ 0.75-0.95 (m, 2H), 1.05-1.19 (m, 2H), 1.25-1.45 (m, 11H), 1.54-1.80 (m, 5H), 2.37-2.65 (m, 2H), 2.80-2.96 (m, 2H), 3.00-3.24 (m, 4H), 4.08 (m, 1H), 4.24 (m, 1H), 4.34 (m, 1H), 4.50 (m, 1H) , 5.08 (s, 2H) , 6. 80 (d, 1H), 6.91-7 .45 (m, 15H), 7.58 (d, 1H), 7. 92 -8.04 (m, 2H), 8. 26 (d, 2H) .

Example 63c

BOC-Trp-Cha-Asp-Phe-$NH_2$

The compound of example 63b (200 mg, 0.25 mmol) and 10% Pd-C in methanol (8 mL) was stirred at ambient temperature under a balloon of hydrogen gas for 2 h. The catalyst was filtered, washed with fresh methanol and the residue upon solvent removal was chromatographed on silica (ethyl acetate:water:acetic acid:pyridine). The solvents were removed in vacuo and the residue was recrystallized from ethanol:water MS(FAB+) m/e 719 $(M+H)^+$. $^1H$ NMR (DMSO-$d_6$, 300 MHz) δ 0.75-0.95 (m, 2H), 1.05-1.47 (m, 12H), 1.55-1.81 (m, 6H), 2.40-3.20 (m, 8H), 4.15-4.53 (m, 4H), 6.81-6.85 (d, 1H), 6.94-7.36 (m, 11H), 7.60 (m, 1H), 7.87-7.97 (m, 2H), 8.22-8.31 (m, 1H). Anal calcd for $C_{38}H_{50}N_6O_8 \cdot 2H_2O$ 0.5 EtOH: C 60.29, 2H 7.26, N 10.82; found: C 60.41, H 6.95, N 10.57.

Example 64

2-[3-(Indole-2-methyl)diketopiperazinyl]valeryl-Asp-Phe-$NH_2$

Example 64a

Nva-N-(carbomethoxymethyl) benzyl ester

To a solution of norvaline benzyl ester hydrochloride (1.00 g, 4.11 mmol) in acetonitrile (30 mL) were added diisopropylethylamine (55 g, 4 mmol), potassium carbonate (1.6 g) and methyl bromoacetate (0.71 g, 4.65 mmol). The heterogeneous mixture was stirred at ambient temperature for 18 h at which time an additional portion of bromoacetate was added (0.32 g, 2.11 mmol) and sodium iodide (63 mg) The mixture was allowed to stand an additional 18 h at ambient temperature then diluted with ethyl acetate and washed with water and brine. After drying over sodium sulfate, the solvent was removed in vacuo and the oily

residue chromatographed (silica gel, hexane-ethyl acetate) to yield 0.91 g (84%) of a clear, colorless oil. MS(CI) m/e 280 $(M+H)^+$. $^1H$ NMR (300 MHz, $CDCl_3$) $\delta$ 0.90 (t, J=7Hz, 3H), 1.32-1.45 (m, 2H), 1.57-1.76 (m, 2H), 3.31-3.48 (m, 3H), 3.70 (s, 3H), 5.16 (s, 2H), 7.32-7.40 (m, 5H).

## Example 64b

### Cbz-D-Trp-Nva-N-(carbomethoxymethyl) benzyl ester

A solution of the compound of example 64a (0.70 g, 2.51 mmol), Cbz-D-Trp (1.27 g, 3,75 mmol), diisopropylethylamine (1.06 g, 8.2 mmol) and BOP-Cl (1.15 g, 4.52 mmol) in methylene chloride (40 mL) and DMF (5 mL) was allowed to stand at 0 °C for three days. The mixture was diluted with ethyl acetate, washed with saturated bicarbonate solution and dried over sodium sulfate. The solvent was removed in vacuo and the residue chromatographed (silica gel, ethyl acetate hexane) to yield 0.17 g of a clear, colorless oil. MS (FAB+) m/e 600 $(M+H)^+$. $^1H$ NMR (300 MHz, $CDCl_3$) $\delta$ 0.78-0.92 (m, 3H), 1.19-1.45 (m, 2H), 1.55-1.85 (m, 4H), 3.08-3.85 (m, 3H), 3.55, 3,70 (s, 3H), 4.02 (dd, 1H), 4.94-5.12 (m, 4H), 6.98-7.68 (m, 16H).

## Example 64c

### 2-[3-(Indole-2-methyl)diketopiperazinyl]valeric acid

A solution containing the compound of example 64b (0.16 g) and 10% Pd-C (80 mg) in methanol (25 mL) was hydrogenated under a balloon of hydrogen gas. After 3 h, the catalyst was filtered, washed with fresh methanol and evaporated to a glassy solid that was chromatographed (silica gel, pyridine-acetic acid-water-ethyl acetate) to yield 75 mg of a white, flocculent solid after lyophilyzation.

## Example 64c

### 2-[3-(Indole-2-methyl)diketopiperazinyl]valeryl-Asp(OBn)-Phe-$NH_2$

A solution of acid of example 64b (60 mg, 0.17 mmol), TFA-Asp(OBn)-Phe-$NH_2$ (110 mg, 0.18 mmol), diisopropylethylamine (24 mg, 0.19 mmol), HOBT (47 mg, 0.35 mmol) and EDCI (35 mg, 0.18 mmol) in methylene chloride (20 mL) and DMF (5 mL) was stirred at ambient temperature for 18 h. The mixture was diluted with ethyl acetate and washed with 1M phosphoric acid, saturated sodium bicarbonate solution and brine. The solvent was dried over sodium sulfate and evaporated to a residue that was chromatographed (silica gel, methanol-chloroform) to yield 114 mg of a white solid. MS(FAB+) m/e 695 $(M+H)^+$. $^1H$ NMR (300 MHz, DMSO-$d_6$) $\delta$ 0.75-0.90 (m, 3H), 0.96-1.19 (m, 2H), 1.41-1.70 (m, 2H), 2.38-2.66 (m, 2H), 2.74-3.20 (m, 4H), 3.28-3.58 (m, 2H), 4.10 (br s, 1H), 4.25-4.85 (m, 4H), 5.07 (br s, 1H), 5.72 (br m, 2H), 6.90-7.55 (m, 16H)

## Example 64d

### 2-[3-(Indole-2-methyl)diketopiperazinyl]valeryl-Asp-Phe-$NH_2$

A solution of the peptide of example 64c (101 mg, 0.15 mmol) and 10% Pd-C (50 mg) in methanol (15 mL) was hydrogenated under a balloon of hydrogen gas. After 2 h, the catalyst was filtered, washed with fresh methanol and evaporated to a residue that was purified by preparative reverse phase HPLC (C-18, acetonitrile-water) and lyopholyzed to yield 40 mg of a white flocculent product. MS(FAB+) m/e 605 $(M+H)^+$. $^1H$ NMR (DMSO-$d_6$, 500 MHz) δ 0.79 (t, J=7Hz, 3H), 1.03 (br q, 2H), 1.42-1.51 (m, 1H), 1.53-1.62 (m, 1H), 2.42-2.52 (m, 2H), 2.65 (dd, J=6Hz, J=16Hz, 1H), 2.82-2.88 (m, 1H), 3.03 (dd, 1H), 3.11 (d, 2H), 4.08-4.13 (m, 1H), 4.33-4.40 (m, 1H), 4.45-4.51 (m, 1H), 4.64-4.71 (m, 1H), 6.95 (t, J=7Hz, 1H), 7.04 (t, 1H), 7.08-7.36 (m, ca.10H), 7.51 (d, 1H), 7.78 (d, 1H), 8.23 (d, 1H). Anal calcd for $C_{31}H_{35}N_6O_7 \cdot 1.5H_2O$: C 58.94, H 6.22, N 13.30; found: C 59.66, H 5.36, N 13.08.

## Example 65

## 2-[3-(Indole-2-methyl)diketopiperazinyl]isocaproyl-Asp-Phe-$NH_2$

### Example 65a

### Leu-N-(carbomethoxymethyl) Benzyl Ester

The compound was prepared in a manner similar to that described in example 64a. MS(CI) m/e 294 $(M+H)^+$. $^1H$ NMR (300 MHz, $CDCl_3$) δ 0.82-0.90 (m, 3H), 1.23-1.34 (m, 4H), 1.59-1.66 (m, 2H), 3.29-3.37 (m, 1H), 3.35 (d, J=17Hz, 1H), 3.45 (d, 1H), 3 71 (s, 3H), 5.17 (d, 2H), 7.32-7.42 (m, 5H).

### Example 65b

### Cbz-D-Trp-Leu-N-(carbomethoxymethyl) Benzyl Ester

The compound was prepared in a manner similar to that described in example 64b. MS(CI) m/e 614 $(M+H)^+$. $^1H$ NMR (300 MHz, $CDCl_3$) δ 0.78-0.89 (m, 6H), 1.03-1.35 (m, 3H), 3.05-3.40 (m, 2H), 3,55, 3,70 (s, 3H), 4.00-4.25 (m, 2H), 4.55 (br t, 1H), 4.72-5.15 (m, 6H), 5.46-5.56 (m, 1H), 6.98-7.40 (m, 16H).

### Example 65c

### -[3-(Indole-2-methyl)diketopiperazinyl]isocaproic acid

The compound was prepared in a manner similar to that described in example 64c. MS(CI) m/e 358 $(M+H)^+$. $^1H$ NMR (300 MHz, DMSO-$d_6$) δ 0.78-0.86 (m, 3H), 0.93-1.29 (m, 4H), 1.52-1.67 (m, 1H), 1.74-1.87 (m, 1H), 3.13 (d, 2H), 3.60 (br s, 2H), 4.12 (br t, 1H), 4.52-4.60 (m, 1H), 6.90-7.16 (m, 3H), 7.32 (d, 1H), 7.52 (d, 1H).

Example 65d

2-[3-[Indole-2-methy])diketopiperazinyl]isocaproyl-Asp(OBn)-Phe-$NH_2$

The compound was prepared in a manner similar to that described in example 64d. MS(FAB+) m/e 708 $(M+H)^+$. $^1H$ NMR (300 MHz, DMSO-$d_6$) δ 0.76-1.28 (m, 8H), 1.40-1.70 (m, 2H), 2.52-2.63 (dd, 1H), 2.74-3.15 (m, 5H), 4.10 (br t, 1H), 4.34-4.42 (m, 1H), 4.52-4.62 (m, 2H), 5.08 (s, 2H), 6.93 (t, J=7Hz, 1H), 7.02 (t, 1H), 7.08-7.39 (m, 13H), 7.50 (d, 1H), 7.80 (d, 1H), 8.18-8.32 (m, 2H).

Example 65e

2-[3-(Indole-2-methyl)diketopiperazinyl]isocaproyl-Asp-Phe-$NH_2$

The compound was prepared in a manner similar to that described for example 64e. MS(FAB+) m/e 691 $(M+H)^+$. $^1H$ NMR (DMSO-$d_6$, 300 MHz) δ 0.77-0.88 (m, 3H), 0.94-1.06 (m, 2H), 1.14-1.31 (m, 3H), 1.41-1.52 (m, 1H), 1.55-1.69 (m, 1H), 2.52-2.69 (m, 1H), 2.80-2.96 (m, 1H), 2.99-3.18 (m, 3H), 4.08-4.18 (m, 1H), 4.31-4.41 (m, 1H), 4.43-4.53 (m, 1H), 4.65-4.75 (m, 1H), 6.89-7.58 (m, ca.12H), 8.15-8.36 (m, 3H) Anal Calcd for $C_{32}H_{38}N_6O_7$ $H_2O$: C 60.37, H 6.33, N 13.20; found: C 60.54, H 5,87, N 12.96.

Example 66

2-[3-(Indole-2-methyl)diketopiperazinyl]caproyl-Asp-Phe-$NH_2$

Example 66a

Nle-N-(carbomethoxymethyl) Benzyl Ester

The compound was prepared in a manner similar to that described for example 64a. MS(Cl) m/e 294 $(M+H)^+$. $^1H$ NMR (300 MHz, $CDCl_3$) δ 0.86 (t, 3H), 1.23-1.35 (m, 4H), 1.61-1.77 (m, 2H), 3.30-3.48 (m, 3H), 3.71 (s, 3H), 5.17 (dd, 2H), 7.32-7.40 (m, 5H).

Example 66b

Cbz-D-Trp-Nle-N-(carbomethoxymethyl) Benzyl Ester

The compound was prepared in a manner similar to that described for example 64b. MS(Cl) m/e 614 $(M+H)^+$. $^1H$ NMR(300 MHz,$CDCl_3$) δ 0.78-0.81 (m, 3H), 1.04-1.36 (m, 4H), 1.57-1.90 (m, 2H), 3.07-3.41 (m, 4H), 3.69 (s, 3H), 3.75 (d, J=18Hz, 1H), 4.20 (d, 1H), 4.55 (br t, 1H), 4.86 (d, J=13Hz,1H), 4.95-5.14 (m, 4H), 5.46-5.60 (m, 1H), 6.95-7.40 (m, 14H), 7.57-7.70 (m, 2H), 7.82 (br s, 1H), 7.95 (br s, 1H).

Example 66c

2-[3-(Indole-2-methyl)diketopiperazinyl]caproic acid

The compound was prepared in a similar manner to that described for example 64c. MS(CI) m/e 358 $(M+H)^+$. $^1H$ NMR (300 MHz, DMSO-$d_6$) δ 0.72-0.89 (m, 3H), 0.95-1.31 (m, 4H), 1.53-1.68 (m, 1H), 1.72-1.86 (m, 1H), 3.12-3.18 (m, 2H), 3.58 (d, 2H), 4.13 (br t, 1H), 4.53-4.64 (m, 1H), 6.96 (t, J=7Hz, 1H), 7.05 (t, 1H), 7.12 (s, 1H), 7.32 (d, J=7Hz, 1H), 7.52 (d, 1H), 8.18 (br s, 1H).

Example 66d

2-[3-(Indole-2-methyl]diketopiperazinyl]caproyl-Asp(OBn)-Phe-$NH_2$

The compound was prepared in a similar manner to that described for example 64d. MS(CI) m/e 709 $(M+H)^+$. $^1H$ NMR (300 MHz, $CDCl_3$) δ 0.88 (t, J=7Hz, 3H), 1.00-1.13 (m, 2H), 1.13-1.36 (m, 3H), 2.74 (dd, J=16 and 6Hz, 1H), 2.85 (dd, 1H), 3.08-3.27 (m, 3H), 3.37 (dd, J=18 and 5Hz, 1H), 3.57 (d, J=17Hz, 1H), 3.86 (d, 1H), 4.17 (br t, 1H), 4.25-4.31 (m, 1H), 4.53 (q, 1H), 4.64 (q, 1H), 5.04 (d, J=12Hz, 1H), 5.09 (d, 1H),7.00-7.41 (m, 16Hz), 7.59 (d, J=8Hz, 1H).

Example 66e

2-[3-(Indole-2-methyl)diketopiperazinyl]caproyl-Asp-Phe-$NH_2$

The compound was prepared in a similar manner to that described for example 64e. MS(FAB+) m/e 691 $(M+H)^+$. $^1H$ NMR (300 MHz, DMSO-$d_6$) δ 0.81 (t, H=7Hz, 3H), 0.92-1.06 (m, 2H), 1.13-1.28 (m, 2H), 1.39-1.51 (m, 1H), 1.52-1.67 (m, 1H), 2.38-2.65 (m, 2H), 2.80-2.90 (m, 1H), 3.02-3.15 (m, 2H), 3.42-3.57 (m, 2H), 4.07-4.15 (m, 1H), 4.19-4.29 (m, 1H), 4.41-4.51 (m, 1H), 4.66-4.74 (m, 1H), 6.91-7.53 (m, 12H). Anal Calcd for $C_{32}H_{38}N_6O_7 \cdot H_2O$: C 60.36, H 6.33, N 13.20; found: C 60.29, H 6.41, N 12.82.

Example 67

2-[3-(Naphthyl-2-methyl)diketopiperazinyl]isocaoroyl-Asp-Phe-$NH_2$

Example 67a

Cbz-D-Trp-β-Nal-N-(carbomethoxymethyl) Benzyl Ester

The compound was prepared in a manner similar to that described for example 64a. MS(CI) m/e 625 $(M+H)^+$. $^1H$ NMR ($CDCl_3$, 300 MHz) δ 0.71-0.96 (m, 8H), 1.24-1.38 (m, 1H), 2.95-3.38 (m, 2H), 3.62-3.80

(m, 5H), 4.80-5.18 (m, 4H), 5.25-5.48 (m, 2H), 7.18-7.82 (m, 18H).

Example 67b

2-[3-(Naphthyl-2-methyl)diketopiperazinyl]isocaproic acid

The compound was prepared in a manner similar to that described for compound of example 64b. MS-(CI) m/e 369 $(M+H)^+$. $^1H$ NMR (DMSO-$d_6$, 300 MHz) δ 0.82 (t, 6H), 1.35-1.38 (m, 1H), 1.61-1.75 (m, 2H), 3.11-3.75 (m, 4H), 4.25-4.40 (m, 1H), 4.78-4.85 (m, 1H), 7.33-7.48 (m, 3H), 7.70 (s, 1H), 7.78-7.89 (m, 3H), 8.41 (s, 1H).

Example 67c

2-[3-(Naphthyl-2-methyl)diketopiperazinyl]isocaproyl-Asp(OBn)-Phe-$NH_2$

To a 0 °C solution containing the compound of example 67b (37 mg, 0.102 mmol) in methylene chloride (10 mL) were added TFA•Asp(OBn)-Phe-$NH_2$ (67 mg, 0.112 mmol), HOBT (33 mg, 0.21 mmol), EDCI (21 mg, 0.12 mmol) and N-methylmorpholine (25 µL, 0.22 mmol). The reaction mixture was stirred at 0 °C for 1 h, warmed to ambient temperature and stirred for an additional 16 h. Upon dilution with methylene chloride (100 mL), the reaction mixture was washed with 1M $H_3PO_4$ (3x), saturated sodium bicarbonate solution (3x) and brine. After drying over sodium sulfate, the solvent was evaporated in vacuo and the residue chromatographed on silica gel (methanol:chloroform) to yield 61 mg of a white solid. MS-(FAB+) m/e 720 $M+H)^+$. $^1H$ NMR (DMSO-$d_6$, 300 MHz) δ 0.81-0.92 (m, 6H), 1.24-1.65 (m, 4H), 2.40-2.64 (m, 2H), 2.79-3.61 (m, 4H), 4.21-4.44 (m, 3H), 4.50 (d, 1H), 4.61-4.71 (m, 1H), 4.96-5.14 (m, 3H), 7.15-7.47 (m, 16H), 7.65 (br s, 1H), 7.75-7.85 (m, 3H), 8.26-8.40 (m, 1H), 8.84 (m, 1H).

Example 67d

2-[3-(Naphthyl-2-methyl)diketopiperazinyl]isocaproyl-Asp-Phe-$NH_2$

To a solution containing 5% palladium-on-carbon (20 mg) and 1,4-cyclohexadiene (1 mL) in methanol (2 mL) was added the compound from example 67c (30 mg, 0.042 mmol). The reaction mixture was stirred 17 h after which time the catalyst was filtered off and the solvent evaporated in vacuo. The residue was chromatographed on silica gel (ethyl acetate:water:pyridine:acetic acid) to yield, after lyopholyzation, 15 mg of a white flocculent solid. MS(FAB+) m/e 630 $(M+H)^+$. $^1H$ NMR (DMSO-$d_6$, 300 MHz) δ 0.80 (t, 6H), 1.24-1.61 (m, 3H), 2.77-2.90 (m, 2H), 3.05-3.64 (m, 4H), 4.05 (s, 2H), 4.19-4.25 (m, 1H), 4.30-4.38 (m, 1H), 4.41-4.48 (m, 1H), 4.85-4.91 (m, 1H), 7.18-7.35 (m, 8H), 7.45-7.51 (m, 2H), 7.70 (s, 2H), 7.82-7.89 (m, 3H), 8.26-8.38 (m, 2H), 12.00 (br s, 1H). Anal calcd for $C_{34}H_{39}N_5O_7 \cdot H_2O$: C 63.04, H 6.38, N 10.81; found: C 62.78, H 6.16, N 10.64.

Example 68

Alternative preparation of 4-Carboxy-6-oxo-3,4,5,6-tetrahydro-1H,5H-azocin[4,5,6-c]indole (Ctp-OH)

## Example 68a

### Ethyl α-(hydroxyimino)-β-(4-(carboethoxymethyl)indol-3-yl)-propanoate

To a solution of 4-(carboethoxymethyl)indole (830 mg, 4.12 mmol) and ethyl bromopyruvate 2-oxime (866 mg, 4.12 mmol) in methylene chloride (40 mL) was added anhydrous $Na_2CO_3$ (2.40 g, 22.66 mmol) at room temperature. After stirring at room temperature for 16 hours, the mixture was filtered and concentrated to dryness. The residue was flash chromatographed ((10% $MeOH/CHCl_3$):Hexane=1:1->2:1) to give 0.98 g of the desired product. TLC $R_f$=0.61 (10%$MeOH/CHCl_3$). MS(CI) m/e 333 (M+H). $^1H$ NMR ($CDCl_3$, 300 MHz) δ 8.17 (br s, -NH), 8.03 (br s, -OH), 7.26 (d, J=7.4 Hz, 1H), 7.12 (t, J=7.4 Hz, 1H), 6.98 (d, J=7.4 Hz, 1H) 6.92 (br s, 1H), 4.30 (s, 2H), 4.28 (q, J=7.4Hz, 2H), 4.18 (q, J=7.4 Hz, 2H), 4.13 (s, 2H), 1.30 (t, J=7.4 Hz, 3H), 1.25 (t, J=7.4 Hz, 3H).

## Example 68b

### Ethyl α-(amino)-β-(4-(ethoxycarbonylmethyl)indol-3-yl)-propanoate

Aluminum strips (797 mg, 29.5 mmol) were amalgamated by immersing in a solution of $HgCl_2$ (216 mg, 0.80 mmol) in $H_2O$ (80 mL) for 15 seconds, then rinsed successively in EtOH and in ether, and added to a solution of the product of Example 68a (0.98 g, 2.95 mmol) in $THF/H_2O$ (10:1, 49.5 mL). After stirring for 3 hours, the mixture was dried with $Na_2SO_4$ and filtered through Celite. The filtrate was concentrated in vacuo to give 0.82 g of a yellow oil. TLC $R_f$=0.45 (10% $MeOH/CHCl_3$). MS(CI) m/e 319 $(M+H)^+$. $^1H$ NMR ($CDCl_3$, 300 MHz) δ 8.12(br m, indole -NH), 7.30 (dd, J=8.1, 0.9 Hz, 1H), 7.14 (t, J=8.1 Hz, 1H), 7.09 (br d, J=2.2 Hz, 2H), 4.03 (d, J=15.5 Hz, 1H), 3.50 (ddd, J=14.7, 4.8, 0.7 Hz, 1H) 3.25 (m, 1H), 3.00 (ddd, J=14.7, 8.5, 0.7 Hz, 1H), 1.25 (t, J=7.2 Hz, 3H), 1.26 (t, J=7.2 Hz, 3H).

## Example 68c

### 4-Carboethoxy-6-oxo-3,4,5,6-tetrahydro-1H,5H-azocin[4,5,6-clindole

A solution of the product of Example 68b (794 mg, 2.94 mmol) in o-xylene (50 mL) was heated at 145° C for 3 days under nitrogen. The reaction mixture was filtered through a thin layer of silica (60μm) and the filter cake was washed with 5% MeOH in $CHCl_3$. The filtrate was concentrated and the residue was taken up in $MeOH/CHCl_3$/toluene to precipitate the product. The first two crops provided 353 mg of the desired product as a yellow solid. TLC $R_f$=0.50 (10% $MeOH/CHCl_3$). m.p. 229-230° C. MS(CI) m/e 273 $(M+H)^+$. $^1H$ NMR ($CDCl_3$, 300 MHz) δ 7.24 (d, J=7.6 Hz, 1H), 7.12 (s 1H), 7.01 (t, 1H), 6.87 (d, 1H), 4.47 (dd, J=9.9, 7.7 Hz, 1H), 4.28 (q, J=7.0 Hz, 2H), 4.20 (d, J=12.7 Hz, 1H), 3.76 (d, J=12.7 Hz, 1H), 3.55-3.65 (m, 2H), 1.34 (t, J=7.0 Hz, 3H). Anal. Calcd. for $C_{15}H_{16}N_2O_3$. 1/5 $H_2O$: C, 65.30; H, 5.99; N, 10.15. Found: C, 65 .35; H, 5.89; N, 10.09.

## Example 68d

4-Carboxy-6-oxo-3,4,5,6-tetrahydro-1H,5H-azocin[4,5,6-clindole (Ctp-OH)

To a solution of the product of Example 68c (40mg, 0.147 mmol) in MeOH (1.5 mL) was added 2 N NaOH 981 µl, 0.162 mmol) solution at room temperature. After stirring for 2 hours, the mixture was concentrated and the resulting residue was taken up in saturated NaHCO3 solution and extratcted with $CHCl_3$ (2x). The aqueous layer was acidified carefully with 4 N HCl to pH 2 and then extracted with EtOAc (10x). The EtOAc extracts were dried over $Na_2SO_4$ and concentrated in vacuo to give 31 mg of the desired product as an off-white solid. TLC $R_f$ = 0.14 (pyridine/$H_2O$/HOAc = 20:11:6):EtOAc = 1:3). m.p. 278-280 °C (dec.). MS(DCI/$NH_3$) m/e 245 $(M+H)^+$, 262 (M+NH4)+. Anal. Calcd. for $C_{13}H_{12}N_2O_3$. 1/10 EtOAc: C, 63.60; H, 5.10; N, 11.07. Found: C, 64.00; H, 5.41; N, 10.77.

Example 69

4-Carboethoxy-3,4,5,6-tetrahydro-1H,5H-azocin[4,5,6-clindole

To a solution of the product of Example 68e (53 mg, 0.19 mmol) in THF (6 mL) was added Lawesson's reagent (118 mg, 0.29 mmol) at room temperature. After stirring for 25 minutes, the reaction mixture was concentrated and the resulting residue was purified by flash chromatography (EtOAc:Hexane = 2:3) to give 33 mg of the corresponding thiol lactam. (TLC $R_f$ = 0.40 (EtOAc:Hexane = 1:1)). To a solution of the thiol lactam (33 mg, 0.11 mmol) in methylene chloride (2 mL) was added a 1 M solution of triethyloxonium tetrafluoroborate in methylene chloride (130 µL, 0.13 mmol) at 0 °C under nitrogen. After stirring overnight at room temperature, the solvent was evaporated and to the resulting residue was added EtOH 92mL) and $NaBH_4$ (5 mg, 0.13 mmol) and let stir for 2 hours. The reaction mixture was washed with saturated aqueous $NaHCO_3$ solution and extracted with EtOAc several times. The organic phase was dried ($Na_2SO_4$), concentrated and the resulting residue purified by flash chromatography (10% MeOH/$CHCl_3$) to give 12 mg of the desired product as a viscous oil. TLC $R_f$ = 0.38 (10% MeOH/$CHCl_3$). MS(CI) m/e 259 $(M+H)^+$. $^1H$ NMR ($CDCl_3$, 300 MHz) δ 8.07 (br, indole NH), 7.25 (m, 1H) 7.12 (t, J = 7.0 Hz, 1H), 6.89 (d, J = 2.2 Hz, 1H), 6.83 (d, J = 7.0 Hz, 1H), 4.18 (q, J = 7.0 Hz, 2H), 3.89 (dd, J = 7.0, 3.7 Hz, 1H), 3.52 (br dd, J = 15.1, 7.0 Hz, 1H), 3.45-3.15 (m, 4H), 2.95 (dt, J = 13.0, 4.4 Hz, 1H), 1.27 (t, J = 7.0 Hz, 3H).

The compounds of formula I are CCK ligands selective for the Type-B receptor, which is found predominantly in the brain, and are useful in the treatment and prevention of CCK-related disorders in man or other mammals. The compounds of the invention are useful in the treatment or prevention of CCK-related disorders of the central nervous and gastrointestinal systems. As CCK agonists at the Type-B receptor, the compounds of the invention mimick the effects of CCK on CCK type-B receptors. The compounds of the invention are useful in the treatment of substance abuse, including alcohol and nicotine addiction and also including drugs of abuse such as cocaine, amphetamines, heroin, cannabinoids (THC and the like), phencyclidene (PCP) and the like. The compounds of the invention are also useful in the treatment of disorders of memory and cognition, and treatment of shock, respiratory and cardiocirculatory insufficiencies. The compounds of the invention are also useful in the treatment of eating disorders related to appetite control.

The ability of the compounds of the invention to interact with CCK receptors and to act as CCK agonists can be demonstrated in vitro using the following protocols:

Protocol for Radioligand Binding Experiments in Guinea Pig Cerebral Cortical and Pancreatic Membrance Preparations

CCK8 (Asp-Tyr($SO_3H$)-Met-Gly-Trp-Met-Asp-Phe-$NH_2$), bestatin and phosphormaidon were purchased from Peptide International (Louisville, KY). EGTA, HEPES, BSA were purchased from Sigma Chemical Co. (St. Louis, MO). [$^{125}$I]-Bolton-Hunter CCK8 (BH-CCK8) (specific activity, 2200 Ci, mmol) was obtained from New England Nuclear (Boston, MA). Male ginea pigs, 250 to 325 g, were obtained from Scientific Small Animal Laboratory and Farm (Arlington Heights, IL). Collangenase, code CLSPA was purchased from Worthington (Frehold, NJ).

Cortical and pancreatic membranes were prepared as described (Lin and Miller; J. Pharmacol. Exp. Ther. 232, 775-780, 1985). In brief, pancreas and cortex were removed and rinsed with ice-cold saline. Visible fat and connective tissues were removed from the pancreas. Tissues were weighed and homogenized in approximately 25 mL of ice-cold 50 mM Tris-HCl buffer, pH 7.4 at 4°C with a Brinkman Polytron for 30 sec, setting 7. The homogenates were centrifuged for 10 min at 1075 x g and the pellet was discarded. The supernantants were saved and centrifuged at 38,730 x g for 20 min. The resultant pellets were rehomogenized in 25 mL of 50 mM Tris-HCl buffer with a Teflon-glass homogenizer, 5 up and down strokes. The homogenates were centrifuged again at 38,730 x g for 20 min. The pellets were then resuspended in 20 mM HEPES, containing 1 mM EGTA, 118 mM NaCl, 4.7 mM KCl, 5mM $MgCl_2$, 100 uM bestatin, 3 uM phosphoramidon, pH 7.4 at 22°C with a Teflon-glass homogenizer, 15 up and down strokes. The resuspension volume used for the cortex was 15 mL per g of original wet weight and 60 mL per g for pancreas.

Incubation Conditions

[$^{125}$I]Bolton-Hunter CCK8 and test compounds were diluted with HEPES-EGTA-salt buffer (see above) containing 0.5% bovine serum albumin (BSA). To 1 mL Skatron polystyrene tubes were added 25 uL of test compounds, 25 uL of [$^{125}$I]BH-CCK8 and 200 uL of membrane suspension. The final BSA concentration was 0.1%. The cortical tissues were incubated at 30°C for 150 min. and pancreatic tissues were incubated at 37°C for 150 min. Incubations were terminated by filtration using Skatron Cell Harvester and SS32 microfiber filter mats. The specific binding of [$^{125}$I]BH-CCK8, defined as the difference betwen binding in the absence and presence of 1 uM CCK8, was 85-90% of total binding in cortex and 90-95% in pancreas. $IC_{50}$s were determined from the Hill analysis. The results of these binding assays are shown in Table 1.

Table 1

| Compound of Example | $IC_{50}$ (nM) | $IC_{50}$ (uM) |
|---|---|---|
| | cortex | pancreas |
| 1 | 2.5 | 2.7 |
| 2 | 37 | 19 |
| 9 | 13 | >1 |
| 15 | 5 | 1 |
| 19 | 54 | 1.8 |
| 23 | 16 | >10 |
| 42 | 13 | 3.8 |
| 43 | 170 | 11 |
| 45 | 12 | 1.7 |
| 47 | 1.2 | 89 |
| 48 | 0.7 | 3.5 |
| 50 | 27 | 5 |
| 52 | 2.6 | 3.5 |
| 54 | 5 | 3.7 |
| 57 | 0.4 | 1.4 |
| 58 | 1.9 | 11 |
| 59 | 0.9 | 4.3 |
| 60 | 8.4 | 1.3 |
| 67 | 33 | >10 |

These results indicate that compounds of the invention possess affinity and selectivity for the cortical CCK receptor.

Protocol for Measuring [Ca++]i Changes in NCI-H345 and H209 Cell Lines

Culture conditions were as described (D.G. Yoder, T.W. Moody, Peptides , 8 , 103 (1987), except that the medium was modified to RPMI 1640 with 2.5% fetal bovine serum (heat inactivated), 5μg/l sodium selenite, 5 mg/l human transferrin, 5 mg/l insulin, penicillin (100 U/l) and streptomycin (100 μg/l). To measure intracellular $Ca^{2+}$, cells were loaded with 1 μM of indo-1,AM, for 60 min at 37°C. The cells were centrifuged at 750 x g for 5 min and resuspended in 40 mL Buffer F (Dulbecco's phosphate buffered saline containing 0.1% glucose, pH 7.4). The cells were counted then twice washed by centrifugation. The final pellet was resuspended (approximately 1 x $10^6$ cells/mL) in Buffer F. The cells were thermally equilibrated in a thermostated cuvette (37°C) for 2 min prior to base-line fluorescence measurement. Fluorescence measurements were made with an SLM 8000C spectrofluorimeter equipped with magnetic microstirrer and polarizing filters. Measurements with indo-1-loaded cells were obtained at excitation wavelength of 350 nM and emission wavelength of 480 and 405 nM and the time interval for the measurements was 0.5 sec. The test compounds were assayed at a single concentration ($10^{-5}$ M) and compared to the response elicited by CCK-8 at $10^{-6}$ M to determine the % maximal response. Calibrations of $[Ca++]i$ were done as described (G. Grynkiewicz, M. Poenie, R. Y. Tsien, J. Biol. Chem. , 260 , 3440 (1985): $[Ca]i = Kd(R-R_{min})/(R_{max}-R)$ where $R_{min}$ and $R_{max}$ were the ratios (480/405) obtained in the presence of excess EGTA (10 mM) and digitonin (50 μM), respectively. $K_d$ is assumed to be 240 nM, and R is the ratio in the presence and the absence of CCK. Basal calcium levels were 147 ± 3 (N=3) and the maximal levels of calcium stimulated with 100 nM CCK-8 were 357 ± 30 (N=3).

Table 2

| Compound of Example | Intrinsic Activity Relative to CCK-8 |
|---|---|
| 6 | 96% |
| 44 | 104 |
| 48 | 103 |
| 52 | 100 |
| 57 | 109 |
| 58 | 100 |
| Boc-CCK-4 | 105 |
| CCK-8(DS) | 53 |

The results indicate that compounds of this invention behave as agonists in stimulating calcium mobilization at CCK-B receptors on small cell lung cancer cell lines.

While not intending to be bound by any theoretical mechanisms of action, the ability of the compunds of this invention to treat substance abuse is thought to result from the ability of the compounds of this invention to enhance response of neurons in the brain (specifically in the ventral tegmental area of Tsai (VTS)) to substances of abuse such as ethanol, nicotine and others. Both ethanol (Gessa, et al, Brain Research , 348 , 201 (1985), Brodie, et al, Brain Research , 508 , 65 (1990)) and nicotine (Mereu, et al, Eur. J. Pharmacol. , 141 , 395 (1987), Brodie and Mueller, Soc. Neurosci. Abstr. , 14 , 1327 (1988)) affect VTA neurons by increasing their firing rate. By enhancing the excitation of these neurons which is induced by the abused substance, smaller amounts of the addictive substance will be required, in the presence of the CCK agonists of this invention, to produce the same rewarding effects as in its absence. This will permit an addicted individual to ingest lessened amounts of the abused substance to achieve the accustomed reward sensation, while alleviating the deleterious side effects associated with the higher doses of these drugs.

The ability of the compounds of the invention to potentiate the response of VTA neurons to ethanol can be demonstrated using the method described below.

Protocol:

Brain slices form Sprague-Dawley rats (100-200 gm) containing the ventral tegmental area were prepared as previously described (Brodie and Dunwiddie, Brain Research , 425 , 106 (1987)). Animals used in this study were treated in strict accordance with the NIH Guide for the Care and use of Laboratory Animals. Coronal sections (400 um thick) were cut and the tissue was place directly in the recording chamber. Small platinum weights were placed on the slice to increase the stability of recordings. The slice

was covered with medium and a superfusion system then maintained the flow of medium at 2 ml/min; the temperature in the recording chamber was kept at 35° C. The composition of the artificial cerebrospinal fluid (aCSF) in these experiments was (in mM) : NaCl 125, KCl 2.5, $NaH_2PO_4$ 1.25, $MgSO_4$ 2, $NaHCO_3$ 25, glucose 11, $CaCl_2$ 2; the aCSF was saturated with 95% $O_2$/ 5%$CO_2$. The flow rate was continuously monitored with a flowmeter and adjustable valves were used to keep the rate constant. The small volume chamber (about 300 microliters) used in this study permitted the rapid infusion and washout of drug solutions.

Ethanol and the CCK agonist compound were added to the aCSF in the fluid delivery tubing by means of a calibrated infusion pump from stock solutions 100 to 1000 times the desired final concentrations. Final concentrations were calculated from aCSF flow rate, pump infusion rate and concentration of drug stock solution. Infusion of drug solutiosn never exceeded 2% of the flow rate of the aCSF and usually was kept below 1%. All drugs were dissolved in degassed distilled water; for concentrations above 30 mM, 95% EtOH was used in the pump. Because the pharmacologically active range for blood ethanol in the rat extends from 10-20 mM for mild motor deficits to 200 mM (lethal), these studies were generally limited to application of ethanol in the range of 20 to 200 mM. In practice, the concentration range of ethanol tested with the compound of Example 48 was 80 to 160 mM. Within this range, the percentage increase in ethanol potency produced by the CCK agonist was not significantly dependent upon ethanol concentration.

Extracellular recording electrodes were made from 1.5 mm diameter glass tubing; tip resistance of the microelectrodes ranged from 6-10 MOhm. At least one hour after preparation of the slice was allowed for equilibration. After this period, the electrode was lowered into the VTA under visual guidance. The VTA is clearly visible in fresh tissue as a grey area medial to the substantia nigra.

Frequency of firing was determined using a window discriminator and ratemeter, the output of which was fed to a chart recorder. In addition, an IBM-PC-based data acquisition system was used to calculate, display and store the frequency of firing over 5 sec and 1 minute intervals.

The protocol for administration of ethanol and the CCK agonist was as follows: Ethanol was added to the superfusate via a calibrated infusion pump and the magnitude of ethanol-induced excitation was measured. The ethanol infusion was halted, and the cell firing rate was allowed to return to pre-ethanol levels. The CCK agonist compound of Example 48 was then added to the superfusion medium via a calibrated infusion pump. At least 10 minutes after the initiation of the CCK agonist infusion, ethanol was infused at the same rate (in order to achieve the same concentration) as prior to the CCK agonist infusion. The increases in firing rate (as percentage of pre-ethanol firing rate) induced by ethanol before and after the CCK agonist infusion were compared to yield the numbers in Table 3

The following table summarizes the results of experiments in which compound of Example 48 was tested to assess its ability to alter the potency of ethanol.

Table 3

| Compound of Example 48 Concentration | % Increase in EtOH Potency | Number of Cells Cells Tested |
|---|---|---|
| 50 nM | 6.8 | 5 |
| 100-250 nM | 12.6 | 5 |
| 400-500 nM | 24 | 3 |

These data demonstrate that the compound of Example 48 increases the excitatory effect of ethanol on VTA neurons.

The compounds of the present invention can be used in the form of salts derived from inorganic or organic acids. These salts include but are not limited to the following: acetate, adipate, alginate, citrate, aspartate, benzoate, benzenesulfonate, bisulfate, butyrate, comphorate, camphorsulfonate, digluconate, cyclopentanepropionate, dodecylsulfate, ethanesulfonate, glucoheptonate, glycerophosphate, hemisulfate, heptanoate, hexanoate, fumarate, hydrochloride, hydrobromide, hydroiodide, 2-hydroxy-ethanesulfonate, lactate, maleate, methanesulfonate, nicotinate, 2-naphthalenesulfonate, oxalate, pamoate, pectinate, persulfate, 3-phenylpropionate, picrate, pivalate, propionate, succinate, tartrate, thiocyanate, tosylate, and undecanoate. Also, the basic nitrogen-containing groups can be quaternized with such agents as loweralkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

The pharmaceutically acceptable salts of the present invention can be synthesized from the compounds of formula I which contain a basic or acidic moiety by conventional chemical methods. Generally, the salts are prepared by reacting the free base or acid with stoichiometric amounts or with an excess of the desired salt forming inorganic or organic acid or base in a suitable solvent or various combinations of solvents.

Example of acids which may be employed to form pharmaceutically acceptable acid addition salts include such inorganic acids as hydrochloric acid, sulphuric acid and phosphoric acid and such organic acids as oxalic acid, maleic acid, succinic acid and citric acid. Other salts include salts with alkali metals or alkaline earth metals, such as sodium, potassium, calcium or magnesium or with organic bases.

The pharmaceutically acceptable salts of the acids of formula I are also readily prepared by conventional procedures such as treating an acid of formula I with an appropriate amount of a base, such as an alkali or alkaline earth metal hydroxide e.g. sodium, potassium lithium, calcium, or magnesium, or an organic base such as an amine, e.g., dibenzylethylenediamine, cyclohexylamine, dicyclohexylamine, trimethylamine, piperidine, pyrrolidine, benzylamine and the like, or a quaternary ammonium hydroxide such as tetramethylammonium hydroxide and the like.

When a compound of formula I is used as an agonist of CCK or gastrin in a human subject, the total daily dose administered in single or divided doses may be in amounts, for example, from 0.001 to 1000 mg a day and more usually 1 to 100 mg. Dosage unit compositions may contain such amounts of submultiples thereof to make up the daily dose.

The amount of active ingredient that may be combined with the carrier materials to produce a single dosage form will vary depending upon the host treated, the particular treatment and the particular mode of administration.

It will be understood, however, that the specific dose level for any particular patient will depend upon a variety of factors including the activity of the specific compound employed, the age, body weight, general health, sex, diet, time of administration, rate of excretion, drug combination, and the severity of the particular disease undergoing therapy.

The compounds of the present invention may be administered orally, parenterally, by inhalation spray, rectally, or topically in dosage unit formulation containing conventional nontoxic pharmaceutically acceptable carriers, adjuvants, and vehicles as desired. The term parenteral as used herein includes subcutaneous injection, intravenous, intramuscular, intrasternal injection, or infusion techniques.

Injectable preparations, for example, sterile injectable aqueous or oleagenous suspensions, may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols which are solid at ordinary temperatures but liquid at the rectal temperature and will therefore melt in the rectum and release the drug.

Solid dosage forms for oral administration may include capsules, tablets, pills, powders, and granules. In such solid dosage form, the active compound may be admixed with at least one inert diluent such as sucrose, lactose or starch. Such dosage forms may also comprise, as is normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsion, solutions, suspensions, syrups, and elixirs containing inert diluents commonly used in the art, such as water. Such compositions may also comprise adjuvants, such as wetting agents, emulsifying and suspending agents, and sweetening, flavoring, and perfuming agents.

The present agents can also be administered in the form of liposomes, As is known in the art, liposomes are generally derived from phospholipids of other lipid substances. Liposomes are formed by

mono- or multilamellar hydrated liquid crystals that are dispersed in an aqueous medium. Any non-toxic, physiologically acceptable and metabolizable lipid capable of forming liposomes can be used. The present compositions in liposome form can contain, in addition to the tetrapeptide of the present invention, stabilizers, preservatives, excipients, and the like. The preferred lipids are he phospholipids and the phosphatidyl cholines, (lecithins), both natural and synthetic.

Methods to form liposomes are known in the art. See, for example, Prescott, Ed., Methods in Cell Biology, volume XIV, Academic Press, New York, NY (1976), p 33. et seq.

The foregoing is merely illustrative of the invention and is not intended to limit the invention to the disclosed compounds. Variations and changes which are obvious to one skilled in the art are intended to be within the scope and nature of the invention which are defined in the appended claims.

**Claims**

1. A compound of the formula:

**A-B-C-D**

wherein A is functionalized acetyl or R9-C(O)- wherein $R_9$ is heterotricyclic or carbotricyclic;

B is a functionalized 2-aminopropionyl residue;

or A-B taken together is functionalized piperazinedionyl or functionalized 5-amino-3-aza-4-keto-hexanoyl;

C is

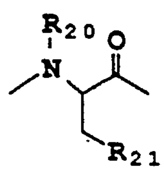

wherein

$R_{20}$ is hydrogen or loweralkyl and

$R_{21}$ is carboxy or tetrazolyl;

or B-C taken together is a bridged Ala-Asp residue or a bridged Ala-(tetrazolyl)Ala residue; and

D is functionalized ethylamino, functionalized tetrahydroisoquinolyl, functionalized piperazinon-1-yl, dehydro-Phe amide or an analog of dehydro-Phe;

or C-D taken together is functionalized succinimidyl; or a pharmaceutically acceptable salt thereof.

2. The compound of Claim 1 wherein A is BOC-Trp or Ctp; B is Met, Leu, Nle, Tpp or 1,4-thiazane-3-carbonyl and D is Phe-$NH_2$, Tiq-$NH_2$, (dehydro)Phe-$NH_2$, (NMe)Phe-$NH_2$, $\alpha$-Nal-$NH_2$ or $\beta$-Nal-$NH_2$.

3. A compound selected from the group consisting of:

Ctp-Leu-Asp-Phe-$NH_2$;

BOC-Trp-Leu-Asp-Tiq-$NH_2$;

Ctp-Leu-Asp-N-Me-Phe-$NH_2$;

Ctp-Leu-Asp-(dehydro)Phe-$NH_2$;

Boc-Trp-Tpp-Asp-Phe-$NH_2$;

Ctp-Tpp-Asp-(NMe)Phe-$NH_2$;

Ctp-Tpp-Asp-$\alpha$-Nal-$NH_2$;

Ctp-Tpp-Asp-$\beta$-Nal-$NH_2$; and

Ctp-1,4-thiazane-3-carbonyl-Asp-Phe-$NH_2$.

4. Ctp-Tpp-Asp-Phe-$NH_2$.

5. Use of a therapeutically effective amount of a compound of claim 1, for mimicking the effects of CCK on CCK type-B receptors in a mammalian host in need of such treatment.

6. Use of a therapeutically effective amount of a compound of claim 1, for treating central nervous system dysfunctions, enhancing learning and memory or appetite related disorders in a mammalian host in need of such treatment.

7. Use of a therapeutically effective amount of a compound of claim 1 for treating substance abuse in a mammalian host in need of such treatment.

8. The use of Claim 7 wherein the substance abuse is alcohol addiction or nicotine addiction.

9. A CCK type-B receptor agonist composition comprising a pharmaceutical carrier and a therapeutically effective amount of a compound of Claim 1.

10 . A process for the preparation of a compound of Claim 1 comprising:

a) stepwise coupling of the appropriately protected amino acids or amino acid analogs represented by A, A-B, B, C, C-D and D as defined herein, followed by removal of the protecting groups; or

b) coupling of the appropriately protected fragments of dipeptide length or greater, said fragments comprising the amino acids or amino acid analogs represented by A, A-B, B, C, C-D and D as defined herein, followed by removal of the protecting groups.

European Patent Office

**PARTIAL EUROPEAN SEARCH REPORT**
which under Rule 45 of the European Patent Convention shall be considered, for the purposes of subsequent proceedings, as the European search report

Application number
EP 90 11 2261

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,X | MOLECULAR PHARMACOLOGY, vol. 36, no. 6, 1989, pages 881-886, The American Society for Pharmacology and Experimental Therapeutics; C.W. LIN et al.: "Distinct requirements for activation at CCK-A and CCK-B/Gastrin receptors: Studies with a C-terminal hydrazide analogue of cholecystokinin tetrapeptide (30-33)"<br>* Pages 881-882,885-886 *<br>-- | 1-4 | C 07 K 5/00<br>A 61 K 37/02 |
| A | PEPTIDES: CHEMISTRY AND BIOLOGY, PROCEEDINGS OF THE 10th AMERICAN PEPTIDE SYMPOSIUM, 23rd-28th May 1987, pages 112-114, edited by Garand R. Marshall, published by ESCOM, Leiden, NL; Y.K. SHUE et al.: "Trans carbon-carbon double bond isosteres of the<br>./. | | TECHNICAL FIELDS SEARCHED (Int. Cl.4)<br>C 07 K |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 3-4

Claims searched incompletely: 1-2,5-10

Claims not searched:

Reason for the limitation of the search:

The scope of claims 1 and 2 was considered too broad to be included in a single search.

| Place of search | Date of completion of the search | Examiner |
|---|---|---|
| THE HAGUE | 08-10-1990 | BEVAN |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

European Patent Office

# PARTIAL EUROPEAN SEARCH REPORT

Application number

EP 90 11 2261



| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| | peptide bond: General methodology and the synthesis of cholecystokinin (CCK) analogs"<br><br>* Whole article *<br><br>---- | 1-4 | |
| | | | TECHNICAL FIELDS SEARCHED (Int Cl 4) |